# EUROPEAN PATENT SPECIFICATION

(11) **EP 4 129 296 B1**
(45) Date of publication and mention of the grant of the patent: **22.10.2025**
(21) Application number: 20726534.9
(22) Date of filing: 07.04.2020
(51) Int. Cl.: A61K 31/551, A61K 31/5513, A61P 1/04, A61P 1/06

(54) **DRUG FOR PREVENTION OR TREATMENT OF IRRITABLE BOWEL SYNDROME OR INFLAMMATORY BOWEL DISEASE**
ARZNEIMITTEL ZUR PRÄVENTION ODER BEHANDLUNG DES REIZDARMSYNDROMS ODER ENTZÜNDLICHER DARMERKRANKUNGEN
MÉDICAMENT POUR LA PRÉVENTION OU LE TRAITEMENT DU SYNDROME DE L'INTESTIN IRRITABLE OU DE LA MALADIE INFLAMMATOIRE DE L'INTESTIN

(30) Priority: 30.03.2020 IT 202000006625
(43) Date of publication of application: 08.02.2023
(73) Proprietor: Nippon Chemiphar Co., Ltd., Chiyoda-ku Tokyo 101-0032 (JP)
(72) Inventor: ANTONIOLI, Luca, 56019 Vecchiano (PI) (IT); PELLEGRINI, Carolina, 55049 Viareggio (LU) (IT); FORNAI, Matteo, 57123 Livorno (IT); BLANDIZZI, Corrado, deceased (IT)
(74) Representative: Studio Torta S.p.A.
(86) International application number: PCT/IB2020/053313
(87) International publication number: WO 2021/198745

(56) References cited:
- EP-A1- 2 397 480
- EP-A1- 2 803 662
- EP-A1- 3 020 707
- WO-A1-2018/104305
- WO-A2-2015/200766
- CA-A1- 2 519 987
- JORGE GUZMAN ET AL: "ADOA3R as a Therapeutic Target in Experimental Colitis: Proof by Validated High-density Oligonucleotide Microarray Analysis :", HHS PUBLIC ACCESS AUTHOR MANUSCRIPT, vol. 12, no. 8, 1 August 2006 (2006-08-01), US, pages 766 - 789, XP055753202, ISSN: 1078-0998, DOI: 10.1097/00054725-200608000-00014
- DATABASE CA [online] CHEMICAL ABSTRACTS SERVICE, COLUMBUS, OHIO, US; 2008, WANG, JINGJIE ET AL: "Effects of expressions of P2X4 receptor in nerve center induced by stimulation of colorectal distention in rats with irritable", XP002801197, retrieved from STN Database accession no. 2009:1055787
- WANG, JINGJIE ET AL: "Effects of expressions of P2X4 receptor in nerve center induced by stimulation of colorectal distention in rats with irritable", WEICHANGBINGXUE HE GANBINGXUE ZAZHI , 17(10), 813-817 CODEN: WHGZAO; ISSN: 1006-5709, 2008
- YUTA MATSUMURA ET AL: "A novel P2X4 receptor-selective antagonist produces anti-allodynic effect in a mouse model of herpetic pain", SCIENTIFIC REPORTS, vol. 6, no. 1, 31 August 2016 (2016-08-31), XP055753420, DOI: 10.1038/srep32461

## Description

### Technical Field

The present invention relates to a medicament for use in preventing or treating irritable bowel syndrome and inflammatory bowel disease.

### Background Art

Irritable bowel syndrome (IBS) is a typical functional gastrointestinal disorder and is a chronic disorder of which core symptoms accompanied by abdominal pains, abdominal discomfort, and an altered defecation related thereto, such as diarrhea and constipation. The symptom of the irritable bowel syndrome may be exacerbated by a psychological factor or stress. According to the ROME III diagnostic criteria, it has been suggested that the symptoms are classified into a diarrhea type, a constipation type, a mixed type which is a combination of features of both the diarrhea type and the constipation type, and an unclassified type which does not satisfy any of the three types, and IBS is subclassified into these types according to a form of stool (25% or more of the entire stool).

As a drug treatment for the irritable bowel syndrome, for example, a tricyclic antidepressant is effective for a diarrhea type, however, a side effect such as drowsiness, constipation, or dry mouth is likely to be caused. A 5-HT₃ antagonist has been used as a drug for constipation-predominant-type irritable bowel syndrome overseas. In addition, a drug having a 5-HT₄ receptor stimulant action or 5-HT₄ receptor stimulant and 5-HT₃ receptor stimulant actions has been effective for abdominal pains and constipation overseas. However, 5-HT drugs are limitedly used due to side effects. Therefore, drug therapy for the irritable bowel syndrome has been so far extremely limited (Non Patent Literature 1).

Inflammatory bowel disease (IBD) is a multifactorial immune disorder which causes a chronic, recurrent inflammation in the intestinal tract. The inflammatory bowel disease is classified broadly into Crohn's disease (CD) and ulcerative colitis (UC) and may be referred to as narrowly defined inflammatory bowel disease such as a simple ulcer and non-specific multiple ulcers of small intestine (Non Patent Literature 2).

Crohn's disease and ulcerative colitis clinically share common symptoms of diarrhea, a bloody stool, abdominal pains, and the like, however, etiology of the inflammatory bowel disease is currently unclear.

However, it is considered that, in a case where a specific environmental factor induces a host who is likely to be genetically affected, disruption of an immune system and/or imbalanced interaction with a microorganism is connected to the onset of chronic enteritis. Hitherto, a Th1 cell has been considered to play an important role in etiology related to chronic inflammation in intestine, particularly in Crohn's disease, and a Th2 cell has been considered to play an important role in ulcerative colitis.

In addition, activation of a Th17 cell and imbalance between Th17/regulatory T (Treg) cells have recently been recognized as important elements in occurrence of inflammation in the intestine (Non Patent Literature 3).

Examples of a cause of increase in incidence of the inflammatory bowel disease can be mentioned insufficient effectiveness and/or safety of a conventional drug (corticosteroid, immunosuppressant, and salicylate). Examples of other treatment options can be mentioned treatment using a biotechnological drug. However, even though the treatment exhibits a certain degree of effectiveness, the treatment is accompanied by a serious side effect.

Therefore, there is currently an ongoing effort to search for a novel drug from which a positive benefit/risk profile is obtained, instead of settling for a pharmacological treatment of IBD (Non Patent Literature 4).

Under such circumstance, over many years, many evidence that ATP plays an important role regarding generation of an immune/inflammatory response have been reported. From these reports, it has been revealed that a P2X4 receptor is involved in mediating such ATP-regulated activation. Particularly, the P2X4 receptor maintains a proinflammatory response which sustains release of a proinflammatory cytokine such as IL-1β and IL-18. Furthermore, there are several evidence showing that P2X4 is involved in a plurality of immune/inflammatory pathological conditions such as postischemic inflammation, rheumatoid arthritis, respiratory tract inflammation asthma, a neurodegenerative disease, and metabolic syndrome (Non Patent Literatures 5 to 7).

As described above, the conventional drugs or treatments in Non Patent Literatures 1 to 7 can also be used as medicaments for treating the irritable bowel syndrome or the inflammatory bowel disease, however, it is known that effectiveness thereof is weak, or the drugs can cause a certain side effect, and thus the drugs are not effective as the medicament for treating the irritable bowel syndrome or the inflammatory bowel disease.

Patent Literature 1 discloses a P2X4 receptor antagonist.

However, a compound described in an example in Patent Literature 1 is a selective serotonin reuptake inhibitor, for example, Paroxetine, Fluoxetine, or the like, and has a structure that is completely different from that of a compound of the present application, which is a benzodiazepine derivative compound. Furthermore, only a result of an experiment using a neuropathic pain pathology model in which a nerve injury (L5 spinal nerve injury model) is performed is shown, and whether the P2X4 receptor antagonist has a treatment effect in the irritable bowel syndrome or the inflammatory bowel disease is not determined.

In addition, Patent Literature 2 also discloses a compound exhibiting a P2X4 receptor antagonistic action, however, as in Patent Literature 1, only an effect exhibited by a neuropathic pain model is shown, and whether the compound has a treatment effect in the irritable bowel syndrome or the inflammatory bowel disease is unclear.

Moreover, the present applicant has also filed patent applications related to the P2X4 receptor antagonist as in Patent Literatures 3 to 9, however, whether the P2X4 receptor antagonist has a treatment effect in the irritable bowel syndrome or the inflammatory bowel disease is unclear in all of the applications.

CA2519987 discloses benzofuro-1,4-diazepin-2-one P2X4 receptor antagonists suitable for the treatment of chronically inflammatory bowel disorders.

WO2018104305 discloses a P2X4 antagonists suitable for the treatment of gastrointestinal disorders including irritable bowel syndrome, inflammatory bowel disease, diarrhea-dominant IBS and Chron's disease.

Jorge Guzman et al., HHS PUBLIC ACCESS AUTHOR MANUSCRIPT, Vol. 12, No. 8, 1 August 2006, pages 766-789 discloses the protective effect of the ADOA3R agonist N(6)-(3-iodobenzyl)-adenosine-5-N-methyluronamide (IB-MECA) on gene dysregulation and injury in a rat chronic model of 2,4,6-trinitrobenzene sulfonic acid (TNBS)--induced colitis.

### Citation List

### Patent Literature

Patent Literature 1: WO 2008/020651 A
Patent Literature 2: WO 2010/093061 A
Patent Literature 3: WO 2008/023847 A
Patent Literature 4: WO 2012/008478 A
Patent Literature 5: WO 2012/014910 A
Patent Literature 6: WO 2012/017876 A
Patent Literature 7: WO 2013/105608 A
Patent Literature 8: WO 2015/005468 A
Patent Literature 9: WO 2015/005467 A

### Non Patent Literature

Non Patent Literature 1: The Japanese Society of Gastroenterology Clinical Practice Guidelines for Functional Dyspepsia 2014_Irritable Bowel Syndrome (IBS)
Non Patent Literature 2: The Journal of the Japanese Society of Internal Medicine 98 (1), 5-11, 2009-01-10
Non Patent Literature 3: Kim DH and Cheon JH (2017) Pathogenesis of Inflammatory Bowel Disease and Recent Advances in Biologic Therapies. Immune network 17: 25-40.
Non Patent Literature 4: Katz S (2007) "Mind the Gap": an unmet need for new therapy in IBD. Journal of clinical gastroenterology 41: 799-809.
Non Patent Literature 5: Kawano A, Tsukimoto M, Mori D, Noguchi T, Harada H, Takenouchi T, Kitani H and Kojima S (2012) Regulation of P2X7-dependent inflammatory functions by P2X4 receptor in mouse macrophages. Biochemical and biophysical research communications 420: 102-107.
Non Patent Literature 6: Sakaki H, Fujiwaki T, Tsukimoto M, Kawano A, Harada H and Kojima S (2013) P2X4 receptor regulates P2X7 receptor-dependent IL-1beta and IL-18 release in mouse bone marrow-derived dendritic cells. Biochemical and biophysical research communications 432: 406-411.
Non Patent Literature 7: Suurvali J, Boudinot P, Kanellopoulos J and Ruutel Boudinot S (2017) P2X4: A fast and sensitive purinergic receptor. Biomedical journal 40: 245-256.
Non Patent Literature 8: Antonioli L, et al. Inhibition of Adenosine Deaminase Attenuates Inflammation in Experimental Colitis. J Pharmacol Exp Ther. 2007; 322(2): 435-42
Non Patent Literature 9: Antonioli L, et al. The Blockade of Adenosine Deaminase Ameliorates Chronic Experimental Colitis through the Recruitment of Adenosine A2A and A3 Receptors. J Pharmacol Exp Ther. 2010; 335(2): 434-42
Non Patent Literature 10: Pellegrini C, et al. Front Pharmacol. 2018; 9: 1405
Non Patent Literature 11: JOHN L. Wallace and CATHERINE M. Keenan. An orally active inhibitor of leukotriene synthesis accelerates healing in a rat model of colitis. The American journal of physiology 1990; 258 (4 Pt 1): G527-34.
Non Patent Literature 12: Fornai M et al. Differential Role of Cyclooxygenase 1 and 2 Isoforms in the Modulation of Colonic Neuromuscular Function in Experimental Inflammation. J Pharmacol Exp Ther. 2006; 317(3): 938-45.

### Summary of Invention

The references to methods of treatment in paragraphs of this description are to be interpreted as references to the compounds, pharmaceutical compositions and medicaments of the present invention for use in a method for treatment of the human (or animal) body by therapy.

### Technical Problem

An object of the present invention is to provide a medicament for use in preventing or treating irritable bowel syndrome or inflammatory bowel disease.

### Solution to Problem

In order to solve the above problem, the present inventor has conducted intensive research, and as a result, the present inventor found that compounds
(A16) 5-[3-(5-thioxo-4H-[1,2,4]oxadiazol-3-yl)phenyl]-1H-naphtho[1,2-b][1,4]diazepine-2,4(3H,5H)-dione;
(A17) a 5-[3-(5-thioxo-4H-[1,2,4]oxadiazol-3-yl)phenyl]-1H-naphtho[1,2-b][1,4]diazepine-2,4(3H,5H)-dione sodium salt; and
(B48) 5-[4-(2-iodobenzoylamino)phenyl]-1H-naphtho[1,2-b][1,4]diazepine-2,4(3H,5H)-dione, that have a P2X4 receptor antagonistic action are useful in preventing or treating the irritable bowel syndrome or the inflammatory bowel disease, thus completing the present invention.

That is, the present invention provides a medicament for use in preventing and/or treating irritable bowel syndrome or inflammatory bowel disease, the medicament comprising a compound having a P2X4 receptor antagonistic action or a pharmaceutically acceptable salt thereof as an active ingredient, as defined in the claims.

The medicament of the present invention can be for use in, for example, preventing or treating the irritable bowel syndrome or the inflammatory bowel disease, particularly for preventing or treating Crohn's disease in the inflammatory bowel disease or preventing or treating ulcerative colitis in the inflammatory bowel disease. Furthermore, the medicament of the present invention can be used for, for example, remission induction therapy or remission maintenance therapy in the inflammatory bowel disease. Furthermore, the medicament of the present invention can be used for, for example, the remission induction therapy or the remission maintenance therapy in Crohn's disease or ulcerative colitis.

Furthermore, the medicament of the present invention can be used for, for example, suppressing or reducing adhesions or ulcers in an intestinal tissue of the irritable bowel syndrome or the inflammatory bowel disease. Furthermore, the medicament of the present invention can be used for, for example, suppressing or reducing a region of transmural inflammation in the intestinal tissue of the irritable bowel syndrome or the inflammatory bowel disease.

Furthermore, the medicament of the present invention can be used for, for example, suppressing or reducing histological damage in the intestinal tissue of the irritable bowel syndrome or the inflammatory bowel disease. Furthermore, the medicament of the present invention can be used for, for example, suppressing or reducing mucosal necrosis in the intestinal tissue of the irritable bowel syndrome or the inflammatory bowel disease. Furthermore, the medicament of the present invention can be used for, for example, suppressing or reducing cellular infiltration in the intestinal tissue of the irritable bowel syndrome or the inflammatory bowel disease. Furthermore, the medicament of the present invention can be used for, for example, protecting or maintaining mucosa in the intestinal tissue of the irritable bowel syndrome or the inflammatory bowel disease.

### Advantageous Effects of Invention

The medicament of the present invention is used as a medicament for preventing or treating irritable bowel syndrome or inflammatory bowel disease, particularly, is used as a medicament for preventing or treating Crohn's disease in the inflammatory bowel disease or as a medicament for preventing or treating ulcerative colitis in the inflammatory bowel disease, furthermore, is used as a medicament for remission induction therapy or remission maintenance therapy in the inflammatory bowel disease, or is used as a medicament for remission induction therapy or remission maintenance therapy in Crohn's disease or ulcerative colitis, furthermore, the medicament is used as, for example, a medicament for suppressing or reducing adhesions or ulcers in an intestinal tissue of irritable bowel syndrome or inflammatory bowel disease or is used as a medicament for suppressing or reducing a region of transmural inflammation in an intestinal tissue of irritable bowel syndrome or inflammatory bowel disease, and furthermore, the medicament is used as, for example, a medicament for suppressing or reducing histological damage in an intestinal tissue of irritable bowel syndrome or inflammatory bowel disease, is used as a medicament for suppressing or reducing mucosal necrosis in an intestinal tissue of irritable bowel syndrome or inflammatory bowel disease, is used as a medicament for suppressing or reducing cellular infiltration in an intestinal tissue of irritable bowel syndrome or inflammatory bowel disease, or is used as a medicament for protecting or maintaining mucosa in an intestinal tissue of irritable bowel syndrome or inflammatory bowel disease, each of which is expected to be highly effective.

### Brief Description of Drawings

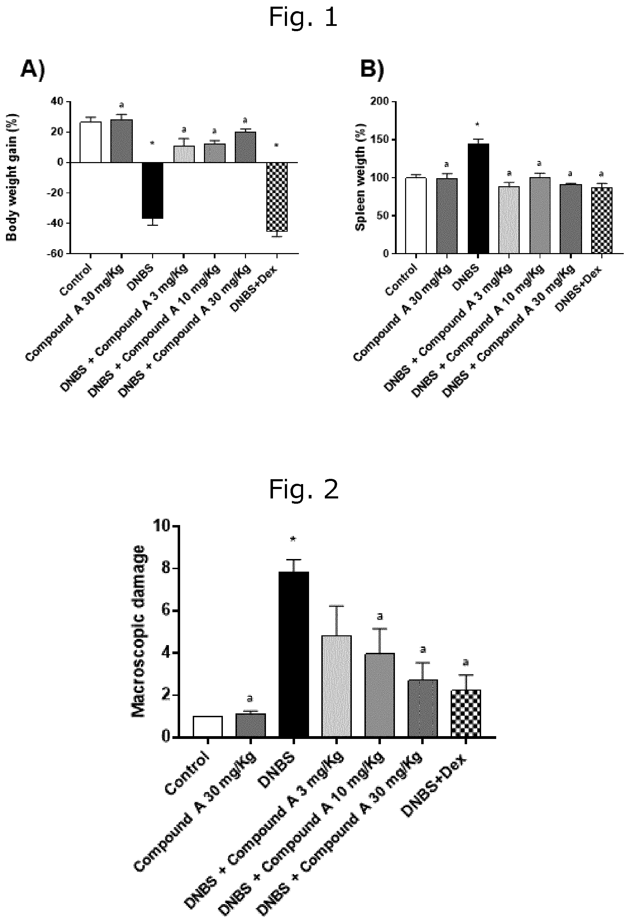

Fig. 1 is a diagram showing comparison of increases or decreases in (A) body weights and (B) pancreas weights in control rats and a DNBS-induced colitis model and further comparison of the same in the model and a case of orally administering Compound A to the control rats and in the model and a case of orally administering Compound A or dexamethasone to the model.
Fig. 2 is a diagram showing comparison of macroscopic damage of colonic tissues in the control rats and the DNBS-induced colitis model and further comparison of the same in the model and the case of orally administering Compound A to the control rats and in the model and the case of orally administering Compound A or dexamethasone to the model.

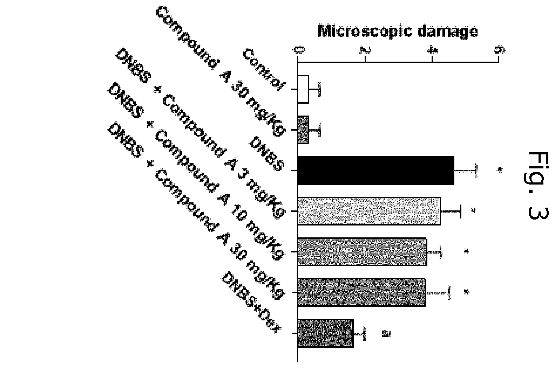

Fig. 3 is a diagram showing comparison of microscopic damage (histological damage) of colonic tissues in the control rats and the DNBS-induced colitis model and further comparison of the same in the model and the case of orally administering Compound A to the control rats and in the model and the case of orally administering Compound A or dexamethasone to the model.

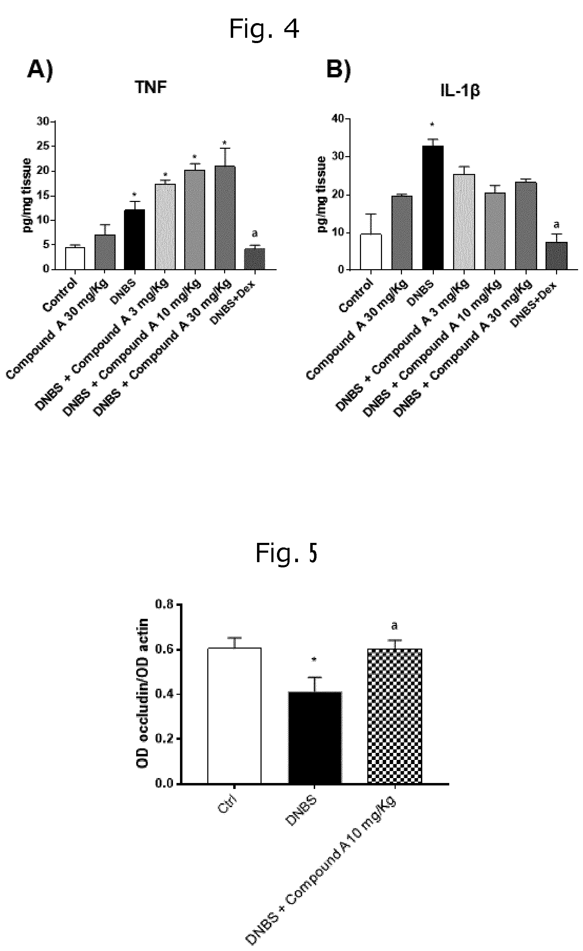

Fig. 4 is a diagram showing comparison of associations with proinflammatory cytokines (A) TNF and (B) interleukin-1β in colonic tissues of the control rats and colonic tissues of the DNBS-induced colitis model that are subjected to colonic damage and further comparison of the same in the model and the case of orally administering Compound A to the control rats and in the model and the case of orally administering Compound A or dexamethasone to the model.
Fig. 5 is a diagram showing comparison of occludin expression effects in colonic tissues of the control rats and colonic tissues of the DNBS-induced colitis model that are subjected to colonic damage and further comparison of the same in the model and a case of orally administering Compound A to the model.

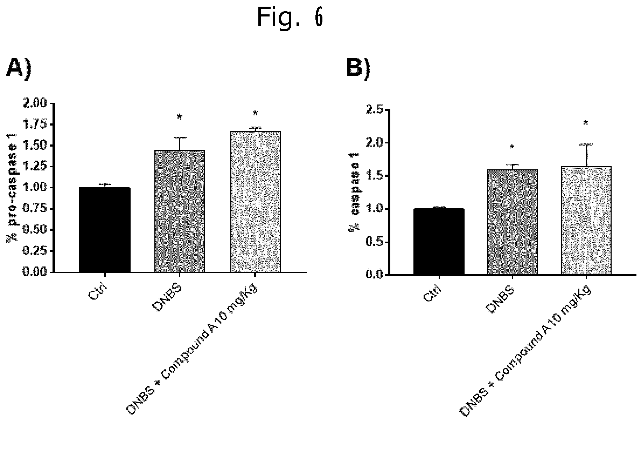

Fig. 6 is a diagram showing comparison of (A) procaspase-1 expression effects and (B) caspase-1 expression effects in colonic tissues of the control rats and colonic tissues of the DNBS-induced colitis model that are subjected to colonic damage and further comparison of the same in the model and the case of orally administering Compound A to the model.

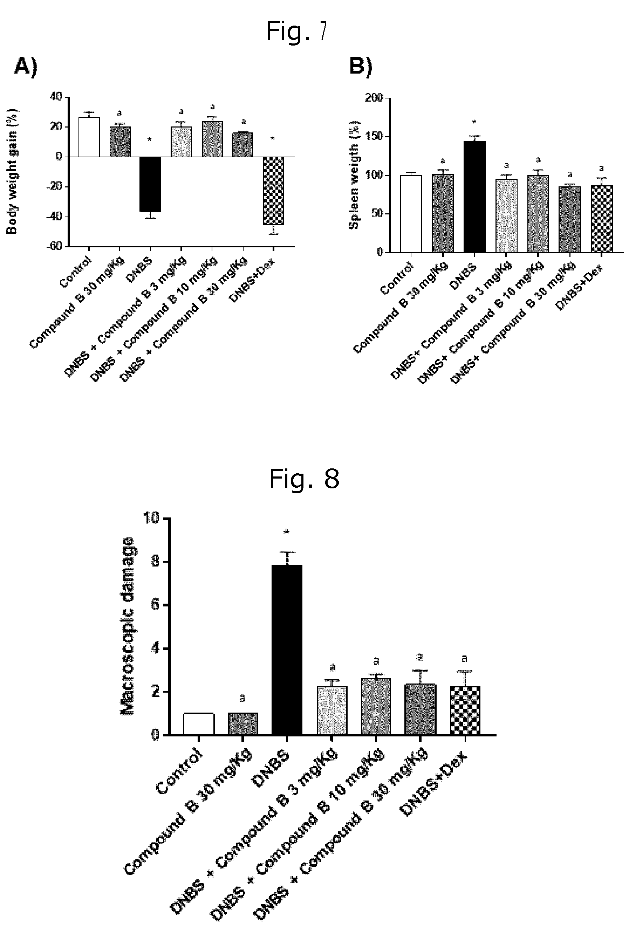

Fig. 7 is a diagram showing comparison of increases or decreases in (A) body weights and (B) pancreas weights in the control rats and the DNBS-induced colitis model and further comparison of the same in the model and a case of orally administering Compound B to the control rats and in the model and a case of orally administering Compound B or dexamethasone to the model.
Fig. 8 is a diagram showing comparison of macroscopic damage of colonic tissues in the control rats and the DNBS-induced colitis model and further comparison of the same in the model and the case of orally administering Compound B to the control rats and in the model and the case of orally administering Compound B or dexamethasone to the model.

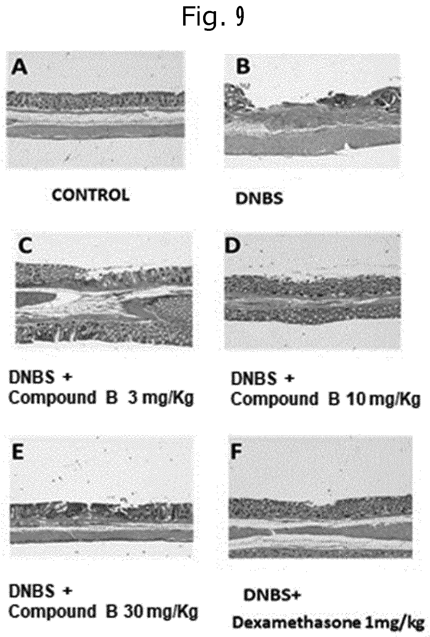

Fig. 9 is a diagram showing comparison of microscopic damage (histological damage) of colonic tissues in the control rats, the DNBS-induced colitis model, and the model orally administered with Compound B or dexamethasone by histological examination of colon specimens.

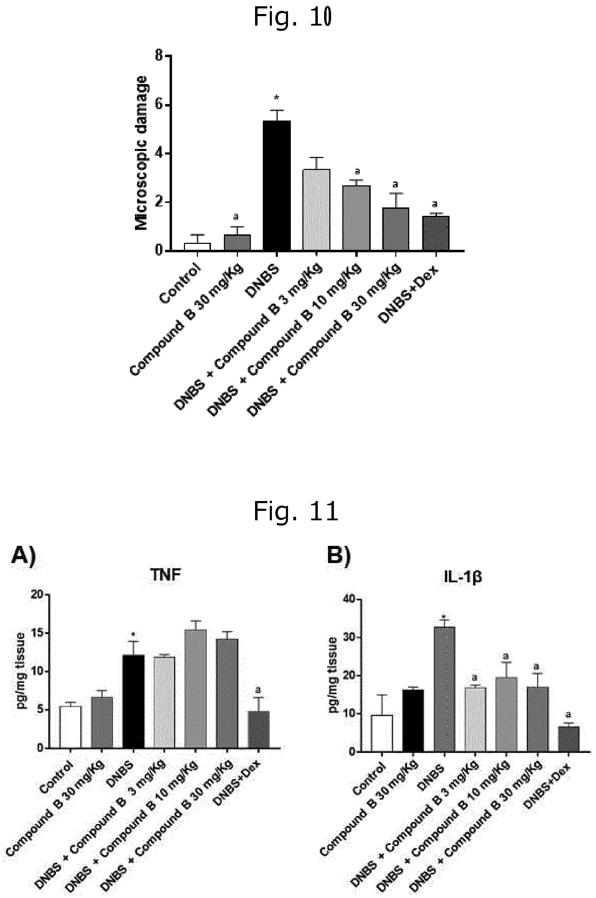

Fig. 10 is a diagram showing comparison of microscopic damage (histological damage) of colonic tissues in the control rats and the DNBS-induced colitis model and further comparison of the same in the model and the case of orally administering Compound B to the control rats and in the model and the case of orally administering Compound B or dexamethasone to the model.
Fig. 11 is a diagram showing comparison of associations with proinflammatory cytokines (A) TNF and (B) interleukin-1β in colonic tissues of the control rats and colonic tissues of the DNBS-induced colitis model that are subjected to colonic damage and further comparison of the same in the model and the case of orally administering Compound B to the control rats and in the model and the case of orally administering Compound B or dexamethasone to the model.

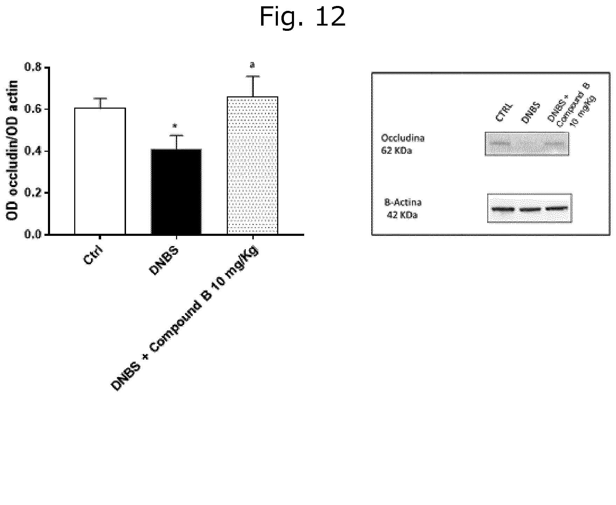

Fig. 12 is a diagram showing comparison of occludin expression effects in colonic tissues of the control rats and colonic tissues of the DNBS-induced colitis model that are subjected to colonic damage and further comparison of the same in the model and a case of orally administering Compound B to the model.

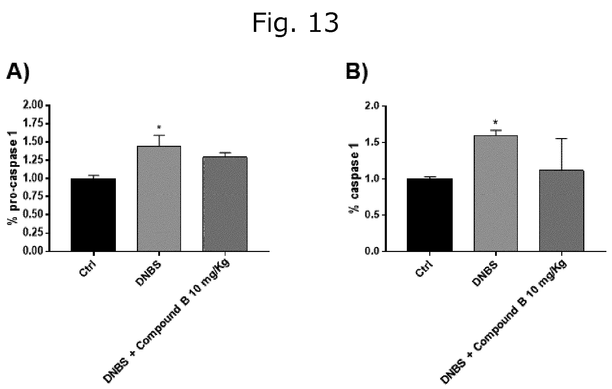

Fig. 13 is a diagram showing comparison of (A) procaspase-1 expression effects and (B) caspase-1 expression effects in colonic tissues of the control rats and colonic tissues of the DNBS-induced colitis model that are subjected to colonic damage and further comparison of the same in the model and the case of orally administering Compound B to the model.

Here, symbols "*" in Fig. 1 to Fig. 8 and Fig. 10 to Fig. 13 indicate that there is a significant difference (P < 0.05) as a result of comparison with control rats, and symbols "a" indicate that there is a significant difference (P < 0.05) as a result of comparison with a DNBS-induced colitis model.

### Description of Embodiments

A medicament of the present invention can be used as a medicament for preventing or treating irritable bowel syndrome or inflammatory bowel disease, and can also be used as a medicament for the following uses.

The medicament of the present invention can be used as a medicament for preventing or treating Crohn's disease or ulcerative colitis in the inflammatory bowel disease.

The medicament of the present invention can be used as a medicament for remission induction therapy or remission maintenance therapy in the inflammatory bowel disease.

The medicament of the present invention can be used as a medicament for remission induction therapy or remission maintenance therapy in the Crohn's disease or the ulcerative colitis.

The medicament of the present invention can be used as a medicament for suppressing or reducing adhesions or ulcers in an intestinal tissue of the irritable bowel syndrome or the inflammatory bowel disease.

The medicament of the present invention can be used as a medicament for suppressing or reducing a region of transmural inflammation in the intestinal tissue of the irritable bowel syndrome or the inflammatory bowel disease.

The medicament of the present invention can be used as a medicament for suppressing or reducing histological damage in the intestinal tissue of the irritable bowel syndrome or the inflammatory bowel disease.

The medicament of the present invention can be used as a medicament for suppressing or reducing mucosal necrosis in the intestinal tissue of the irritable bowel syndrome or the inflammatory bowel disease.

The medicament of the present invention can be used as a medicament for suppressing or reducing cellular infiltration in the intestinal tissue of the irritable bowel syndrome or the inflammatory bowel disease.

The medicament of the present invention can be used as a medicament for protecting or maintaining mucosa in the intestinal tissue of the irritable bowel syndrome or the inflammatory bowel disease.

As another aspect, the medicament of the present invention can be used as a medicament for the following uses.

The medicament of the present invention can be used as a medicament for preventing or treating the irritable bowel syndrome or the inflammatory bowel disease accompanied by adhesions or ulcers in the intestinal tissue.

The medicament of the present invention can be used as a medicament for preventing or treating the irritable bowel syndrome or the inflammatory bowel disease accompanied by development of a region of transmural inflammation in the intestinal tissue.

The medicament of the present invention can be used as a medicament for preventing or treating the irritable bowel syndrome or the inflammatory bowel disease accompanied by histological damage in the intestinal tissue.

The medicament of the present invention can be used as a medicament for preventing or treating the irritable bowel syndrome or the inflammatory bowel disease accompanied by necrosis or damage of mucosa in the intestinal tissue.

The medicament of the present invention can be used as a medicament for preventing or treating the irritable bowel syndrome or the inflammatory bowel disease accompanied by cellular infiltration in the intestinal tissue.

The medicament of the present invention can be used as a medicament for preventing or treating the irritable bowel syndrome or the inflammatory bowel disease accompanied by increase in a proinflammatory cytokine.

The medicament of the present invention can be used as a medicament for preventing or treating the irritable bowel syndrome or the inflammatory bowel disease accompanied by increase in interleukin-1β.

The medicament of the present invention can be used as a medicament for preventing or treating the irritable bowel syndrome or the inflammatory bowel disease accompanied by colonic damage and increase in a proinflammatory cytokine.

The medicament of the present invention can be used as a medicament for preventing or treating the irritable bowel syndrome or the inflammatory bowel disease accompanied by colonic damage and increase in interleukin-1β.

In the present specification, the term "prevention" is a concept comprising preventing onset of a "diseased" or "abnormal" symptom, state, or disease before an outbreak thereof and an action or a method therefor.

In the present specification, the term "treatment" is a concept comprising eliminating, completely curing, healing, or remitting a "diseased" or "abnormal" symptom, state, or disease and an action or a method therefor, suppressing exacerbation of a "diseased" or "abnormal" symptom, state, or disease and an action or a method therefor, and improvement. Here, the term "improvement" is a concept comprising approach of a "diseased" or "abnormal" symptom, state, or disease to a "healthy" or "normal" state or an action or a method therefor, and causing a "diseased" or "abnormal" symptom, state, or disease to be in a "healthy" or "normal" state or an action or a method therefor. Therefore, the term "improvement" in one embodiment comprises a concept in which a numerical value which serves as an index of a "diseased" or "abnormal" symptom or state becomes small or large so as to approach a normal value or be the normal value in accordance with the "improvement". Furthermore, the term "suppression" is a concept comprising stopping or slowing down exacerbation or progression of a symptom, state, or disease and therefor, and improving the symptom, state, or disease or an action or a method therefor. Here, the term "improvement" has the meaning described above. The expression "exacerbation or progression of symptom, state, or disease" comprises exacerbation or progression of a "diseased" or "abnormal" symptom, state, or disease and exacerbation or progression from a "healthy" or "normal" state to a "diseased" or "abnormal" symptom, state, or disease. The term "suppression" in one embodiment is stopping or slowing down exacerbation or progression of a symptom, state, or disease or an action or a method therefor. The term "suppression" in another embodiment means stopping or slowing down exacerbation or progression of a symptom, state, or disease.

The term "treatment" in one embodiment is eliminating, completely curing, healing, or remitting a "diseased" or "abnormal" symptom, state, or disease and an action or a method therefor. The term "treatment" in another embodiment is eliminating, completely curing, healing, or remitting a "diseased" or "abnormal" symptom, state, or disease. In the present specification, the term "remission induction therapy" means a treatment method conducted for remission of a "diseased" or "abnormal" symptom, state, or disease during onset or exacerbation of the symptom, state, or disease, particularly during an acute phase of the exacerbation. Furthermore, the term "remission maintenance therapy" means a treatment method conducted for maintaining a remitted state of the symptom, state, or disease or for prevention of relapse of the symptom, state, or disease. "Remission induction therapy" in one embodiment is a treatment method conducted for remission of irritable bowel syndrome or inflammatory bowel disease, or a symptom accompanying the irritable bowel syndrome or the inflammatory bowel disease, and the term "remission maintenance therapy" means a treatment method conducted for maintaining a remitted stated of the irritable bowel syndrome or the inflammatory bowel disease, or a symptom accompanying the irritable bowel syndrome or the inflammatory bowel disease, or for preventing relapse of the irritable bowel syndrome or the inflammatory bowel disease, or a symptom accompanying the irritable bowel syndrome or the inflammatory bowel disease.

In the present specification, the expression "protection of mucosa" means maintaining or preserving a tissue or function of normal mucosa, or suppressing damage or failure of a tissue or function of mucosa. Here, the term "suppression" has the meaning described above. The expression "maintenance of mucosa" means preserving a tissue or function of normal mucosa, or suppressing deterioration of a tissue or function of mucosa. Here, the term "suppression" has the meaning described above.

In the present specification, the symptom, state, or disease, a numerical value which serves as an index of these, or a state or a function of a tissue of mucosa can be evaluated by comparison thereof before and after administration of the medicament provided by the present invention or by comparison between a group to which a placebo or a control that does not contain the medicament provided by the present invention is not administered and a group to which the medicament provided by the present invention is administered.

In the present specification, the term "suppression" can be used as a term meaning stopping or slowing down increase of supply in a living body.

In the present specification, the expression "maintenance of health of large intestinal tissue" means maintaining or preserving a normal large intestinal tissue or a function thereof, or suppressing damage or failure of a large intestinal tissue or a function thereof. Here, the term "suppression" has the meaning described above. The term "contribution" refers to exhibiting power to cause a certain state or function to become a desired state or function. The expression "contribute to maintenance of health of large intestinal tissue" means that, in one embodiment, as a result of some damage to a large intestine tissue, for example, exhibiting power to cause the damaged state and its function into the normal state of the large intestine tissue and its function.

As an active ingredient of the medicament of the present invention, compounds represented by the following formulas
(A16) 5-[3-(5-thioxo-4H-[1,2,4]oxadiazol-3-yl)phenyl]-1H-naphtho[1,2-b][1,4]diazepine-2,4(3H,5H)-dione;
(A17) a 5-[3-(5-thioxo-4H-[1,2,4]oxadiazol-3-yl)phenyl]-1H-naphtho[1,2-b][1,4]diazepine-2,4(3H,5H)-dione sodium salt; and
(B48) 5-[4-(2-iodobenzoylamino)phenyl]-1H-naphtho[1,2-b][1,4]diazepine-2,4(3H,5H)-dione.
or a pharmaceutically acceptable salt thereof can be used.

Symbols used in the tables below and the like are as follows. Me: methyl group, Et: ethyl group, Pr: n-propyl group, iPr: isopropyl group, tBu: tert-butyl group, Ac: acetyl group, Ph: phenyl group

In the tables below and the like, a substituent may be marked together with a position number indicating a substitution position of the substituent. In addition, in order to distinguish position numbers of positions that appear to be identical to each other in a chemical formula, one position number may be indicated with a prime symbol "'" for convenience, however, as long as a definitive structure for a compound name can be specified, position numbers may be indicated without using the prime symbol.

The compounds used in the present invention are:
(A16) 5-[3-(5-thioxo-4H-[1,2,4]oxadiazol-3-yl)phenyl]-1H-naphtho[1,2-b][1,4]diazepine-2,4(3H,5H)-dione;
(A17) a 5-[3-(5-thioxo-4H-[1,2,4]oxadiazol-3-yl)phenyl]-1H-naphtho[1,2-b][1,4]diazepine-2,4(3H,5H)-dione sodium salt; and
(B48) 5-[4-(2-iodobenzoylamino)phenyl]-1H-naphtho[1,2-b][1,4]diazepine-2,4(3H,5H)-dione.

Other compounds are for disclosure only.

(A-1) A compound represented by the following general formula (A) or a pharmaceutically acceptable salt thereof: 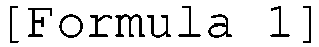 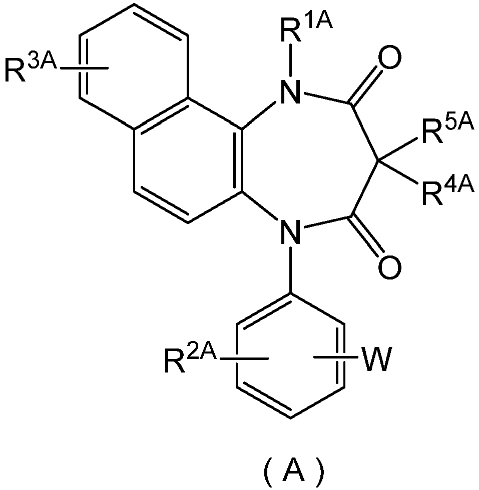
(in the formula, R^{1A} represents a hydrogen atom, an alkyl group having 1 to 8 carbon atoms, an alkenyl group having 2 to 8 carbon atoms, an alkyl group having 1 to 8 carbon atoms and substituted with 1 to 3 halogen atoms, or an alkyl group having 1 to 3 carbon atoms and substituted with a phenyl group;
R^{2A} and R^{3A} may be the same or different, and represent a hydrogen atom, an alkyl group having 1 to 8 carbon atoms, an alkoxy group having 1 to 8 carbon atoms, an alkyl group having 1 to 8 carbon atoms and substituted with 1 to 3 halogen atoms, an alkoxy group having 1 to 8 carbon atoms and substituted with 1 to 3 halogen atoms, a halogen atom, a hydroxyl group, a nitro group, a cyano group, an amino group, an alkylamino group having 1 to 8 carbon atoms, a dialkylamino group having 2 to 8 carbon atoms, an acylamino group having 2 to 8 carbon atoms, an acylamino group having 2 to 8 carbon atoms and substituted with 1 to 3 halogen atoms, an alkylsulfonylamino group having 1 to 8 carbon atoms, a carboxyl group, an acyl group having 2 to 8 carbon atoms, an alkoxycarbonyl group (the alkoxy moiety has 1 to 8 carbon atoms), a carbamoyl group, an alkylthio group having 1 to 8 carbon atoms, an alkylsulfinyl group having 1 to 8 carbon atoms, an alkylsulfonyl group having 1 to 8 carbon atoms, or a sulfamoyl group;
R^{4A} and R^{5A} may be the same or different, and represent a hydrogen atom, an alkyl group having 1 to 8 carbon atoms, an alkyl group having 1 to 8 carbon atoms and substituted with 1 to 3 halogen atoms, or an alkyl group having 1 to 3 carbon atoms and substituted with a phenyl group; and
W^{A} represents a five or six membered heterocyclic ring comprising 1 to 4 nitrogen atoms as the members of the ring, which may have a substituent)

In the general formula (A), examples of the alkyl group having 1 to 8 carbon atoms represented by R^{1A}, R^{2A}, R^{3A}, R^{4A}, and R^{5A} can comprise a methyl group, an ethyl group, a propyl group, an isopropyl group, a butyl group, an i-butyl group, a t-butyl group, a pentyl group, a hexyl group, and the like.

Examples of the alkenyl group having 2 to 8 carbon atoms represented by R^{1A} can comprise an allyl group and the like.

Examples of the alkyl group having 1 to 8 carbon atoms and substituted with 1 to 3 halogen atoms represented by R^{1A}, R^{2A}, R^{3A}, R^{4A}, and R^{5A} or R^{11A}, R^{12A}, R^{13A}, R^{14A}, and R^{15A} can comprise a methyl group, an ethyl group, a propyl group, an isopropyl group, a butyl group, a t-butyl group, and the like substituted with 1 to 3 halogen atoms such as a fluorine atom, a chlorine atom, and a bromine atom, and preferable examples thereof can comprise a trifluoromethyl group, a chloromethyl group, a 2-chloroethyl group, a 2-bromoethyl group, a 2-fluoroethyl group, and the like.

Examples of the alkyl group having 1 to 3 carbon atoms and substituted with a phenyl group represented by R^{1A}, R^{4A}, and R^{5A} can comprise a benzyl group and the like.

Examples of the alkoxy group having 1 to 8 carbon atoms represented by R^{2A} and R^{3A} can comprise a methoxy group, an ethoxy group, a propoxy group, an isopropoxy group, a butoxy group, an i-butoxy group, a t-butoxy group, a pentyloxy group, a hexyloxy group, and the like.

Examples of the alkoxy group having 1 to 8 carbon atoms and substituted with 1 to 3 halogen atoms represented by R^{2A} and R^{3A} can comprise a methyl group, an ethyl group, a propyl group, an isopropyl group, a butyl group, a t-butyl group, and the like substituted with 1 to 3 halogen atoms such as a fluorine atom, a chlorine atom, and a bromine atom, and preferable examples thereof can comprise a trifluoromethoxy group, a 2-chloroethoxy group, a 2-bromoethoxy group, a 2-fluoroethoxy group, and the like.

Examples of the halogen atom represented by R^{2A} and R^{3A} can comprise a fluorine atom, a chlorine atom, a bromine atom, and the like.

Examples of the alkylamino group having 1 to 8 carbon atoms represented by R^{2A} and R^{3A} can comprise a methylamino group, an ethylamino group, and the like.

Examples of the dialkylamino group having 1 to 8 carbon atoms represented by R^{2A} and R^{3A} can comprise a dimethylamino group, a diethylamino group, and the like.

Examples of the acylamino group having 2 to 8 carbon atoms represented by R^{2A} and R^{3A} can comprise an acetylamino group.

Examples of the acylamino group having 2 to 8 carbon atoms and substituted with 1 to 3 halogen atoms represented by R^{2A} and R^{3A} can comprise a trifluoromethylcarbonylamino group.

Examples of the alkylsulfonylamino group having 1 to 8 carbon atoms represented by R^{2A} and R^{3A} can comprise a methylsulfonylamino group.

Examples of the acyl group having 2 to 8 carbon atoms represented by R^{2A} and R^{3A} can comprise an acetyl group.

Examples of the alkoxycarbonyl group (the alkoxy moiety has 1 to 8 carbon atoms) represented by R^{2A} and R^{3A} can comprise a methoxycarbonyl group, an ethoxycarbonyl group, and the like.

Examples of the alkylthio group having 1 to 8 carbon atoms represented by R^{2A} and R^{3A} can comprise a methylthio group.

Examples of the alkylsulfinyl group having 1 to 8 carbon atoms represented by R^{2A} and R^{3A} can comprise a methylsulfinyl group.

Examples of the alkylsulfonyl group having 1 to 8 carbon atoms represented by R^{2A} and R^{3A} can comprise a methylsulfonyl group.

Examples of the five or six membered heterocyclic ring comprising 1 to 4 nitrogen atoms as the members of the ring, which may have a substituent, represented by W^{A} can comprise tetrazole, 1,2,4-triazole, 1,2,3-triazole, 1,2,4-oxadiazole, pyrazole, imidazole, oxazole, isoxazole, pyrrole, thiazole, pyridine, and pyrrolidine. Examples of the substituent that may be comprised in the five or six membered heterocyclic ring comprising 1 to 4 nitrogen atoms as the members of the ring, which may have a substituent, represented by W^{A} can comprise an alkyl group having 1 to 8 carbon atoms such as a methyl group and an ethyl group, an alkyl group having 1 to 8 carbon atoms and substituted with 1 to 3 halogen atoms such as a trifluoromethyl group, a halogen atom such as a fluorine atom, a cyano group, an oxo group, a thioxo group, and the like.

R^{2A} and R^{3A} in the general formula (A) may have 1 to 3 same or different ones in the benzene ring substituted by R^{2A} and R^{3A}.

As the compound represented by the general formula (A), the following compounds are preferable.
(A-2) The compound according to (A-1), in which W^{A} represents tetrazole, 1,2,4-triazole, 1,2,3-triazole, 1,2,4-oxadiazole, pyrazole, or imidazole that may have a substituent selected from an alkyl group having 1 to 8 carbon atoms, an alkyl group having 1 to 8 carbon atoms and substituted with 1 to 3 halogen atoms, a halogen atom, a cyano group, an oxo group, and a thioxo group.
(A-3) The compound according to (A-1) or (A-2), in which W^{A} represents tetrazole, 1,2,4-triazole, or 1,2,3-triazole that may have a substituent selected from an alkyl group having 1 to 8 carbon atoms, an alkyl group having 1 to 8 carbon atoms and substituted with 1 to 3 halogen atoms, a halogen atom, and a cyano group.
(A-4) The compound according to any one of (A-1) to (A-3), in which W^{A} represents 5-oxo-1,2,4-oxadiazole or 5-thioxo-1,2,4-oxadiazole.
(A-5) The compound according to any one of (A-1) to (A-4), in which W^{A} represents tetrazole.
(A-6) The compound according to any one of (A-1) to (A-5), in which R^{1A} represents a hydrogen atom or an alkyl group having 1 to 8 carbon atoms.
(A-7) The compound according to any one of (A-1) to (A-6), in which R^{1A} represents a hydrogen atom.
(A-8) The compound according to any one of (A-1) to (A-7), in which R^{4A} represents a hydrogen atom, and R^{5A} represents a hydrogen atom or an alkyl group having 1 to 8 carbon atoms.
(A-9) The compound according to any one of (A-1) to (A-8), in which R^{4A} and R^{5A} both represent hydrogen atoms.
(A-10) The compound according to any one of (A-1) to (A-9), in which R^{2A} represents a hydrogen atom, an alkyl group having 1 to 8 carbon atoms, an alkoxy group having 1 to 8 carbon atoms, an alkyl group having 1 to 8 carbon atoms and substituted with 1 to 3 halogen atoms, an alkoxy group having 1 to 8 carbon atoms and substituted with 1 to 3 halogen atoms, a halogen atom, a hydroxyl group, a nitro group, a cyano group, an amino group, a carboxyl group, an acyl group having 2 to 8 carbon atoms, or an alkoxycarbonyl group (the alkoxy moiety has 1 to 8 carbon atoms).
(A-11) The compound according to any one of (A-1) to (A-10), in which R^{2A} represents a hydrogen atom.
(A-12) The compound according to any one of (A-1) (A-11), in which R^{3A} represents a hydrogen atom, an alkyl group having 1 to 8 carbon atoms, an alkoxy group having 1 to 8 carbon atoms, an alkyl group having 1 to 8 carbon atoms and substituted with 1 to 3 halogen atoms, an alkoxy group having 1 to 8 carbon atoms and substituted with 1 to 3 halogen atoms, a halogen atom, a hydroxyl group, a nitro group, a cyano group, an amino group, a carboxyl group, an acyl group having 2 to 8 carbon atoms, or an alkoxycarbonyl group (the alkoxy moiety has 1 to 8 carbon atoms).
(A-13) The compound according to any one of (A-1) to (A-12), in which R^{3A} represents a hydrogen atom.

Examples of the pharmaceutically acceptable salt of the compound represented by the general formula (A) can comprise hydrochloride and the like, in a case where R^{2A} and R^{3A} in the general formula (A) represent amino groups or the like. In addition, in a case where R^{2A} and R^{3A} in the general formula (A) represent carboxyl groups, examples of the pharmaceutically acceptable salt of the compound represented by the general formula (A) can comprise a salt of an alkali metal such as sodium, potassium, and lithium.

Typical compounds comprised in the compounds represented by the general formula (A) are as follows.

### <Typical Compound A-100>

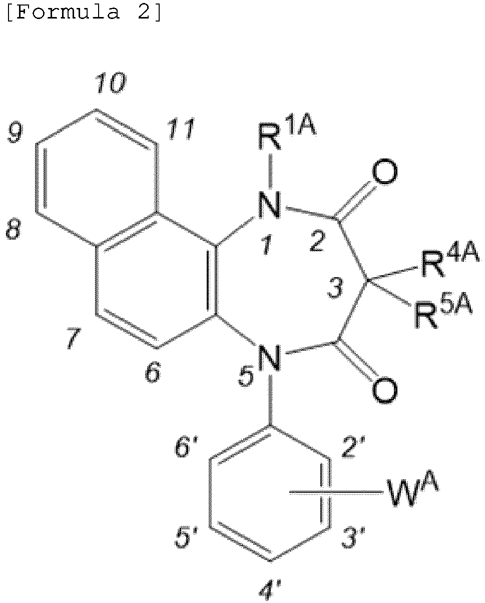

(R¹², R^{4A}, R^{5A}, and W^{A} in the formula and a substitution position of W^{A} are indicated in Tables 1 to 3)

In Tables 1 to 3, the substitution position of W^{A} indicates a substitution position on a benzene ring. That is, positions 2, 3, and 4 in the tables correspond to positions 2', 3', and 4' in a formula of Typical Compound A-100, respectively.

**[Table 1]**

| R¹ | Position of W | W | R⁴/R⁵ |
|---|---|---|---|
| H | 2- | 1H-tetrazol-5-yl | H/H |
| H | 3- | 1H-tetrazol-5-yl | H/H |
| H | 3- | (1-methyl-1H-tetrazol)-5-yl | H/H |
| H | 4- | 1H-tetrazol-5-yl | H/H |
| CH₃ | 3- | 1H-tetrazol-5-yl | H/H |
| CH₃ | 3- | 1H-tetrazol-5-yl | CH₃/H |
| benzyl | 3- | 1H-tetrazol-5-yl | H/H |
| H | 3- | 1H-tetrazol-1-yl | H/H |
| H | 3- | 1H-tetrazol-1-yl | CH₃/CH₃ |
| H | 3- | (1,2,3-triazol)-5-yl | H/H |
| H | 3- | (1,2,4-triazol)-3-yl | H/H |
| H | 4- | (1,2,4-triazol)-3-yl | H/H |

**[Table 2]**

| R¹ | Position of W | W | R⁴/R⁵ |
|---|---|---|---|
| H | 2- | (1,2,4-triazol)-1-yl | H/H |
| H | 3- | (1,2,4-triazol)-1-yl | H/H |
| H | 3- | [5-(trifluoromethyl)-1,2,4-triazol]-3-yl | H/H |
| H | 3- | [5-(trifluoromethyl)-1,2,4-triazol]-3-yl | ethyl/H |
| H | 3- | [5-fluoro-1,2,3-triazol]-4-yl | H/H |
| H | 3- | [5-fluoro-1,2,3-triazol]-4-yl | CH₃/CH₃ |
| H | 3- | [5-cyano-1,2,3-triazol]-4-yl | H/H |
| H | 4- | 1H-imidazol-1-yl | H/H |
| H | 4- | 1H-imidazol-1-yl | Pr/H |
| H | 3- | 1H-imidazol-2-yl | H/H |
| H | 3- | 1H-imidazol-4-yl | H/H |
| H | 3- | imidazolin-2-yl | H/H |

**[Table 3]**

| R¹ | Position of W | W | R⁴/R⁵ |
|---|---|---|---|
| H | 2- | pyrazol-3-yl | H/H |
| H | 3- | pyrazol-4-yl | H/H |
| H | 3- | pyrazol-5-yl | CH₃/H |
| H | 3- | (1,2,4-oxadiazol)-3-yl | H/H |
| H | 3- | (1,3,4-oxadiazol)-2-yl | H/H |
| H | 3- | (5-oxo-1,2,4-oxadiazol)-3-yl | H/H |
| H | 3- | pyrrol-1-yl | H/H |
| H | 4- | pyrrolidin-2-yl | H/H |
| CH₃ | 4- | pyrrolidin-2-yl | CH₃/H |
| H | 4- | (1,3-oxazol)-5-yl | H/H |
| H | 3- | (1,3-oxazol)-5-yl | H/H |
| H | 2- | (1,3-thiazol)-5-yl | H/H |

### <Typical Compound A-200>

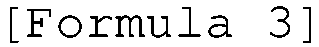

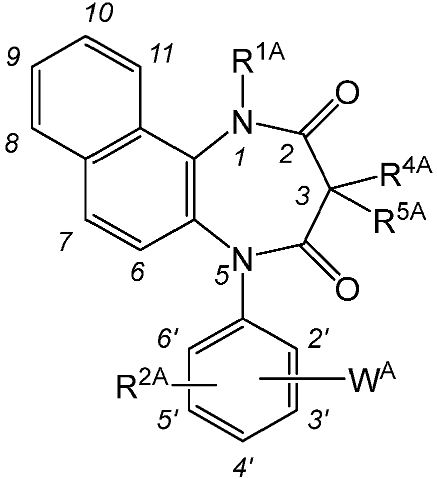

(R^{1A}, R^{2A}, R^{4A}, R^{5A}, and W^{A} in the formula and a substitution position of W^{A} are indicated in Tables 4 and 5)

In Tables 4 and 5, the substitution position of W^{A} indicates a substitution position on a benzene ring. That is, positions 2, 3, and 4 in the tables correspond to positions 2', 3', and 4' in a formula of Typical Compound A-200, respectively.

**[Table 4]**

| R¹ | R² | Position of W | W | R⁴ /R⁵ |
|---|---|---|---|---|
| H | 4-OH | 3- | 1H-tetrazol-5-yl | H/H |
| H | 4-OCH₃ | 3- | 1H-tetrazol-5-yl | H/H |
| CH₃ | 2-Cl | 3- | 1H-tetrazol-5-yl | H/H |
| H | 2,6-Cl | 3- | 1H-tetrazol-5-yl | H/H |
| H | 4-F | 3- | 1H-tetrazol-5-yl | H/H |
| H | 4-Br | 3- | 1H-tetrazol-5-yl | ethyl/H |
| H | 3-OCH₃ | 4- | (1-methyl-1H-tetrazol)-5-yl | H/H |
| H | 4-CH₃ | 3- | 1H-tetrazol-5-yl | H/H |

**[Table 5]**

| R¹ | R² | Position of W | W | R⁴ /R⁵ |
|---|---|---|---|---|
| H | 4-Cl | 3- | (1,2,3-triazol)-5-yl | CH₃/H |
| H | 4-CF₃ | 3- | (1,2,3-triazol)-5-yl | H/H |
| H | 3-SCH₃ | 4- | (1,2,4-triazol)-1-yl | H/H |
| H | 3-SO₂CH₃ | 4- | 1H-imidazol-1-yl | H/H |
| H | 3-NHSO₂CH₃ | 4- | 1H-imidazol-1-yl | H/H |
| H | 4-OCH₃ | 3- | 1H-imidazol-4-yl | H/H |
| H | 4-F | 2- | pyrazol-3-yl | H/H |

### <Typical Compound A-300>

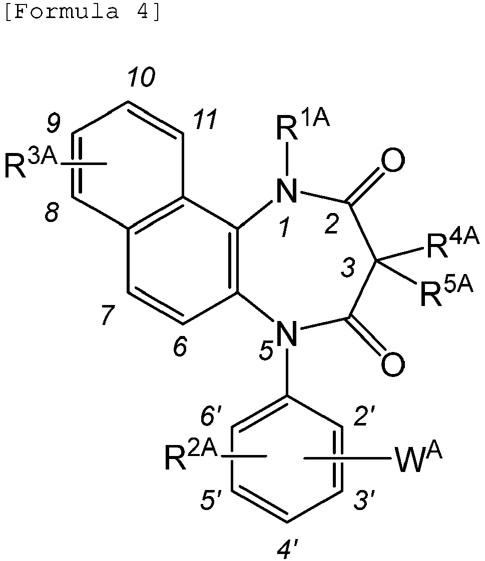

(R^{1A}, R^{2A}, R^{3A}, R^{4A}, R^{5A} and W^{A} in the formula and a substitution position of W^{A} are indicated in Tables 6 and 7)

In Tables 6 and 7, the substitution position of W^{A} indicates a substitution position on a benzene ring. That is, positions 3 and 4 in the tables correspond to positions 3' and 4' in a formula of Typical Compound A-300, respectively.

**[Table 6]**

| R¹ | R² | Position of W | W | R³ | R⁴/R⁵ |
|---|---|---|---|---|---|
| H | H | 3- | 1H-tetrazol-5-yl | 9-Br | H/H |
| H | 4-OCH₃ | 3- | 1H-tetrazol-5-yl | 9-Cl | H/H |
| H | 4-OH | 3- | 1H-tetrazol-5-yl | 10-OCH₃ | H/H |
| H | 2-Cl | 3- | 1H-tetrazol-5-yl | 9-Br | H/H |
| H | 2,6-Cl | 3- | 1H-tetrazol-5-yl | 9-CH₃ | H/H |
| H | H | 3- | 1H-tetrazol-5-yl | 10-Cl | CH₃/H |
| H | 3-OCH₃ | 4- | (1-methyl-1H-tetrazol)-5-yl | 9-CF₃ | H/H |

**[Table 7]**

| R¹ | R² | Po-si--tion of W | W | R³ | R⁴/R⁵ |
|---|---|---|---|---|---|
| H | 4-CH₃ | 3- | 1H-tetrazol-1-yl | 9-CN | Pr/H |
| CH₃ | H | 3- | (1,2,3-triazol)-5-yl | 9-OH | H/H |
| ethyl | H | 3- | (1,2,3-triazol)-5-yl | 10-F | H/H |
| H | 3-Br | 4- | (1,2,4-triazol)-1-yl | 9-SCH₃ | H/H |
| allyl | H | 4- | 1H-imidazol-1-yl | 8-OCH₃ | H/H |
| H | H | 3- | 1H-imidazol-1-yl | 10-OCH₃ | CH₃/CH₃ |

(B-1) A compound represented by the following general formula (BI) or a pharmaceutically acceptable salt thereof: 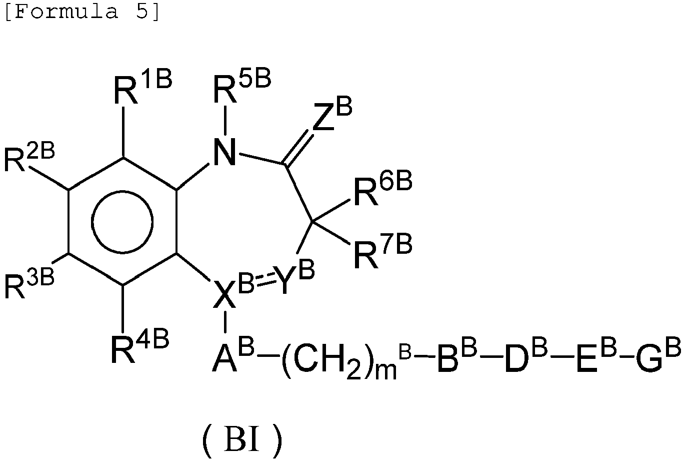
(in the formula, R^{1B} and R^{2B} may be the same or different, and represent a hydrogen atom, an alkyl group having 1 to 8 carbon atoms, a cycloalkyl group having 3 to 8 carbon atoms, an alkenyl group having 2 to 8 carbon atoms, an alkoxy group having 1 to 8 carbon atoms, an alkyl group having 1 to 8 carbon atoms and substituted with 1 to 3 halogen atoms, an alkoxy group having 1 to 8 carbon atoms and substituted with 1 to 3 halogen atoms, a halogen atom, a hydroxyl group, a nitro group, a cyano group, an amino group, an alkylamino group having 1 to 8 carbon atoms, a dialkylamino group having 2 to 8 carbon atoms, an acylamino group having 2 to 8 carbon atoms, a carboxyl group, an acyl group having 2 to 8 carbon atoms, an alkoxycarbonyl group (the alkoxy moiety has 1 to 8 carbon atoms), a phenyl group which may be substituted, a pyridyl group which may be substituted, or an aralkyl group (the aryl moiety has 6 to 10 carbon atoms, and the alkylene moiety has 1 to 8 carbon atoms), or
R^{1B} and R^{2B} may bind together to form a condensed ring selected from a naphthalene ring, a quinoline ring, an isoquinoline ring, a tetrahydronaphthalene ring, an indane ring, a tetrahydroquinoline ring, and a tetrahydroisoquinoline ring together with the benzene ring to which they bind, and the ring constituted by R^{1B} and R^{2B} bound to each other, together with the carbon atoms to which R^{1B} and R^{2B} bind may be substituted with 1 to 4 of the same or different substituents selected from an alkyl group having 1 to 8 carbon atoms, a cycloalkyl group having 3 to 8 carbon atoms, an alkenyl group having 2 to 8 carbon atoms, an alkoxy group having 1 to 8 carbon atoms, an alkyl group having 1 to 8 carbon atoms and substituted with 1 to 3 halogen atoms, an alkoxy group having 1 to 8 carbon atoms and substituted with 1 to 3 halogen atoms, a halogen atom, a hydroxyl group, a nitro group, a cyano group, an amino group, an alkylamino group having 1 to 8 carbon atoms, a dialkylamino group having 2 to 8 carbon atoms, an acylamino group having 2 to 8 carbon atoms, a carboxyl group, an acyl group having 2 to 8 carbon atoms, an alkoxycarbonyl group (the alkoxy moiety has 1 to 8 carbon atoms), and an aralkyl group (the aryl moiety has 6 to 10 carbon atoms, and the alkylene moiety has 1 to 8 carbon atoms),
R^{3B} and R^{4B} may be the same or different, and represent a hydrogen atom, an alkyl group having 1 to 8 carbon atoms, an alkenyl group having 2 to 8 carbon atoms, an alkoxy group having 1 to 8 carbon atoms, an alkyl group having 1 to 8 carbon atoms and substituted with 1 to 3 halogen atoms, an alkoxy group having 1 to 8 carbon atoms and substituted with 1 to 3 halogen atoms, a halogen atom, a hydroxyl group, a nitro group, a cyano group, an amino group, an alkylamino group having 1 to 8 carbon atoms, a dialkylamino group having 2 to 8 carbon atoms, an acylamino group having 2 to 8 carbon atoms, a carboxyl group, an acyl group having 2 to 8 carbon atoms, an alkoxycarbonyl group (the alkoxy moiety has 1 to 8 carbon atoms), or an aralkyl group (the aryl moiety has 6 to 10 carbon atoms, and the alkylene moiety has 1 to 8 carbon atoms),
R^{5B} represents a hydrogen atom, an alkyl group having 1 to 8 carbon atoms, an alkenyl group having 2 to 8 carbon atoms, an alkyl group having 1 to 8 carbon atoms and substituted with 1 to 3 halogen atoms, an alkyl group having 1 to 8 carbon atoms and substituted with a hydroxyl group, or an aralkyl group (the aryl moiety has 6 to 10 carbon atoms, and the alkylene moiety has 1 to 8 carbon atoms),
R^{6B} and R^{7B} may be the same or different, and represent a hydrogen atom, an alkyl group having 1 to 8 carbon atoms, an alkenyl group having 2 to 8 carbon atoms, an alkoxy group having 1 to 8 carbon atoms, an alkyl group having 1 to 8 carbon atoms and substituted with 1 to 3 halogen atoms, an alkoxy group having 1 to 8 carbon atoms and substituted with 1 to 3 halogen atoms, a halogen atom, a hydroxyl group, or an amino group,
X^{B} represents C, CH, or N,
Y^{B} represents N, NH, or C(=O),
provided that when X^{B} is N, Y^{B} is not N or NH, and
when X^{B} is C or CH, Y^{B} is not C(=O),
the double line consisting of the solid line and the broken line represents a single bond or a double bond,
Z^{B} represents an oxygen atom or a sulfur atom,
A^{B} represents a benzene ring, a pyridine ring, a thiophene ring, a pyrimidine ring, a naphthalene ring, a quinoline ring, or an indole ring, which may have 1 to 4 of the same or different substituents selected from an alkyl group having 1 to 8 carbon atoms, an alkenyl group having 2 to 8 carbon atoms, an alkoxy group having 1 to 8 carbon atoms, an alkyl group having 1 to 8 carbon atoms and substituted with 1 to 3 halogen atoms, an alkoxy group having 1 to 8 carbon atoms and substituted with 1 to 3 halogen atoms, a halogen atom, a hydroxyl group, a nitro group, a cyano group, an amino group, an alkylamino group having 1 to 8 carbon atoms, a dialkylamino group having 2 to 8 carbon atoms, an aralkyl group (the aryl moiety has 6 to 10 carbon atoms, and the alkylene moiety has 1 to 8 carbon atoms), a phenyl group, and a pyridyl group, as a substituent, or represents an atomic bond,
B^{B} represents N(R^{8B})C(=O), NHCONH, CON(R^{9B} NHC (=S) NH, N(R^{10B})SO₂, SO_{2N}(R^{11B}), or OSO₂, wherein
R^{BB}, R^{9B}, R^{10B}, and R^{11B} represent a hydrogen atom, an alkyl group having 1 to 8 carbon atoms, an alkyl group having 1 to 8 carbon atoms and substituted with 1 to 3 halogen atoms, an alkyl group having 1 to 8 carbon atoms and substituted with a hydroxyl group, or an aralkyl group (the aryl moiety has 6 to 10 carbon atoms, and the alkylene moiety has 1 to 8 carbon atoms),
D^{B} represents an alkylene chain having 1 to 6 carbon atoms, which may have 1 to 4 of the same or different substituents selected from an alkyl group having 1 to 8 carbon atoms, an alkenyl group having 2 to 8 carbon atoms, an alkyl group having 1 to 8 carbon atoms and substituted with 1 to 3 halogen atoms, an alkyl group having 1 to 8 carbon atoms and substituted with a hydroxyl group, and an aralkyl group (the aryl moiety has 6 to 10 carbon atoms, and the alkylene moiety has 1 to 8 carbon atoms), as a substituent, and may further have a double bond, or represents an atomic bond,
E^{B} represents O, S, NR^{12B}, or an atomic bond, wherein
R^{12B} represents a hydrogen atom, an alkyl group having 1 to 8 carbon atoms, an alkenyl group having 2 to 8 carbon atoms, an alkyl group having 1 to 8 carbon atoms and substituted with 1 to 3 halogen atoms, an alkyl group having 1 to 8 carbon atoms and substituted with a hydroxyl group, or an aralkyl group (the aryl moiety has 6 to 10 carbon atoms, and the alkylene moiety has 1 to 8 carbon atoms),
G^{B} represents piperazine, piperidine, morpholine, cyclohexane, benzene, naphthalene, quinoline, quinoxaline, benzimidazole, thiophene, imidazole, thiazole, oxazole, indole, benzofuran, pyrrole, pyridine, or pyrimidine, which may have 1 to 4 of the same or different substituents selected from an alkyl group having 1 to 8 carbon atoms, an alkenyl group having 2 to 8 carbon atoms, an alkoxy group having 1 to 8 carbon atoms, an alkyl group having 1 to 8 carbon atoms and substituted with 1 to 3 halogen atoms, an alkoxy group having 1 to 8 carbon atoms and substituted with 1 to 3 halogen atoms, a halogen atom, a hydroxyl group, a nitro group, a cyano group, an amino group, an alkylamino group having 1 to 8 carbon atoms, a dialkylamino group having 2 to 8 carbon atoms, an acyl group having 2 to 8 carbon atoms, a methylenedioxy group, a carboxyl group, an alkylsulfinyl group having 1 to 6 carbon atoms, an alkylthio group having 1 to 6 carbon atoms, an alkylsulfonyl group having 1 to 6 carbon atoms, an aralkyl group (the aryl moiety has 6 to 10 carbon atoms, and the alkylene moiety has 1 to 8 carbon atoms), a phenyl group which may be substituted, a pyridyl group which may be substituted, an imidazolyl group which may be substituted, an oxazolyl group which may be substituted, and a thiazolyl group which may be substituted, as a substituent, and
m^{B} represents an integer of 0 to 5,
provided that when R^{1B} and R^{2B} do not bind together to form a ring, those compounds are excluded wherein, X^{B} is C, Y^{B} is N, the double line consisting of the solid line and the broken line is a double bond, Z^{B} is an oxygen atom, A^{B} is a benzene ring, m^{B} is 0, B^{B} is C(=O)NH, E^{B} is an atomic bond, and G^{B} is a phenyl group).

(B-2) A compound represented by the following general formula (BII) or a pharmaceutically acceptable salt thereof: 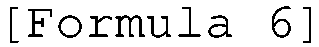 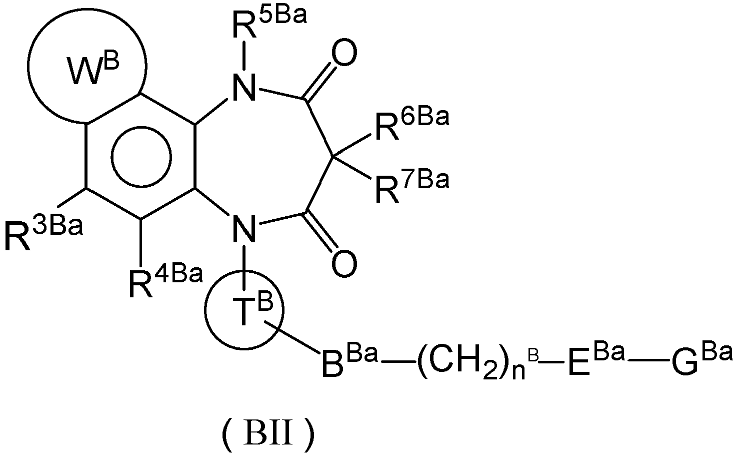 (In the formula, 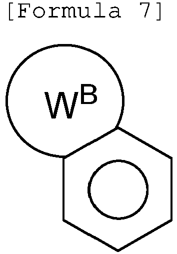
represents a naphthalene ring, a quinoline ring, an isoquinoline ring, a tetrahydronaphthalene ring, an indane ring, a tetrahydroquinoline ring, or a tetrahydroisoquinoline ring, and
these rings may be substituted with 1 to 4 of the same or different substituents selected from an alkyl group having 1 to 8 carbon atoms, an alkenyl group having 2 to 8 carbon atoms, an alkoxy group having 1 to 8 carbon atoms, an alkyl group having 1 to 8 carbon atoms and substituted with 1 to 3 halogen atoms, an alkoxy group having 1 to 8 carbon atoms and substituted with 1 to 3 halogen atoms, a halogen atom, a hydroxyl group, a nitro group, a cyano group, an amino group, an alkylamino group having 1 to 8 carbon atoms, a dialkylamino group having 2 to 8 carbon atoms, an acylamino group having 2 to 8 carbon atoms, a carboxyl group, an acyl group having 2 to 8 carbon atoms, an alkoxycarbonyl group (the alkoxy moiety has 1 to 8 carbon atoms), and an aralkyl group (the aryl moiety has 6 to 10 carbon atoms, and the alkylene moiety has 1 to 8 carbon atoms),
R^{3Ba} and R^{4Ba} may be the same or different, and represent a hydrogen atom, an alkyl group having 1 to 8 carbon atoms, an alkenyl group having 2 to 8 carbon atoms, an alkoxy group having 1 to 8 carbon atoms, an alkyl group having 1 to 8 carbon atoms and substituted with 1 to 3 halogen atoms, an alkoxy group having 1 to 8 carbon atoms and substituted with 1 to 3 halogen atoms, a halogen atom, a hydroxyl group, a nitro group, a cyano group, an amino group, an alkylamino group having 1 to 8 carbon atoms, a dialkylamino group having 2 to 8 carbon atoms, an acylamino group having 2 to 8 carbon atoms, a carboxyl group, an acyl group having 2 to 8 carbon atoms, an alkoxycarbonyl group (the alkoxy moiety has 1 to 8 carbon atoms), or an aralkyl group (the aryl moiety has 6 to 10 carbon atoms, and the alkylene moiety has 1 to 8 carbon atoms),
R^{5Ba} represents a hydrogen atom, an alkyl group having 1 to 8 carbon atoms, an alkenyl group having 2 to 8 carbon atoms, an alkyl group having 1 to 8 carbon atoms and substituted with 1 to 3 halogen atoms, an alkyl group having 1 to 8 carbon atoms and substituted with a hydroxyl group, or an aralkyl group (the aryl moiety has 6 to 10 carbon atoms, and the alkylene moiety has 1 to 8 carbon atoms),
R^{6Ba} and R^{7Ba} may be the same or different, and represent a hydrogen atom, an alkyl group having 1 to 8 carbon atoms, an alkenyl group having 2 to 8 carbon atoms, an alkoxy group having 1 to 8 carbon atoms, an alkyl group having 1 to 8 carbon atoms and substituted with 1 to 3 halogen atoms, an alkoxy group having 1 to 8 carbon atoms and substituted with 1 to 3 halogen atoms, a halogen atom, a hydroxyl group, or an amino group, 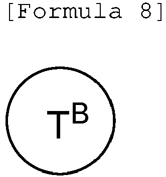
represents a benzene ring, a pyridine ring, a thiophene ring, a pyrimidine ring, a naphthalene ring, a quinoline ring, or an indole ring, which may have 1 to 4 of the same or different substituents selected from an alkyl group having 1 to 8 carbon atoms, an alkenyl group having 2 to 8 carbon atoms, an alkoxy group having 1 to 8 carbon atoms, an alkyl group having 1 to 8 carbon atoms and substituted with 1 to 3 halogen atoms, an alkoxy group having 1 to 8 carbon atoms and substituted with 1 to 3 halogen atoms, a halogen atom, a hydroxyl group, a nitro group, a cyano group, an amino group, an alkylamino group having 1 to 8 carbon atoms, a dialkylamino group having 2 to 8 carbon atoms, an aralkyl group (the aryl moiety has 6 to 10 carbon atoms, and the alkylene moiety has 1 to 8 carbon atoms), a phenyl group, and a pyridyl group, as a substituent,
B^{Ba} represents N(R^{8Ba})C(=O), NHCONH, CON(R^{9Ba}), NHC(=S)NH, N(R^{10Ba})SO₂, SO₂N(R^{11Ba}), or OSO₂, wherein
R^{8Ba}, R^{9Ba}, R^{10Ba}, and R^{11Ba} represent a hydrogen atom, an alkyl group having 1 to 8 carbon atoms, an alkyl group having 1 to 8 carbon atoms and substituted with 1 to 3 halogen atoms, an alkyl group having 1 to 8 carbon atoms and substituted with a hydroxyl group, or an aralkyl group (the aryl moiety has 6 to 10 carbon atoms, and the alkylene moiety has 1 to 8 carbon atoms),
E^{Ba} represents O, S, NR^{12Ba}, or an atomic bond, wherein
R^{12Ba} represents a hydrogen atom, an alkyl group having 1 to 8 carbon atoms, an alkenyl group having 2 to 8 carbon atoms, an alkyl group having 1 to 8 carbon atoms and substituted with 1 to 3 halogen atoms, an alkyl group having 1 to 8 carbon atoms and substituted with a hydroxyl group, or an aralkyl group (the aryl moiety has 6 to 10 carbon atoms, and the alkylene moiety has 1 to 8 carbon atoms),
G^{Ba} represents piperazine, piperidine, morpholine, cyclohexane, benzene, naphthalene, quinoline, quinoxaline, benzimidazole, thiophene, imidazole, thiazole, oxazole, indole, benzofuran, pyrrole, pyridine, or pyrimidine, which may have 1 to 4 of the same or different substituents selected from an alkyl group having 1 to 8 carbon atoms, an alkenyl group having 2 to 8 carbon atoms, an alkoxy group having 1 to 8 carbon atoms, an alkyl group having 1 to 8 carbon atoms and substituted with 1 to 3 halogen atoms, an alkoxy group having 1 to 8 carbon atoms and substituted with 1 to 3 halogen atoms, a halogen atom, a hydroxyl group, a nitro group, a cyano group, an amino group, an alkylamino group having 1 to 8 carbon atoms, a dialkylamino group having 2 to 8 carbon atoms, an acyl group having 2 to 8 carbon atoms, a methylenedioxy group, a carboxyl group, an alkylsulfinyl group having 1 to 6 carbon atoms, an alkylthio group having 1 to 6 carbon atoms, an alkylsulfonyl group having 1 to 6 carbon atoms, an aralkyl group (the aryl moiety has 6 to 10 carbon atoms, and the alkylene moiety has 1 to 8 carbon atoms), a phenyl group which may be substituted, a pyridyl group which may be substituted, an imidazolyl group which may be substituted, an oxazolyl group which may be substituted, and a thiazolyl group which may be substituted, as a substituent, and
n^{B} represents an integer of 0 to 5).

Next, the substituents in the general formulas (BI) and (BII) of the present specification is described.

Examples of the alkyl group having 1 to 8 carbon atoms can comprise a methyl group, an ethyl group, a propyl group, an isopropyl group, a butyl group, an i-butyl group, a t-butyl group, a pentyl group, a hexyl group, and the like.

Examples of the cycloalkyl group having carbon atoms 3 to 8 can comprise a cyclopropyl group, a cyclohexyl group, and the like.

Examples of the alkenyl group having 2 to 8 carbon atoms can comprise an allyl group, and the like.

Examples of the alkoxy group having 1 to 8 carbon atoms can comprise a methoxy group, an ethoxy group, a propoxy group, an isopropoxy group, a butoxy group, an i-butoxy group, a t-butoxy group, a pentyloxy group, a hexyloxy group, and the like.

Examples of the alkyl group having 1 to 8 carbon atoms and substituted with 1 to 3 halogen atoms can comprise a methyl group, an ethyl group, a propyl group, an isopropyl group, a butyl group, a t-butyl group, and the like substituted with 1 to 3 halogen atoms such as a fluorine atom, a chlorine atom, and a bromine atom, and preferable examples thereof can comprise a trifluoromethyl group, a chloromethyl group, a 2-chloroethyl group, a 2-bromoethyl group, a 2-fluoroethyl group, and the like.

Examples of the alkoxy group having 1 to 8 carbon atoms substituted with 1 to 3 halogen atoms can comprise a methoxy group, an ethoxy group, a propoxy group, an isopropoxy group, a butoxy group, a t-butoxy group, and the like substituted with 1 to 3 halogen atoms such as a fluorine atom, a chlorine atom, and a bromine atom, and preferable examples thereof can comprise a trifluoromethoxy group, a chloromethoxy group, a 2-chloroethoxy group, a 2-bromoethoxy group, a 2-fluoroethoxy group, and the like.

Examples of the halogen atom can comprise a fluorine atom, a chlorine atom, a bromine atom, and the like.

Examples of the alkylamino group having 1 to 8 carbon atoms can comprise a methylamino group, an ethylamino group, and the like.

Examples of the dialkylamino group having 2 to 8 carbon atoms can comprise a dimethylamino group, a diethylamino group, and the like.

Examples of the acylamino group having 2 to 8 carbon atoms can comprise an acetylamino group and the like.

Examples of the acyl group having 2 to 8 carbon atoms can comprise an acetyl group and the like.

Examples of the alkoxycarbonyl group (the alkoxy moiety has 1 to 8 carbon atoms) can comprise a methoxycarbonyl group and the like.

Examples of the aralkyl group (the aryl moiety has 6 to 10 carbon atoms, and the alkylene moiety has 1 to 8 carbon atoms) can comprise a benzyl group and the like.

Examples of the alkyl group having 1 to 8 carbon atoms and substituted with a hydroxyl group can comprise a 2-hydroxyethyl group and the like.

Examples of the alkylsulfinyl group having 1 to 6 carbon atoms can comprise a methanesulfinyl group and the like.

Examples of the alkylthio group having 1 to 6 carbon atoms can comprise a methylthio group and the like.

Examples of the alkylsulfonyl group having 1 to 6 carbon atoms can comprise a methanesulfonyl group and the like.

Examples of the substituent that may be comprised in the phenyl group which may be substituted, the pyridyl group which may be substituted, the imidazolyl group which may be substituted, the oxazolyl group which may be substituted, and the thiazolyl group which may be substituted can comprise a halogen atom, an alkyl group having 1 to 8 carbon atoms, an alkoxy group having 1 to 8 carbon atoms, an alkyl group having 1 to 8 carbon atoms and substituted with 1 to 3 halogen atoms, an alkoxy group having 1 to 8 carbon atoms and substituted with 1 to 3 halogen atoms, and the like.

As the compound represented by the general formula (BI), the following compounds are preferable.

### (B-1-1)

The compound according to (B-1), in which R^{1B} and R^{2B} may be the same or different, and represent a hydrogen atom, an alkyl group having 1 to 8 carbon atoms, an alkenyl group having 2 to 8 carbon atoms, an alkoxy group having 1 to 8 carbon atoms, an alkyl group having 1 to 8 carbon atoms and substituted with 1 to 3 halogen atoms, an alkoxy group having 1 to 8 carbon atoms and substituted with 1 to 3 halogen atoms, a halogen atom, a hydroxyl group, a nitro group, a cyano group, an amino group, a phenyl group which may be substituted, a pyridyl group which may be substituted, or an aralkyl group (the aryl moiety has 6 to 10 carbon atoms, and the alkylene moiety has 1 to 8 carbon atoms).

### (B-1-2)

The compound according to (B-1) or (B-1-1), in which R^{1B} and R^{2B} bind together to form a naphthalene ring or a tetrahydronaphthalene ring together with the benzene ring to which they bind, and the benzene ring or the cyclohexene ring constituted by R^{1B} and R^{2B}, bound to each other, together with the carbon atoms to which R^{1B} and R^{2B} bind may be substituted with 1 to 4 of the same or different substituents selected from an alkyl group having 1 to 8 carbon atoms, an alkenyl group having 2 to 8 carbon atoms, an alkoxy group having 1 to 8 carbon atoms, an alkyl group having 1 to 8 carbon atoms and substituted with 1 to 3 halogen atoms, an alkoxy group having 1 to 8 carbon atoms and substituted with 1 to 3 halogen atoms, a halogen atom, a hydroxyl group, a nitro group, a cyano group, an amino group, an alkylamino group having 1 to 8 carbon atoms, a dialkylamino group having 2 to 8 carbon atoms, an acylamino group having 2 to 8 carbon atoms, a carboxyl group, an acyl group having 2 to 8 carbon atoms, an alkoxycarbonyl group (the alkoxy moiety has 1 to 8 carbon atoms), and an aralkyl group (the aryl moiety has 6 to 10 carbon atoms, and the alkylene moiety has 1 to 8 carbon atoms),

### (B-1-3)

The compound according to (B-1) or (B-1-1), in which R^{1B} and R^{2B} bind together to form a naphthalene ring together with the benzene ring to which they bind, and the benzene ring constituted by R^{1B} and R^{2B}, bound to each other, together with the carbon atoms to which R^{1B} and R^{2B} bind may be substituted with 1 to 4 of the same or different substituents selected from an alkyl group having 1 to 8 carbon atoms, an alkenyl group having 2 to 8 carbon atoms, an alkoxy group having 1 to 8 carbon atoms, an alkyl group having 1 to 8 carbon atoms and substituted with 1 to 3 halogen atoms, an alkoxy group having 1 to 8 carbon atoms and substituted with 1 to 3 halogen atoms, a halogen atom, a hydroxyl group, and an amino group.

### (B-1-4)

The compound according to (B-1) or (B-1-1), in which R^{1B} and R^{2B} bind together to form a naphthalene ring or a tetrahydronaphthalene ring together with the benzene ring to which they bind.

### (B-1-5)

The compound according to any one of (B-1) and (B-1-1) to (B-1-4), in which R^{3B} and R^{4B} may be the same or different, and represent a hydrogen atom, an alkyl group having 1 to 8 carbon atoms, an alkenyl group having 2 to 8 carbon atoms, an alkoxy group having 1 to 8 carbon atoms, an alkyl group having 1 to 8 carbon atoms and substituted with 1 to 3 halogen atoms, an alkoxy group having 1 to 8 carbon atoms and substituted with 1 to 3 halogen atoms, a halogen atom, a hydroxyl group, a nitro group, a cyano group, an amino group, or an aralkyl group (the aryl moiety has 6 to 10 carbon atoms, and the alkylene moiety has 1 to 8 carbon atoms).

### (B-1-6)

The compound, the compound according to any one of (B-1) and (B-1-1) to (B-1-5), in which R^{5B} represents a hydrogen atom, an alkyl group having 1 to 8 carbon atoms, or an aralkyl group (the aryl moiety has 6 to 10 carbon atoms, and the alkylene moiety has 1 to 8 carbon atoms).

### (B-1-7)

The compound according to any one of (B-1) and (B-1-1) to (B-1-6), in which R^{5B} represents a hydrogen atom.

### (B-1-8)

The compound according to any one of (B-1) and (B-1-1) to (B-1-7), in which R^{6B} and R^{7B} may be the same or different, and represent a hydrogen atom, an alkyl group having 1 to 8 carbon atoms, an alkoxy group having 1 to 8 carbon atoms, an alkyl group having 1 to 8 carbon atoms and substituted with 1 to 3 halogen atoms, or an alkoxy group having 1 to 8 carbon atoms and substituted with 1 to 3 halogen atoms, or a pharmaceutically acceptable salt thereof.

### (B-1-9)

The compound according to any one of (B-1) and (B-1-1) to (B-1-8), in which R^{6B} and R^{7B} both represent hydrogen atoms.

### (B-1-10)

The compound according to any one of (B-1) and (B-1-1) to (B-1-9), in which R^{3B}, R^{4B}, R^{5B}, R^{6B}, and R^{7B} are hydrogen atoms.

### (B-1-11)

The compound according to any one of (B-1) and (B-1-1) to (B-1-10), in which X^{B} represents N, Y^{B} represents C(=O), and the double line consisting of the solid line and the broken line represents a single bond.

### (B-1-12)

The compound according to any one of (B-1) and (B-1-1) to (B-1-11), in which X^{B} represents C, Y^{B} represents N, and the double line consisting of the solid line and the broken line represents a double bond.

### (B-1-13)

The compound according to any one of (B-1) and (B-1-1) to (B-1-12), in which Z^{B} represents an oxygen atom.

### (B-1-14)

The compound according to any one of (B-1) and (B-1-1) to (B-1-13), in which A^{B} represents a phenyl group or a pyridyl group that may have 1 to 4 of the same or different substituents selected from an alkyl group having 1 to 8 carbon atoms, an alkenyl group having 2 to 8 carbon atoms, an alkoxy group having 1 to 8 carbon atoms, an alkyl group having 1 to 8 carbon atoms and substituted with 1 to 3 halogen atoms, an alkoxy group having 1 to 8 carbon atoms and substituted with 1 to 3 halogen atoms, a halogen atom, a hydroxyl group, a nitro group, a cyano group, an amino group, an alkylamino group having 1 to 8 carbon atoms, a dialkylamino group having 2 to 8 carbon atoms, an aralkyl group (the aryl moiety has 6 to 10 carbon atoms, and the alkylene moiety has 1 to 8 carbon atoms), a phenyl group, and a pyridyl group as a substituent.

### (B-1-15)

The compound according to any one of (B-1) and (B-1-1) to (B-1-14), in which A^{B} represents a phenyl group that may have 1 to 4 of the same or different substituents selected from an alkyl group having 1 to 8 carbon atoms, an alkenyl group having 2 to 8 carbon atoms, an alkoxy group having 1 to 8 carbon atoms, an alkyl group having 1 to 8 carbon atoms and substituted with 1 to 3 halogen atoms, an alkoxy group having 1 to 8 carbon atoms and substituted with 1 to 3 halogen atoms, a halogen atom, a hydroxyl group, a nitro group, a cyano group, and an amino group as a substituent.

### (B-1-16)

The compound according to any one of (B-1) and (B-1-1) to (B-1-15), in which A^{B} represents a phenyl group or a pyridyl group.

### (B-1-17)

The compound according to any one of (B-1) and (B-1-1) to (B-1-16), in which A^{B} represents an atomic bond.

### (B-1-18)

The compound according to any one of (B-1) and (B-1-1) to (B-1-17), in which B^{B} represents NHC(=O), NHCONH, CONH, NHC(=S)NH, NHSO₂, SO₂NH, or OSO₂.

### (B-1-19)

The compound according to any one of (B-1) and (B-1-1) to (B-1-18), in which B^{B} represents NHC(=O), NHCONH, or NHSO₂.

### (B-1-20)

The compound according to any one of (B-1) and (B-1-1) to (B-1-19), in which B^{B} represents NHC(=O) or NHSO₂.

### (B-1-21)

The compound according to any one of (B-1) and (B-1-1) to (B-1-20), in which B^{B} represents NHC(=O).

### (B-1-22)

The compound according to any one of (B-1) and (B-1-1) to (B-1-21), in which D^{B} represents an alkylene chain having 1 to 6 carbon atoms that may have 1 to 4 of the same or different substituents selected from an alkyl group having 1 to 8 carbon atoms and an alkyl group having 1 to 8 carbon atoms and substituted with 1 to 3 halogen atoms as a substituent, and may further have a double bond.

### (B-1-23)

The compound according to any one of (B-1) and (B-1-1) to (B-1-22), in which D^{B} represents an atomic bond.

### (B-1-24)

The compound according to any one of (B-1) and (B-1-1) to (B-1-23), in which D^{B} has 1 to 4 of the same or different substituents selected from an alkyl group having 1 to 8 carbon atoms and an alkenyl group having 2 to 8 carbon atoms as a substituent.

### (B-1-25)

The compound according to any one of (B-1) and (B-1-1) to (B-1-24), in which D^{B} has 1 to 4 of the same or different substituents selected from an alkyl group having 1 to 3 carbon atoms and an alkenyl group having 2 or 3 carbon atoms as a substituent.

### (B-1-26)

The compound according to any one of (B-1) and (B-1-1) to (B-1-25), in which E^{B} represents an atomic bond.

### (B-1-27)

The compound according to any one of (B-1) and (B-1-1) to (B-1-26), in which G^{B} represents piperazine, piperidine, morpholine, cyclohexane, benzene, naphthalene, quinoline, quinoxaline, benzimidazole, thiophene, imidazole, thiazole, oxazole, indole, benzofuran, pyrrole, pyridine, or pyrimidine that may have 1 to 4 of the same or different substituents selected from an alkyl group having 1 to 8 carbon atoms, an alkenyl group having 2 to 8 carbon atoms, an alkoxy group having 1 to 8 carbon atoms, an alkyl group having 1 to 8 carbon atoms and substituted with 1 to 3 halogen atoms, an alkoxy group having 1 to 8 carbon atoms and substituted with 1 to 3 halogen atoms, a halogen atom, a hydroxyl group, a nitro group, a cyano group, an amino group, an alkylamino group having 1 to 8 carbon atoms, a dialkylamino group having 2 to 8 carbon atoms, an acyl group having 2 to 8 carbon atoms, a methylenedioxy group, a carboxyl group, an alkylsulfinyl group having 1 to 6 carbon atoms, an alkylthio group having 1 to 6 carbon atoms, and an alkylsulfonyl group having 1 to 6 carbon atoms as a substituent.

### (B-1-28)

The compound according to any one of (B-1) and (B-1-1) to (B-1-27), in which G^{B} represents benzene that may have 1 to 4 of the same or different substituents selected from an alkyl group having 1 to 8 carbon atoms, an alkenyl group having 2 to 8 carbon atoms, an alkoxy group having 1 to 8 carbon atoms, an alkyl group having 1 to 8 carbon atoms and substituted with 1 to 3 halogen atoms, an alkoxy group having 1 to 8 carbon atoms and substituted with 1 to 3 halogen atoms, a halogen atom, a hydroxyl group, a nitro group, a cyano group, an amino group, an alkylamino group having 1 to 8 carbon atoms, a dialkylamino group having 2 to 8 carbon atoms, an acyl group having 2 to 8 carbon atoms, a methylenedioxy group, a carboxyl group, an alkylsulfinyl group having 1 to 6 carbon atoms, an alkylthio group having 1 to 6 carbon atoms, and an alkylsulfonyl group having 1 to 6 carbon atoms as a substituent.

### (B-1-29)

The compound according to any one of (B-1) and (B-1-1) to (B-1-28), in which G^{B} represents benzene or pyridine that may have 1 to 4 of the same or different substituents selected from an alkyl group having 1 to 8 carbon atoms, an alkenyl group having 2 to 8 carbon atoms, an alkoxy group having 1 to 8 carbon atoms, an alkyl group having 1 to 8 carbon atoms and substituted with 1 to 3 halogen atoms, a halogen atom, a hydroxyl group, a nitro group, an amino group, a dialkylamino group having 2 to 8 carbon atoms, a carboxyl group, an alkylsulfinyl group having 1 to 6 carbon atoms, an alkylthio group having 1 to 6 carbon atoms, and an alkylsulfonyl group having 1 to 6 carbon atoms as a substituent.

### (B-1-30)

The compound according to any one of (B-1) and (B-1-1) to (B-1-29), in which G^{B} represents benzene that may have 1 to 4 of the same or different substituents selected from an alkyl group having 1 to 8 carbon atoms and substituted with 1 to 3 halogen atoms, a halogen atom, and a hydroxyl group as a substituent.

### (B-1-31)

The compound according to any one of (B-1) and (B-1-1) to (B-1-30), in which m^{B} represents 0.

### (B-1-32)

The compound according to any one of (B-1) and (B-1-1) to (B-1-31), in which A^{B} represents a benzene ring, m^{B} represents 0, B^{B} represents NHC(=O) or NHSO₂, D^{B} represents an alkyl group having 1 to 3 carbon atoms or an atomic bond, E^{B} represents an atomic bond, and G^{B} represents benzene that may have 1 to 4 of the same or different substituents selected from an alkyl group having 1 to 8 carbon atoms, an alkyl group having 1 to 8 carbon atoms and substituted with 1 to 3 halogen atoms, a halogen atom, and a hydroxyl group as a substituent.

### (B-1-33)

The compound according to any one of (B-1) and (B-1-1) to (B-1-32), in which A^{B} represents a benzene ring, m^{B} represents 0, B^{B} represents NHC(=O), D^{B} represents an atomic bond, E^{B} represents an atomic bond, and G^{B} represents benzene that may have 1 to 4 of the same or different substituents selected from an alkyl group having 1 to 8 carbon atoms and substituted with 1 to 3 halogen atoms, a halogen atom, and a hydroxyl group as a substituent.

### (B-1-34)

The compound according to any one of (B-1) and (B-1-1) to (B-1-33), in which R^{1B} and R^{2B} bind together to form a naphthalene ring together with the benzene ring to which they bind, R^{3B}, R^{4B}, R^{5B}, R^{6B}, and R^{7B} represent hydrogen atoms, X^{B} represents N, Y^{B} represents C(=O), the double line consisting of the solid line and the broken line represents a single bond, Z^{B} represents an oxygen atom, A^{B} represents a benzene ring, m^{B} represents 0, B^{B} represents NHC(=O) or NHSO₂, D^{B} represents an alkyl group having 1 to 3 carbon atoms or an atomic bond, E^{B} represents an atomic bond, and G^{B} represents benzene that may have 1 to 4 of the same or different substituents selected from an alkyl group having 1 to 8 carbon atoms, an alkyl group having 1 to 8 carbon atoms and substituted with 1 to 3 halogen atoms, a halogen atom, and a hydroxyl group as a substituent.

### (B-1-35)

The compound according to any one of (B-1) and (B-1-1) to (B-1-34), in which, in the general formula (BI), R^{1B} and R^{2B} bind together to form a naphthalene ring together with the benzene ring to which they bind, R^{3B}, R^{4B}, R^{5B}, R^{6B}, and R^{7B} represent hydrogen atoms, X^{B} represents N, Y^{B} represents C(=O), the double line consisting of the solid line and the broken line represents a single bond, Z^{B} represents an oxygen atom, A^{B} represents a benzene ring, m^{B} represents 0, B^{B} represents NHC(=O), D^{B} represents an atomic bond, E^{B} represents an atomic bond, and G^{B} represents benzene that may have 1 to 4 of the same or different substituents selected from an alkyl group having 1 to 8 carbon atoms and substituted with 1 to 3 halogen atoms, a halogen atom, and a hydroxyl group as a substituent.

As the compound represented the general formula (BII), the following compounds are preferable.

### (B-2-1)

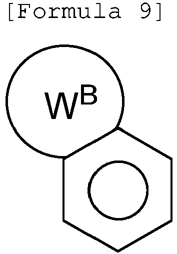

The compound according to (B-2) in which the above moiety represents a naphthalene ring or a tetrahydronaphthalene ring that may have 1 to 4 of the same or different substituents selected from an alkyl group having 1 to 8 carbon atoms, an alkenyl group having 2 to 8 carbon atoms, an alkoxy group having 1 to 8 carbon atoms, an alkyl group having 1 to 8 carbon atoms and substituted with 1 to 3 halogen atoms, an alkoxy group having 1 to 8 carbon atoms and substituted with 1 to 3 halogen atoms, a halogen atom, a hydroxyl group, a nitro group, a cyano group, an amino group, an alkylamino group having 1 to 8 carbon atoms, a dialkylamino group having 2 to 8 carbon atoms, an acylamino group having 2 to 8 carbon atoms, a carboxyl group, an acyl group having 2 to 8 carbon atoms, an alkoxycarbonyl group (the alkoxy moiety has 1 to 8 carbon atoms), and an aralkyl group (the aryl moiety has 6 to 10 carbon atoms, and the alkylene moiety has 1 to 8 carbon atoms) as a substituent.

### (B-2-2)

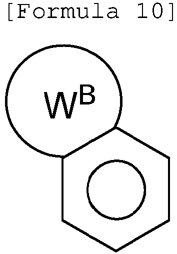

The compound according to (B-2) or (B-2-1) in which the above moiety represents a naphthalene ring that may have 1 to 4 of the same or different substituents selected from an alkyl group having 1 to 8 carbon atoms, an alkenyl group having 2 to 8 carbon atoms, an alkoxy group having 1 to 8 carbon atoms, an alkyl group having 1 to 8 carbon atoms and substituted with 1 to 3 halogen atoms, an alkoxy group having 1 to 8 carbon atoms and substituted with 1 to 3 halogen atoms, a halogen atom, a hydroxyl group, and an amino group as a substituent.

### (B-2-3)

The compound according to any one of (B-2), (B-2-1), and (B-2-2) in which R^{3Ba} and R^{4Ba} may be the same or different, and represent a hydrogen atom, an alkyl group having 1 to 8 carbon atoms, an alkenyl group having 2 to 8 carbon atoms, an alkoxy group having 1 to 8 carbon atoms, an alkyl group having 1 to 8 carbon atoms and substituted with 1 to 3 halogen atoms, an alkoxy group having 1 to 8 carbon atoms and substituted with 1 to 3 halogen atoms, a halogen atom, a hydroxyl group, a nitro group, a cyano group, an amino group, or an aralkyl group (the aryl moiety has 6 to 10 carbon atoms, and the alkylene moiety has 1 to 8 carbon atoms).

### (B-2-4)

The compound according to any one of (B-2) and (B-2-1) to (B-2-3), in which R^{5Ba} represents a hydrogen atom, an alkyl group having 1 to 8 carbon atoms, or an aralkyl group (the aryl moiety has 6 to 10 carbon atoms, and the alkylene moiety has 1 to 8 carbon atoms).

### (B-2-5)

The compound according to any one of (B-2) and (B-2-1) to (B-2-4), in which R^{5Ba} represents a hydrogen atom.

### (B-2-6)

The compound according to any one of (B-2) and (B-2-1) to (B-2-5), in which R^{6Ba} and R^{7Ba} may be the same or different, and represent a hydrogen atom, an alkyl group having 1 to 8 carbon atoms, an alkoxy group having 1 to 8 carbon atoms, an alkyl group having 1 to 8 carbon atoms and substituted with 1 to 3 halogen atoms, or an alkoxy group having 1 to 8 carbon atoms and substituted with 1 to 3 halogen atoms.

### (B-2-7)

The compound according to any one of (B-2) and (B-2-1) to (B-2-6), in which R^{6Ba} and R^{7Ba} both represent hydrogen atoms.

### (B-2-8)

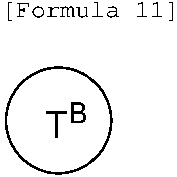

The compound according to any one of (B-2) and (B-2-1) to (B-2-7), in which the above moiety represents a phenyl group or a pyridyl group that may have 1 to 4 of the same or different substituents selected from an alkyl group having 1 to 8 carbon atoms, an alkenyl group having 2 to 8 carbon atoms, an alkoxy group having 1 to 8 carbon atoms, an alkyl group having 1 to 8 carbon atoms and substituted with 1 to 3 halogen atoms, an alkoxy group having 1 to 8 carbon atoms and substituted with 1 to 3 halogen atoms, a halogen atom, a hydroxyl group, a nitro group, a cyano group, an amino group, an alkylamino group having 1 to 8 carbon atoms, a dialkylamino group having 2 to 8 carbon atoms, an aralkyl group (the aryl moiety has 6 to 10 carbon atoms, and the alkylene moiety has 1 to 8 carbon atoms), a phenyl group, and a pyridyl group as a substituent.

### (B-2-9)

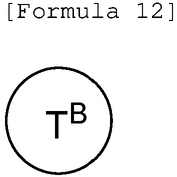

The compound according to any one of (B-2) and (B-2-1) to (B-2-8), in which the above moiety represents a phenyl group that may have 1 to 4 of the same or different substituents selected from an alkyl group having 1 to 8 carbon atoms, an alkenyl group having 2 to 8 carbon atoms, an alkoxy group having 1 to 8 carbon atoms, an alkyl group having 1 to 8 carbon atoms and substituted with 1 to 3 halogen atoms, an alkoxy group having 1 to 8 carbon atoms and substituted with 1 to 3 halogen atoms, a halogen atom, a hydroxyl group, a nitro group, a cyano group, and an amino group as a substituent.

### (B-2-10)

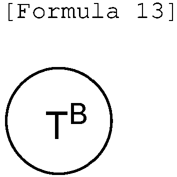

The compound according to any one of (B-2) and (B-2-1) to (B-2-9), in which the above moiety represents an atomic bond.

### (B-2-11)

The compound according to any one of (B-2) and (B-2-1) to (B-2-10), in which B^{Ba} represents NHC(=O), NHCONH, CONH, NHC (=S) NH, NHSO₂, SO₂NH, or OSO₂.

### (B-2-12)

The compound according to any one of (B-2) and (B-2-1) to (B-2-11), in which B^{Ba} represents NHC(=O), NHCONH, or NHSO₂.

### (B-2-13)

The compound according to any one of (B-2) and (B-2-1) to (B-2-12), in which E^{Ba} represents an atomic bond.

### (B-2-14)

The compound according to any one of (B-2) and (B-2-1) to (B-2-13), in which G^{Ba} represents piperazine, piperidine, morpholine, cyclohexane, benzene, naphthalene, quinoline, quinoxaline, benzimidazole, thiophene, imidazole, thiazole, oxazole, indole, benzofuran, pyrrole, pyridine, or pyrimidine that may have 1 to 4 of the same or different substituents selected from an alkyl group having 1 to 8 carbon atoms, an alkenyl group having 2 to 8 carbon atoms, an alkoxy group having 1 to 8 carbon atoms, an alkyl group having 1 to 8 carbon atoms and substituted with 1 to 3 halogen atoms, an alkoxy group having 1 to 8 carbon atoms and substituted with 1 to 3 halogen atoms, a halogen atom, a hydroxyl group, a nitro group, a cyano group, an amino group, an alkylamino group having 1 to 8 carbon atoms, a dialkylamino group having 2 to 8 carbon atoms, an acyl group having 2 to 8 carbon atoms, a methylenedioxy group, a carboxyl group, an alkylsulfinyl group having 1 to 6 carbon atoms, an alkylthio group having 1 to 6 carbon atoms, and an alkylsulfonyl group having 1 to 6 carbon atoms as a substituent.

### (B-2-15)

The compound according to any one of (B-2) and (B-2-1) to (B-2-14), in which G^{Ba} represents benzene that may have 1 to 4 of the same or different substituents selected from an alkyl group having 1 to 8 carbon atoms, an alkenyl group having 2 to 8 carbon atoms, an alkoxy group having 1 to 8 carbon atoms, an alkyl group having 1 to 8 carbon atoms and substituted with 1 to 3 halogen atoms, an alkoxy group having 1 to 8 carbon atoms and substituted with 1 to 3 halogen atoms, a halogen atom, a hydroxyl group, a nitro group, a cyano group, an amino group, an alkylamino group having 1 to 8 carbon atoms, a dialkylamino group having 2 to 8 carbon atoms, an acyl group having 2 to 8 carbon atoms, a methylenedioxy group, a carboxyl group, an alkylsulfinyl group having 1 to 6 carbon atoms, an alkylthio group having 1 to 6 carbon atoms, and an alkylsulfonyl group having 1 to 6 carbon atoms as a substituent.

### (B-2-16)

The compound according to any one of (B-2) and (B-2-1) to (B-2-15), in which n^{B} represents 0.

In the general formula (BI), it is preferable that R^{B1} and R^{B2} bind together to form a condensed ring selected from a naphthalene ring or a tetrahydronaphthalene ring together with the benzene ring to which they bind, and it is particularly preferable that R^{B1} and R^{B2} bind together to form a naphthalene ring together with the benzene ring to which they bind.

In the general formula (BI), it is preferable that R^{B3}, R^{B4}, R^{B5}, R^{B6}, and R^{B7} represent hydrogen atoms.

In the general formula (BI), it is preferable that X^{B} represents N, Y^{B} represents C(=O), and the double line consisting of the solid line and the broken line represents a single bond.

In the general formula (BI), it is preferable that Z^{B} represents an oxygen atom.

In the general formula (BI), it is preferable that A^{B} represents a benzene ring or a pyridine ring, and it is particularly preferable that A^{B} represents a benzene ring.

In the general formula (BI), it is preferable that m^{B} represents 0 to 4, and it is particularly preferable that m^{B} represents 0.

In the general formula (BI), it is preferable that B^{B} represents N(R^{8B})C(=O) or N(R^{10B})SO₂, and in this case, it is more preferable that R^{8B} and R^{10B} represent hydrogen atoms. Furthermore, in the general formula (BI), it is particularly preferable that B^{B} represents NHC(=O).

In the general formula (BI), it is preferable that D^{B} represents an alkylene chain which has 1 to 4 of the same or different substituents selected from an alkyl group having 1 to 8 carbon atoms and an alkenyl group having 2 to 8 carbon atoms as a substituent, or represents an atomic bond, it is more preferable that D^{B} represents an alkylene chain which has 1 to 4 of the same or different substituents selected from an alkyl group having 1 to 3 carbon atoms and an alkenyl group having 2 or 3 carbon atoms as a substituent, or represents an atomic bond, and it is particularly preferable that D^{B} represents an atomic bond.

In the general formula (BI), it is preferable that E^{B} represents O or an atomic bond, and it is particularly preferable that E^{B} represents an atomic bond.

In the general formula (BI), it is preferable that G^{B} represents benzene or pyridine which may have 1 to 4 of the same or different substituents selected from an alkyl group having 1 to 8 carbon atoms, an alkenyl group having 2 to 8 carbon atoms, an alkoxy group having 1 to 8 carbon atoms, an alkyl group having 1 to 8 carbon atoms and substituted with 1 to 3 halogen atoms, a halogen atom, a hydroxyl group, a nitro group, an amino group, a dialkylamino group having 2 to 8 carbon atoms, a carboxyl group, an alkylsulfinyl group having 1 to 6 carbon atoms, an alkylthio group having 1 to 6 carbon atoms, and an alkylsulfonyl group having 1 to 6 carbon atoms as a substituent, and it is particularly preferable that G^{B} represents benzene which may have 1 to 4 of the same or different substituents selected from an alkyl group having 1 to 8 carbon atoms and substituted with 1 to 3 halogen atoms, a halogen atom, and a hydroxyl group as a substituent.

In the general formula (BI), it is particularly preferable that A^{B} represents a benzene ring, m^{B} represents 0, B^{B} represents NHC(=O) or NHSO₂, D^{B} represents an alkyl group having 1 to 3 carbon atoms or an atomic bond, E^{B} represents an atomic bond, and G^{B} represents benzene which may have 1 to 4 of the same or different substituents selected from an alkyl group having 1 to 8 carbon atoms, an alkyl group having 1 to 8 carbon atoms and substituted with 1 to 3 halogen atoms, a halogen atom, and a hydroxyl group as a substituent.

In the general formula (BI), it is particularly preferable that A^{B} represents a benzene ring, m^{B} represents 0, B^{B} represents NHC(=O), D^{B} represents an atomic bond, E^{B} represents an atomic bond, and G^{B} represents benzene which may have 1 to 4 of the same or different substituents selected from an alkyl group having 1 to 8 carbon atoms and substituted with 1 to 3 halogen atoms, a halogen atom, and a hydroxyl group as a substituent.

In the general formula (BI), it is more preferable that R^{B1} and R^{B2} bind together to form a naphthalene ring together with the benzene ring to which they bind, R^{B3}, R^{B4}, R^{B5}, R^{B6}, and R^{B7} represent hydrogen atoms, X^{B} represents N, Y^{B} represents C(=O), the double line consisting of the solid line and the broken line represents a single bond, Z^{B} represents an oxygen atom, A^{B} represents a benzene ring, m^{B} represents 0, B^{B} represents NHC(=O) or NHSO₂, D^{B} represents an alkyl group having 1 to 3 carbon atoms or an atomic bond, E^{B} represents an atomic bond, and G^{B} represents benzene which may have 1 to 4 of the same or different substituents selected from an alkyl group having 1 to 8 carbon atoms, an alkyl group having 1 to 8 carbon atoms and substituted with 1 to 3 halogen atoms, a halogen atom, and a hydroxyl group as a substituent.

In the general formula (BI), it is particularly preferable that R^{B1} and R^{B2} bind together to form a naphthalene ring together with the benzene ring to which they bind, R^{B3}, R^{B4}, R^{B5}, R^{B6}, and R^{B7} represent hydrogen atoms, X^{B} represents N, Y^{B} represents C(=O), the double line consisting of the solid line and the broken line represents a single bond, Z^{B} represents an oxygen atom, A^{B} represents a benzene ring, m^{B} represents 0, B^{B} represents NHC(=O), D^{B} represents an atomic bond, E^{B} represents an atomic bond, and G^{B} represents benzene which may have 1 to 4 of the same or different substituents selected from an alkyl group having 1 to 8 carbon atoms and substituted with 1 to 3 halogen atoms, a halogen atom, and a hydroxyl group as a substituent.

Typical compounds comprised in the compounds represented by the general formulas (BI) and/or (BII) are as follows.

### <Typical Compound Example B-100>

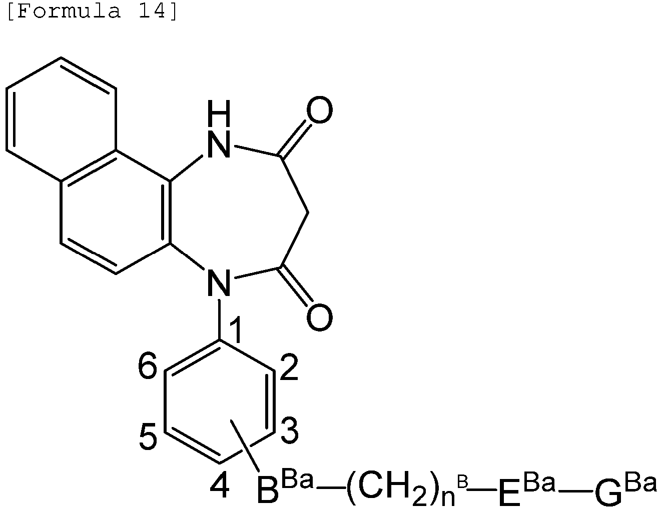

(B^{Ba} (substitution position), n^{B}, E^{Ba}, and G^{Ba} in the formula are indicated in Tables 8 to 17)

**[Table 8]**

| B^{Ba} (substitution position) | n^{B} | E^{Ba} | G^{Ba} |
|---|---|---|---|
| NHCO(4) | 0 | Atomic bond | Phenyl |
| NHCO(4) | 0 | Atomic bond | (2-CF₃)Phenyl |
| NHCO(4) | 0 | Atomic bond | (3-Br)Phenyl |
| NHCO(4) | 0 | Atomic bond | (4-CF₃)Phenyl |
| NHCO(4) | 0 | Atomic bond | (2-Me)Phenyl |
| NHCO(4) | 0 | Atomic bond | (2,6-Me)Phenyl |
| NHCO(4) | 0 | Atomic bond | (2, 6-Cl)Phenyl |
| NHCO(4) | 0 | Atomic bond | (3-Cl)Phenyl |
| NHCO(4) | 1 | Atomic bond | Phenyl |
| NHC(=S)NH(4) | 0 | Atomic bond | Phenyl |
| NHCO(4) | 0 | Atomic bond | (2,3-OMe)Phenyl |
| NHCO(4) | 0 | Atomic bond | (2-OMe)Phenyl |
| NHCO(4) | 1 | Atomic bond | (2-Cl)Phenyl |
| NHCO(4) | 0 | Atomic bond | (2,3-Me)Phenyl |
| NHCO(4) | 0 | Atomic bond | (2,5-Me)Phenyl |
| NHCO(4) | 0 | Atomic bond | (2-Cl,5-Br)Phenyl |

**[Table 9]**

| B^{Ba} (substitution position) | n^{B} | E^{Ba} | G^{Ba} |
|---|---|---|---|
| NHCO(4) | 0 | Atomic bond | (2,4-Cl)Phenyl |
| NHCO(4) | 0 | Atomic bond | (2-OH)Phenyl |
| NHCO(4) | 0 | Atomic bond | (2,3-OH)Phenyl |
| NHC(=O)NH(4) | 0 | Atomic bond | Phenyl |
| NHCO(4) | 1 | Atomic bond | (2, 6-Cl) Phenyl |
| NHCO(4) | 1 | Atomic bond | (2-OMe)Phenyl |
| NHCO(4) | 1 | Atomic bond | (2-OH)Phenyl |
| NHC (=S) NH (4) | 0 | Atomic bond | (2-Cl)Phenyl |
| NHCO(4) | 0 | Atomic bond | (3-CF₃)Phenyl |
| NHCO(4) | 1 | Atomic bond | (2-CF₃)Phenyl |
| NHC(=O)NH(4) | 0 | Atomic bond | (2-Cl)Phenyl |
| NHCO(4) | 0 | Atomic bond | (2-Cl,3-OMe)Phenyl |
| NHCO(4) | 2 | Atomic bond | Phenyl |
| NHCO(4) | 0 | Atomic bond | 3-indolyl |
| NHCO(4) | 0 | Atomic bond | (2-Cl,3-OH) Phenyl |
| NHCO(4) | 1 | O | Phenyl |

**[Table 10]**

| B^{Ba} (substitution position) | n^{B} | E^{Ba} | G^{Ba} |
|---|---|---|---|
| NHCO(4) | 1 | Atomic bond | (2-Cl,4-OMe)Phenyl |
| NHCO(4) | 0 | Atomic bond | (1-Me) imidazol 2-yl |
| NHCO(4) | 1 | Atomic bond | (2,4-Cl)Phenyl |
| NHCO(4) | 1 | Atomic bond | (2-Cl,4-OH) Phenyl |
| NHCO(4) | 1 | Atomic bond | pyridin 3-yl |
| NHCO(4) | 0 | Atomic bond | Benzimidazol 2-yl |
| NHCO(4) | 0 | Atomic bond | (2-Cl)Phenyl |
| NHCO(4) | 0 | Atomic bond | (2-Br)Phenyl |
| NHCO(4) | 0 | Atomic bond | (2-I)Phenyl |
| NHCO(4) | 1 | Atomic bond | (2-Me)Phenyl |
| NHCO(4) | 0 | Atomic bond | quinoxalin 2-yl |
| NHCO(4) | 0 | Atomic bond | (5-Me) thiophen 2-yl |
| NHCO (3) | 1 | Atomic bond | (2-Cl)Phenyl |
| NHCO(4) | 0 | Atomic bond | (2,4,6-Me)Phenyl |
| NHCO(4) | 0 | Atomic bond | (2-Et) Phenyl |
| NHC (=S) NH (4) | 0 | Atomic bond | (2-Me)Phenyl |

**[Table 11]**

| B^{Ba} (substitution position) | n^{B} | E^{Ba} | G^{Ba} |
|---|---|---|---|
| NHCO(4) | 0 | Atomic bond | (4-NMe₂)Phenyl |
| NHCO(4) | 1 | O | (2,4-Cl)Phenyl |
| NHCO(4) | 1 | O | (2-Me)Phenyl |
| NHCO(4) | 0 | Atomic bond | (2-Ac)Phenyl |
| NHCO(4) | 0 | Atomic bond | (2-tBu)Phenyl |
| NHCO (3) | 0 | Atomic bond | (2-I)Phenyl |
| NHCO(4) | 0 | Atomic bond | (1-Me)piperidin 4-yl |
| NHCO(4) | 0 | Atomic bond | benzofuran 2-yl |
| NHCO(4) | 0 | Atomic bond | (1-Me)indol 3-yl |
| NHCO(4) | 0 | Atomic bond | (2-allyl)Phenyl |
| NHCO(4) | 0 | Atomic bond | (2-nPr)Phenyl |
| NHCO(4) | 0 | Atomic bond | (2-iPrO)Phenyl |
| NHCO(4) | 0 | Atomic bond | 3-Me thiophen 2-yl |
| NHCO(4) | 1 | O | (2-Me,3-Cl)Phenyl |
| NHCO(4) | 0 | Atomic bond | (2-CF₃,4-F)Phenyl |
| NHCO(4) | 0 | Atomic bond | (2-OMe,4-F)Phenyl |

**[Table 12]**

| B^{Ba} (substitution position) | n^{B} | E^{Ba} | G^{Ba} |
|---|---|---|---|
| NHCO(4) | 0 | Atomic bond | (2-OH, 4-F) Phenyl |
| NHCO (3) | 1 | Atomic bond | (2-I)Phenyl |
| NHCO(4) | 0 | Atomic bond | (3-NMe₂)Phenyl |
| NHCO(4) | 0 | Atomic bond | (2-OMe,4-I)Phenyl |
| NHCO(4) | 0 | Atomic bond | (2-OMe, 6-F) Phenyl |
| NHCO(4) | 0 | Atomic bond | (2-OH,4-I)Phenyl |
| NHCO(4) | 0 | Atomic bond | (2-OH, 6-F)Phenyl |
| NHCO(4) | 0 | Atomic bond | (2-F) Phenyl |
| NHCO(4) | 0 | Atomic bond | (2-NMe₂)Phenyl |
| NHCO(4) | 0 | Atomic bond | (2-OMe,6-Me)Phenyl |
| NHCO(4) | 0 | Atomic bond | (2-OH, 6-Me) Phenyl |
| NHCO(4) | 2 | Atomic bond | (2-Me)Phenyl |
| CONH(4) | 0 | Atomic bond | Phenyl |
| CONH(4) | 1 | Atomic bond | Phenyl |
| NHCO(4) | 2 | Atomic bond | (2-Cl)Phenyl |
| CONH(4) | 1 | Atomic bond | (2-Cl)Phenyl |

**[Table 13]**

| B^{Ba} (substitution position) | n^{B} | E^{Ba} | G^{Ba} |
|---|---|---|---|
| CONH(4) | 0 | Atomic bond | (2-Cl) Phenyl |
| NHCO(4) | 0 | Atomic bond | (5-Br, 2, 3-methylenedioxy)Phenyl |
| NHCO(4) | 0 | Atomic bond | (2-OMe,6-Br)Phenyl |
| NHCO(4) | 0 | Atomic bond | (2-OH, 6-Br)Phenyl |
| NHCO(4) | 0 | Atomic bond | (2-OMe,6-Cl)Phenyl |
| NHCO(4) | 0 | Atomic bond | (2-OH, 6-Cl) Phenyl |
| NHCO(4) | 0 | Atomic bond | (2-OH,6-OMe)Phenyl |
| NHCO(4) | 0 | Atomic bond | (2-OMe,6-CF₃)Phenyl |
| NHCO(4) | 0 | Atomic bond | (2-OH, 6-CF₃)Phenyl |
| NHCO(4) | 0 | Atomic bond | (2-Cl,5-SMe)Phenyl |
| NHCO(4) | 0 | Atomic bond | (2-SMe)Phenyl |
| NHCO(4) | 0 | Atomic bond | (3-SMe)Phenyl |
| NHCO(4) | 0 | Atomic bond | (2-OMe,6-Et)Phenyl |
| NHCO(4) | 0 | Atomic bond | (3-SO₂Me)Phenyl |
| NHCO(4) | 0 | Atomic bond | (2-OH, 6-Et)Phenyl |
| NHCO(4) | 0 | Atomic bond | (3-S(=O)Me)Phenyl |

**[Table 14]**

| B^{Ba} (substitution position) | n^{B} | E^{Ba} | G^{Ba} |
|---|---|---|---|
| NHCO(4) | 0 | Atomic bond | (2-Cl,5-S (=O) Me) Phenyl |
| NHCO(4) | 0 | Atomic bond | (2-S (=O) Me) Phenyl |
| NHCO(4) | 0 | Atomic bond | (3-Cl) pyridin 2-yl |
| NHCO(4) | 0 | Atomic bond | (2-OMe,3-Cl)Phenyl |
| NHCO(4) | 0 | Atomic bond | (3-Me) pyridin 2-yl |
| NHCO(4) | 0 | Atomic bond | (2-OH,3-Cl)Phenyl |
| NHCO(4) | 0 | Atomic bond | (3-OH) pyridin 2-yl |
| NHCO(4) | 0 | Atomic bond | (3-Vinyl) pyridin 2-yl |
| NHCO (4) | 0 | Atomic bond | (2-Et) pyridin 2-yl |
| NHSO₂(4) | 0 | Atomic bond | (2-NO₂)Phenyl |
| NHSO₂(4) | 0 | Atomic bond | Phenyl |
| NHSO₂(4) | 0 | Atomic bond | (3-Br)Phenyl |
| NHSO₂(4) | 0 | Atomic bond | (3-OMe)Phenyl |
| NHSO₂(3) | 0 | Atomic bond | (2-NO₂)Phenyl |
| NMeSO₂(3) | 0 | Atomic bond | (2-NO₂)Phenyl |
| NHSO₂(3) | 0 | Atomic bond | naphthalen 2-yl |

**[Table 15]**

| B^{Ba} (substitution position) | n^{B} | E^{Ba} | G^{Ba} |
|---|---|---|---|
| NHSO₂(3) | 0 | Atomic bond | naphthalen 1-yl |
| NHSO₂(4) | 0 | Atomic bond | Cyclohexyl |
| NHSO₂(4) | 0 | Atomic bond | pyridin 3-yl |
| NHSO₂(4) | 0 | Atomic bond | (4-iPr)Phenyl |
| NHSO₂(4) | 1 | Atomic bond | Phenyl |
| NHSO₂(4) | 0 | Atomic bond | thiophen 2-yl |
| NHSO₂(4) | 0 | Atomic bond | naphthalen 2-yl |
| NBnSO₂(4) | 0 | Atomic bond | (2-NO₂)Phenyl |
| NMeSO₂(4) | 0 | Atomic bond | (3-Br)Phenyl |
| NMeSO₂(4) | 0 | Atomic bond | (2-NO₂)Phenyl |
| N(CH₂CH₂OH)SO₂(4) | 0 | Atomic bond | (2-NO₂)Phenyl |
| NHSO₂(4) | 1 | Atomic bond | (2-Cl)Phenyl |
| NHSO₂(4) | 1 | Atomic bond | (3-Br)Phenyl |
| NHSO₂(4) | 0 | Atomic bond | (2-CF₃)Phenyl |
| NHSO₂(4) | 1 | Atomic bond | (2-Br)Phenyl |
| NHSO₂(4) | 1 | Atomic bond | (2-Me)Phenyl |

**[Table 16]**

| B^{Ba} (substitution position) | n^{B} | E^{Ba} | G^{Ba} |
|---|---|---|---|
| NHSO₂(4) | 1 | Atomic bond | (2-NO₂)Phenyl |
| NHSO₂(4) | 2 | Atomic bond | Phenyl |
| NHSO₂(4) | 1 | Atomic bond | (4-Cl)Phenyl |
| NMeSO₂(4) | 1 | Atomic bond | (2-CF₃)Phenyl |
| NMeSO₂(4) | 1 | Atomic bond | (2-Et) Phenyl |
| NMeSO₂(4) | 1 | Atomic bond | (2, 3-Me)Phenyl |
| NMeSO₂(4) | 2 | Atomic bond | (2-Cl)Phenyl |
| NMeSO₂(4) | 1 | Atomic bond | (2-NO₂)Phenyl |
| NMeSO₂(4) | 1 | Atomic bond | (2-NH₂)Phenyl |
| NMeSO₂(4) | 1 | Atomic bond | (2-NMe₂)Phenyl |

**[Table 17]**

| B^{Ba} (substitution position) | n^{B} | E^{Ba} | G^{Ba} |
|---|---|---|---|
| NHCO(4) | 0 | Atomic bond | Pyridin 4-yl |
| NHCO(4) | 1 | O | Pyridin 3-yl |
| NHCO(4) | 0 | Atomic bond | Pyridin 3-yl |
| NHCO(4) | 0 | Atomic bond | (2-Me)Pyridin 3-yl |
| NHCO(4) | 0 | Atomic bond | (2-Cl)Pyridin 3-yl |
| NHCO (4) | 1 | O | Pyridin 2-yl |
| NHCO(4) | 0 | Atomic bond | (4-CF₃)Pyridin 3-yl |
| NHCO(4) | 0 | Atomic bond | (2-iPr)Phenyl |

### <Typical Compound Example B-200>

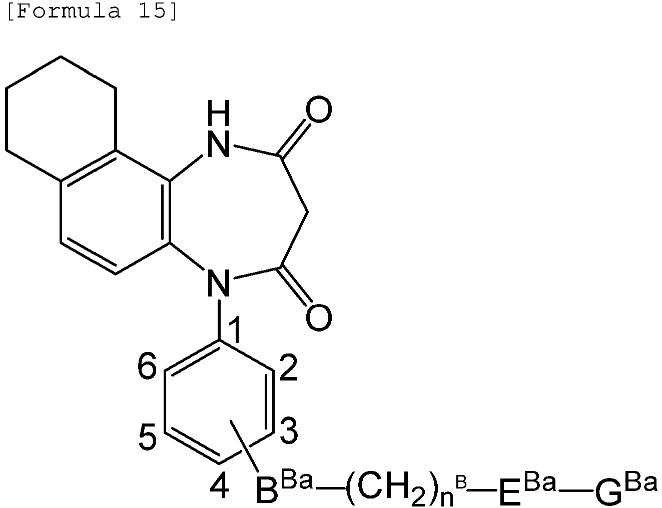

(B^{Ba} (substitution position), n^{B}, E^{Ba}, and G^{Ba} in the formula are indicated in Tables 18 and 19)

**[Table 18]**

| B^{Ba}(substitution position) | n^{B} | E^{Ba} | G^{Ba} |
|---|---|---|---|
| NHCO(4) | 0 | Atomic bond | Cyclohexyl |
| NHCO(4) | 0 | Atomic bond | (6-Me) pyridin-2-yl |
| NHCO(4) | 0 | Atomic bond | (2-Me)pyridin-3-yl |
| NHCO(4) | 0 | Atomic bond | (2-OMe,3-Me)Phenyl |
| NHCO(4) | 0 | Atomic bond | (2,3-Cl)Phenyl |
| NHCO (4) | 0 | Atomic bond | (2-OH, 3-Me) Phenyl |
| NHCO(4) | 0 | Atomic bond | (2-I)Phenyl |
| NHCO(4) | 1 | Atomic bond | (1-Me)pyrrol 2-yl |
| NHCO(4) | 1 | Atomic bond | (2-tBu)Phenyl |
| NHCO(4) | 0 | Atomic bond | (2-Isopropenyl) phenyl |
| NHCO (4) | 0 | Atomic bond | (2-iPr)Phenyl |
| NHCO(4) | 1 | Atomic bond | morpholin 2-yl |
| NHCO(4) | 0 | Atomic bond | (2-Cl)pyridin 2-yl |

**[Table 19]**

| B^{Ba} (substitution position) | n^{B} | E^{Ba} | G^{Ba} |
|---|---|---|---|
| NHSO₂(4) | 0 | Atomic bond | (2-NO₂)Phenyl |
| NMeSO₂(4) | 0 | Atomic bond | (2-NO₂)Phenyl |
| SO₂NH(4) | 0 | Atomic bond | Phenyl |
| OSO₂(4) | 0 | Atomic bond | (3-Br)Phenyl |
| NHSO₂(4) | 1 | Atomic bond | (2-Cl)Phenyl |
| NHSO₂(4) | 0 | Atomic bond | (3-Br)Phenyl |
| NHSO₂(4) | 0 | Atomic bond | (3-OMe)Phenyl |
| NHSO₂(4) | 1 | Atomic bond | (2,3-Cl)Phenyl |
| NHSO₂(4) | 1 | Atomic bond | (2, 6-Cl) Phenyl |
| NHSO₂(4) | 1 | Atomic bond | (2-I)Phenyl |
| NMeSO₂(4) | 1 | Atomic bond | (2-Cl)Phenyl |

### <Typical Compound Example B-300>

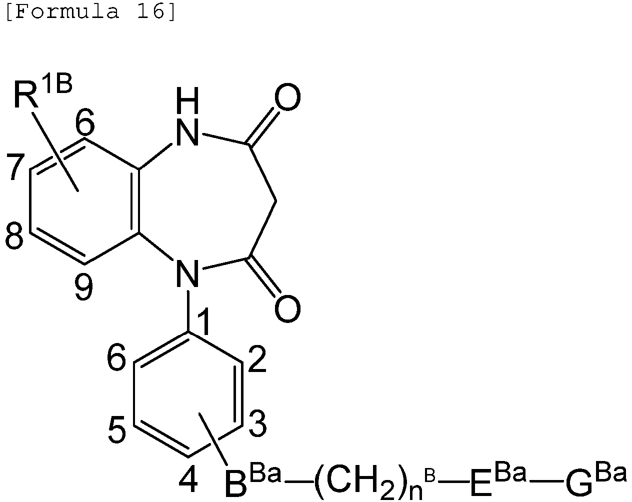

(R^{1B}, B^{Ba} (substitution position), n^{B}, E^{Ba}, and G^{Ba} in the formula are indicated in Table 20)

**[Table 20]**

| R^{1B} | B^{Ba} (substitution position) | n^{B} | E^{Ba} | G^{Ba} |
|---|---|---|---|---|
| 7-OMe | NHCO(4) | 0 | Atomic bond | (2,3-Me)Phenyl |
| 7-OH | NHCO(4) | 0 | Atomic bond | (2,3-Me)Phenyl |
| 6-Me | NHCO(4) | 0 | Atomic bond | (2,3-Me)Phenyl |
| 6, 7-Me | NHCO(4) | 0 | Atomic bond | (2-I)Phenyl |
| 6-Et | NHCO(4) | 0 | Atomic bond | (2-I)Phenyl |
| 7-Ph | NHCO(4) | 0 | Atomic bond | (2-Isopropyl))Phenyl |
| 7-(Pyridin-3yl) | NHCO(4) | 0 | Atomic bond | (2-Isopropyl))Phenyl |
| 7-(Pyridin-2yl) | NHCO(4) | 0 | Atomic bond | (2-Isopropyl) Phenyl |
| 7-Cl | NHSO₂(4) | 0 | Atomic bond | (2-Isopropyl) Phenyl |
| 7-Br | NHSO₂(4) | 0 | Atomic bond | (2-Isopropyl) Phenyl |
| 7-CF₃ | NHSO₂(4) | 0 | Atomic bond | (2-Isopropyl) Phenyl |
| H | NHSO₂(4) | 0 | Atomic bond | (2-Isopropyl) Phenyl |
| 6-Me, 7-Br | NHSO₂(4) | 0 | Atomic bond | (2-Isopropyl) Phenyl |
| 7-OMe | NHSO₂(4) | 1 | Atomic bond | (2-Cl)Phenyl |
| 7-OH | NHSO₂(4) | 1 | Atomic bond | (2-Cl)Phenyl |
| 6-Me | NHSO₂(4) | 1 | Atomic bond | (2-Cl)Phenyl |

### <Typical Compound Example B-400>

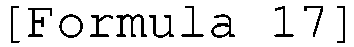

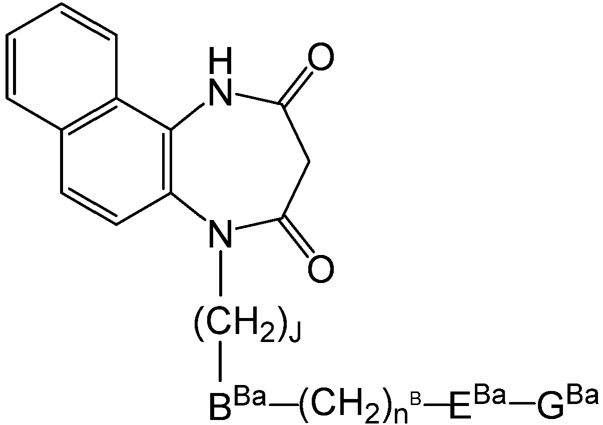

(B^{Ba} (substitution position), n^{B}, E^{Ba}, and G^{Ba} in the formula are indicated in Table 21)

**[Table 21]**

| B^{Ba} (substitution position) | n^{B} | E^{Ba} | G^{Ba} |
|---|---|---|---|
| NHCO | 0 | Atomic bond | (2-Cl,3-OMe)Phenyl |
| NHCO | 0 | Atomic bond | (2-I)Phenyl |
| NHSO₂ | 1 | Atomic bond | (2-Cl)Phenyl |
| NHSO₂ | 1 | Atomic bond | (2-Cl)Phenyl |

### <Typical Compound Example B-500>

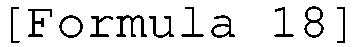

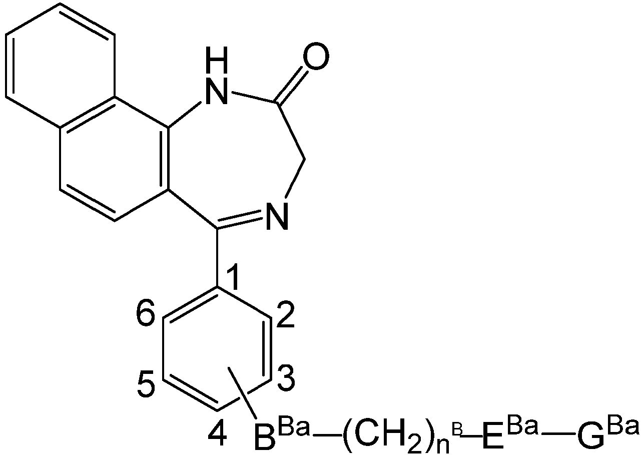

(B^{Ba} (substitution position), n^{B}, E^{Ba}, and G^{Ba} in the formula are indicated in Table 22)

**[Table 22]**

| B^{Ba} (substitution position) | n^{B} | E^{Ba} | G^{Ba} |
|---|---|---|---|
| NHCO(4) | 0 | Atomic bond | (2-Cl,3-OMe)Phenyl |
| NHCO(4) | 0 | Atomic bond | (2-Cl,3-OH)Phenyl |
| NHCO(4) | 0 | Atomic bond | (2-tBu)Phenyl |
| NHCO(4) | 0 | Atomic bond | (2-Cl,6-OMe)Phenyl |
| NHCO(4) | 0 | Atomic bond | (2-Cl,6-OH)Phenyl |
| NHSO2 (3) | 0 | Atomic bond | Phenyl |
| NHSO2 (4) | 0 | Atomic bond | (2-Cl)Phenyl |

### <Typical Compound Example B-600>

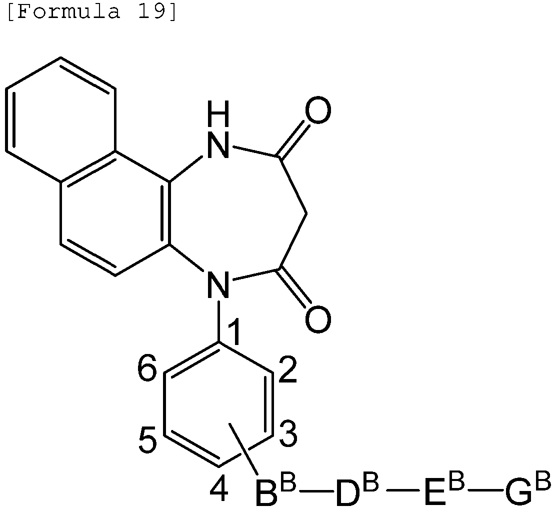

(B^{B} (substitution position), D^{B}, E^{B}, and G^{B} in the formula are indicated in Table 23)

**[Table 23]**

| B^{B}(substitution position) | D^{B} | E^{B} | G^{B} |
|---|---|---|---|
| NHCO(4) | C(Me)H | Atomic bond | Phenyl |
| NHCO(4) | C(Me)₂ | Atomic bond | Phenyl |
| NHCO(4) | CH=CH | Atomic bond | Phenyl |
| NHCO(4) | C(Me)H | O | Phenyl |
| NHCO(4) | C(Me)₂ | O | Phenyl |
| NHCO(4) | CH=CH | Atomic bond | (2-Me)Phenyl |
| NHCO(4) | CH=CH | Atomic bond | (2-Cl)Phenyl |

### <Typical Compound Example B-700>

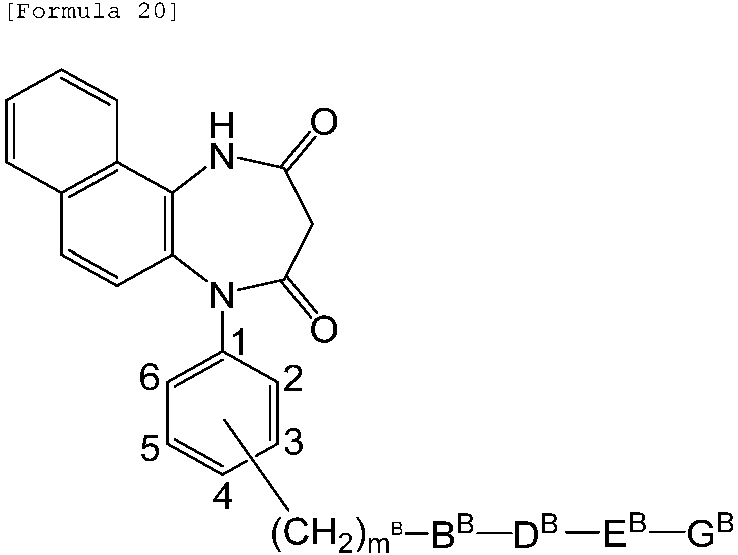

(m^{B} (substitution position), B^{B}, D^{B}, E^{B}, and G^{B} in the formula are indicated in Table 24)

**[Table 24]**

| m^{B} (substitution position) | B^{B} | D^{B} | E^{B} | G^{B} |
|---|---|---|---|---|
| 1 (4) | NHCO | Atomic bond | Atomic bond | Phenyl |
| 1 (4) | NHCO | Atomic bond | Atomic bond | (2-Cl) Phenyl |
| 1(4) | NHSO₂ | CH₂ | Atomic bond | (2-Cl) Phenyl |

### <Typical Compound Example B-800>

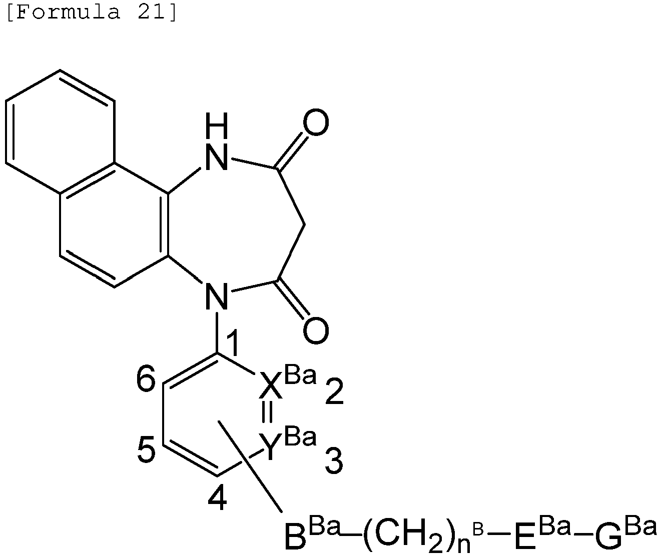

(X^{Ba}, Y^{Ba}, B^{Ba} (substitution position), n^{B}, E^{Ba}, and G^{Ba} in the formula are indicated in Table 25)

**[Table 25]**

| X^{Ba} | Y^{Ba} | B^{Ba} (substitution position) | n^{B} | E^{Ba} | G^{Ba} |
|---|---|---|---|---|---|
| CH | C-F | NHCO(4) | 0 | Atomic bond | (2,3-Me)Phenyl |
| CH | C-OH | NHCO(4) | 0 | Atomic bond | (2,3-Me)Phenyl |
| CH | C-F | NHCO(4) | 0 | Atomic bond | (2-I)Phenyl |
| CH | N | NHCO(4) | 0 | Atomic bond | (2-I)Phenyl |
| CH | N | NHCO(4) | 0 | Atomic bond | Phenyl |
| N | CH | NHCO(4) | 0 | Atomic bond | (2-I)Phenyl |
| CH | N | NHCO(4) | 0 | Atomic bond | (2-Cl)Phenyl |
| CH | N | NHCO(4) | 0 | Atomic bond | (2-OH)Phenyl |
| CH | N | NHC(=O)NH(4) | 0 | Atomic bond | (2-OH)Phenyl |
| CH | N | NHCO(4) | 0 | Atomic bond | (2-OH, 6-Me)Phenyl |
| CH | N | NHCO(4) | 0 | Atomic bond | (2-OH, 6-Cl) Phenyl |
| CH | N | NHCO (3) | 0 | Atomic bond | (2-OH, 6-Cl) Phenyl |
| CH | N | NHCO(4) | 0 | Atomic bond | (2-Cl)pyridin 2-yl |
| CH | N | NHCO(4) | 1 | Atomic bond | (2-Cl)pyridin 2-yl |
| CH | N | NHCO(4) | 0 | Atomic bond | (2-Me)pyridin 2-yl |
| CH | C-OMe | NHSO₂(4) | 1 | Atomic bond | (2-Cl)Phenyl |
| CH | C-OH | NHSO₂(4) | 1 | Atomic bond | (2-Cl)Phenyl |

### <Typical Compound Example B-900>

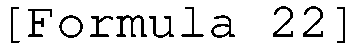

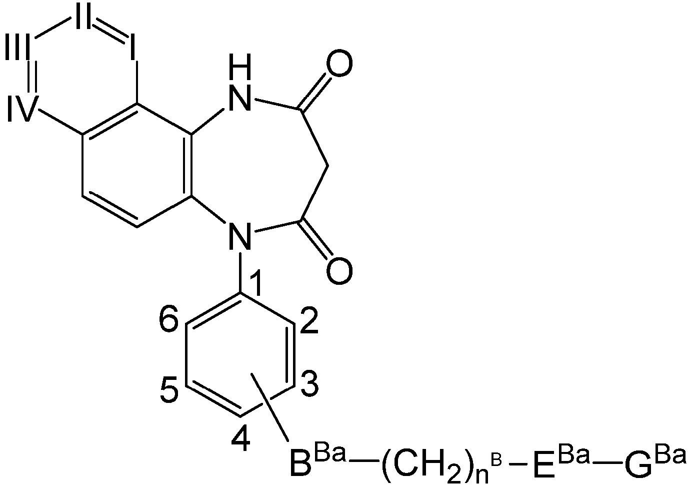

(I=II-III=IV, B^{Ba} (substitution position), n^{B}, E^{Ba}, and G^{Ba} in the formula are indicated in Table 26)

**[Table 26]**

| I=II-III=IV | B^{Ba} (substitution position) | n^{B} | E^{Ba} | G^{Ba} |
|---|---|---|---|---|
| N=CH-CH=CH | NHCO (4) | 0 | Atomic bond | (2-I) Phenyl |
| CH=N-CH=CH | NHCO (4) | 0 | Atomic bond | (2-I) Phenyl |
| CH=CH-N=CH | NHCO (4) | 0 | Atomic bond | (2-I) Phenyl |
| CH=CH-CH=N | NHCO (4) | 0 | Atomic bond | (2-I) Phenyl |
| N=CH-CH=CH | NHCO (4) | 1 | O | Phenyl |
| N=CH-CH=CH | NHCO (3) | 0 | Atomic bond | (2-I) Phenyl |
| N=CH-CH=CH | NHCO(4) | 0 | Atomic bond | (2-Cl) Phenyl |
| N=CH-CH=CH | NHCO (4) | 0 | Atomic bond | (2-OH) Phenyl |
| N=CH-CH=CH | NHC (=O)NH (4) | 0 | Atomic bond | (2-OH)Phenyl |
| N=CH-CH=CH | NHCO (4) | 1 | O | (2-OH, 6-Me)Phenyl |
| N=CH-CH=CH | NHCO (4) | 0 | Atomic bond | (2-OH, 6-Cl) Phenyl |
| N=CH-CH=CH | NHCO (3) | 0 | Atomic bond | (2-OH, 6-Cl) Phenyl |
| N=CH-CH=CH | NHCO (4) | 0 | Atomic bond | (2-Cl)pyridin 2-yl |
| N=CH-CH=CH | NHCO (4) | 1 | Atomic bond | (2-Cl)pyridin 2-yl |
| N=CH-CH=CH | NHCO (4) | 0 | Atomic bond | (2-Me)pyridin 2-yl |
| CH=CH-N=CH | NHCO (4) | 0 | Atomic bond | (2-Cl)pyridin 3-yl |

### <Typical Compound Example B-1000>

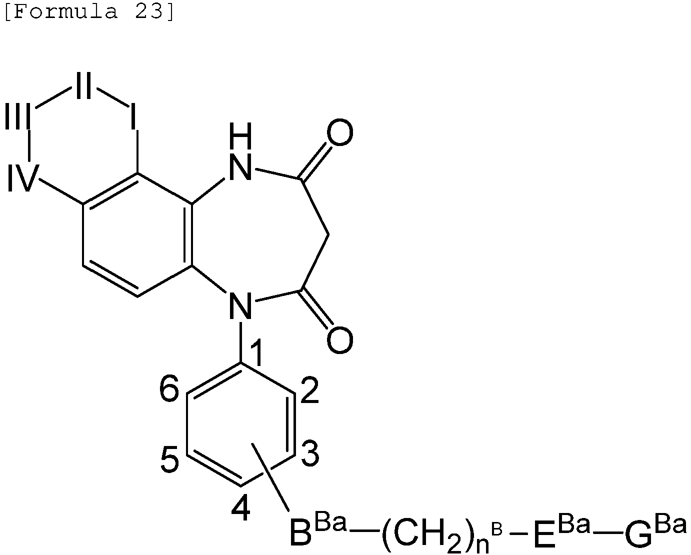

(I-II-III-IV, B^{Ba} (substitution position), n^{B}, E^{Ba}, and G^{Ba} in the formula are indicated in Table 27)

**[Table 27]**

| I-II-III-IV | B^{Ba} (substitution position) | n^{B} | E^{Ba} | G^{Ba} |
|---|---|---|---|---|
| NH-CH₂-CH₂-CH₂ | NHCO (4) | 0 | Atomic bond | (2-I) Phenyl |
| CH₂-NH-CH₂-CH₂ | NHCO (4) | 0 | Atomic bond | (2-I) Phenyl |
| CH₂-CH₂-NH-CH₂ | NHCO (4) | 0 | Atomic bond | (2-I) Phenyl |
| CH₂-CH₂-CH₂-NH | NHCO (4) | 0 | Atomic bond | (2-I) Phenyl |
| CH₂-CH₂-NH-CH₂ | NHCO (4) | 1 | O | Phenyl |
| CH₂-CH₂-NH-CH₂ | NHCO (3) | 0 | Atomic bond | (2-I) Phenyl |
| CH₂-CH₂-NH-CH₂ | NHCO (4) | 0 | Atomic bond | (2-Cl) Phenyl |
| CH₂-CH₂-NH-CH₂ | NHCO (4) | 0 | Atomic bond | (2-Cl) pyridin 3-yl |
| CH₂-CH₂-NH-CH₂ | NHCO (4) | 0 | Atomic bond | (2-OH) Phenyl |
| CH₂-CH₂-NH-CH₂ | NHC (=O) NH (4) | 0 | Atomic bond | (2-OH) Phenyl |
| CH₂-CH₂-NH-CH₂ | NHCO (4) | 1 | O | (2-OH, 6-Me) Phenyl |
| CH₂-CH₂-NH-CH₂ | NHCO (4) | 0 | Atomic bond | (2-OH, 6-Cl) Phenyl |
| CH₂-CH₂-NH-CH₂ | NHCO (3) | 0 | Atomic bond | (2-OH, 6-Cl) Phenyl |
| CH₂-CH₂-NH-CH₂ | NHCO (4) | 0 | Atomic bond | (2-Cl) pyridin 2-yl |
| CH₂-CH₂-NH-CH₂ | NHCO (4) | 1 | Atomic bond | (2-Cl) pyridin 2-yl |
| CH₂-CH₂-NH-CH₂ | NHCO (4) | 0 | Atomic bond | (2-Me) pyridin 2-yl |
| CH₂-CH₂-NH-CH₂ | NHCO (4) | 0 | Atomic bond | (2-Cl) pyridin 3-yl |

### <Typical Compound Example B-1100>

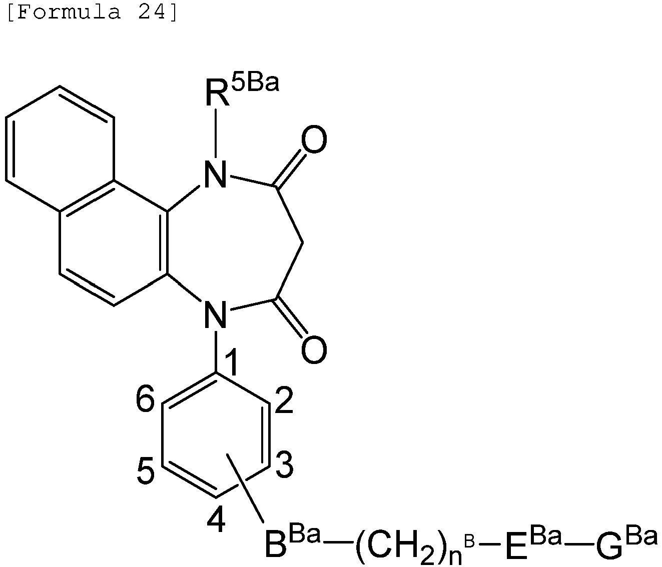

(R^{5Ba}, B^{Ba} (substitution position), n^{B}, E^{Ba}, and G^{Ba} in the formula are indicated in Table 28)

**[Table 28]**

| R^{5Ba} | B^{Ba} (substitution position) | n^{B} | E^{Ba} | G^{Ba} |
|---|---|---|---|---|
| Bn | NBnSO₂ (4) | 0 | Atomic bond | (2-NO₂) Phenyl |
| Me | NBnSO₂ (4) | 0 | Atomic bond | (2-NO₂) Phenyl |
| Et | NBnSO₂ (4) | 0 | Atomic bond | (2-NO₂) Phenyl |

Since the compounds represented by the general formula (A) are disclosed in WO 2010/093061 A, all of the compounds can be easily obtained by referring to this international publication.

Since the compounds represented by the general formulas (BI) and (BII) are disclosed in WO 2013/105608 A, all of the compounds can be easily obtained by referring to this international publication.

Furthermore, WO 2010/093061 A and WO 2013/105608 A disclose that the compounds represented by the general formulas (A) to (BII) have a P2X4 receptor antagonistic action.

Specific examples of a preferable compound comprised in the compounds represented by the general formulas (A) to (BII) or a pharmaceutically acceptable salt thereof are shown below, however, the compound or a pharmaceutically acceptable salt thereof that can be used as the active ingredient of the medicament of the present invention is
(A16) 5-[3-(5-thioxo-4H-[1,2,4]oxadiazol-3-yl)phenyl]-1H-naphtho[1,2-b][1,4]diazepine-2,4(3H,5H)-dione;
(A17) a 5-[3-(5-thioxo-4H-[1,2,4]oxadiazol-3-yl)phenyl]-1H-naphtho[1,2-b][1,4]diazepine-2,4(3H,5H)-dione sodium salt; and
(B48) 5-[4-(2-iodobenzoylamino)phenyl]-1H-naphtho[1,2-b] [1,4]diazepine-2,4(3H,5H)-dione.

The other compounds are for reference only.
(Compound A1) 5-[3-(1H-tetrazol-5-yl)phenyl]-1H-naphtho[1,2-b][1,4]diazepine-2,4(3H,5H)-dione;
(Compound A2) 5-[3-(1H-tetrazol-5-yl)phenyl]-1H-naphtho[1,2-b][1,4]diazepine-2,4(3H,5H)-dione sodium salt;
(Compound A3) 5-[3-(1H-tetrazol-5-yl)phenyl]-1H-naphtho[1,2-b][1,4]diazepine-2,4(3H,5H)-dione potassium salt;
(Compound A4) 5-[4-(1H-tetrazol-5-yl)phenyl]-1H-naphtho[1,2-b][1,4]diazepine-2,4(3H,5H)-dione;
(Compound A5) 5-[4-(1H-tetrazol-5-yl)phenyl]-1H-naphtho[1,2-b][1,4]diazepine-2,4(3H,5H)-dione sodium salt;
(Compound A6) 1-methyl-5-[3-(1H-tetrazol-5-yl)phenyl]-1H-naphtho[1,2-b][1,4]diazepine-2,4(3H,5H)-dione;
(Compound A7) 1,3-dimethyl-5-[3-(1H-tetrazol-5-yl)phenyl]-1H-naphtho[1,2-b][1,4]diazepine-2,4(3H,5H)-dione;
(Compound A8) 5-[2-chloro-5-(1H-tetrazol-5-yl)phenyl]-1H-naphtho[1,2-b][1,4]diazepine-2,4(3H,5H)-dione;
(Compound A9) 5-[2-chloro-5-(1H-tetrazol-5-yl)phenyl]-1H-naphtho[1,2-b][1,4]diazepine-2,4(3H,5H)-dione sodium salt;
(Compound A10) 5-[2-methyl-5-(1H-tetrazol-5-yl)phenyl]-1H-naphtho[1,2-b][1,4]diazepine-2,4(3H,5H)-dione;
(Compound A11) 5-[2-methyl-5-(1H-tetrazol-5-yl)phenyl]-1H-naphtho[1,2-b][1,4]diazepine-2,4(3H,5H)-dione sodium salt;
(Compound A12) 5-[2-bromo-5-(1H-tetrazol-5-yl)phenyl]-1H-naphtho[1,2-b][1,4]diazepine-2,4(3H,5H)-dione;
(Compound A13) 5-[3-(2-methyl-2H-tetrazol-5-yl)phenyl]-1H-naphtho[1,2-b][1,4]diazepine-2,4(3H,5H)-dione;
(Compound A14) 5-[3-(1-methyl-1H-tetrazol-5-yl)phenyl]-1H-naphtho[1,2-b][1,4]diazepine-2,4(3H,5H)-dione;
(Compound A15) 5-[3-(5-oxo-4H-[1,2,4]oxadiazol-3-yl)phenyl]-1H-naphtho[1,2-b][1,4]diazepine-2,4(3H,5H)-dione;
(Compound A16) 5-[3-(5-thioxo-4H-[1,2,4]oxadiazol-3-yl)phenyl]-1H-naphtho[1,2-b][1,4]diazepine-2,4(3H,5H)-dione;
(Compound A17) 5-[3-(5-thioxo-4H-[1,2,4]oxadiazol-3-yl)phenyl]-1H-naphtho[1,2-b][1,4]diazepine-2,4(3H,5H)-dione sodium salt;
(Compound A18) 5-[3-(oxazol-2-yl)phenyl]-1H-naphtho[1,2-b][1,4]diazepine-2,4(3H,5H)-dione;
(Compound A19) 5-[3-(1H-pyrazol-4-yl)phenyl]-1H-naphtho[1,2-b] [1,4]diazepine-2,4(3H,5H)-dione;
(Compound A20) 5-[4-fluoro-3-(1H-tetrazol-5-yl)phenyl]-1H-naphtho[1,2-b][1,4]diazepine-2,4(3H,5H)-dione;
(Compound A21) 5-[4-fluoro-3-(1H-tetrazol-5-yl)phenyl]-1H-naphtho[1,2-b][1,4]diazepine-2,4(3H,5H)-dione sodium salt;
(Compound B1) 5-(4-benzoylaminophenyl)-1H-naphtho[1,2-b] [1,4]diazepine-2,4(3H,5H)-dione;
(Compound B2) 5-[4-[2-(trifluoromethyl)benzoyl]aminophenyl]-1H-naphtho[1,2-b][1,4]diazepine-2,4(3H,5H)-dione;
(Compound B3) 5-[4-(3-bromobenzoyl)aminophenyl]-1H-naphtho[1,2-b] [1,4]diazepine-2,4(3H,5H)-dione;
(Compound B4) 5-[4-[4-(trifluoromethyl)benzoyl]aminophenyl]-1H-naphtho[1,2-b][1,4]diazepine-2,4(3H,5H)-dione;
(Compound B5) 5-[4-(2-methylbenzoyl)aminophenyl]-1H-naphtho[1,2-b] [1,4]diazepine-2,4(3H,5H)-dione;
(Compound B6) 5-[4-(2,6-dimethylbenzoylamino)phenyl]-1H-naphtho[1,2-b][1,4]diazepine-2,4(3H,5H)-dione;
(Compound B7) 5-[4-(2,6-dichlorobenzoyl)aminophenyl]-1H-naphtho[1,2-b][1,4]diazepine-2,4(3H,5H)-dione;
(Compound B8) 5-[4-(3-chlorobenzoyl)aminophenyl]-1H-naphtho[1,2-b] [1,4]diazepine-2,4(3H,5H)-dione;
(Compound B9) 5[4-(2-phenylacetylamino)phenyl]-1H-naphtho[1,2-b] [1,4]diazepine-2,4(3H,5H)-dione;
(Compound B10) 1-[4-(2,4-dioxo-1,2,3,4-tetrahydronaphtho[1,2-b] [1,4]diazepin-5-yl)phenyl]-3-phenylthiourea;
(Compound B11) 5-[4-(2,3-dimethoxybenzoylamino)phenyl]-1H-naphtho[1,2-b][1,4]diazepine-2,4(3H,5H)-dione;
(Compound B12) 5-[4-(2-methoxybenzoylamino)phenyl]-1H-naphtho[1,2-b][1,4]diazepine-2,4(3H,5H)-dione;
(Compound B13) 5-[4-[(2-chlorophenylacetyl)amino]phenyl]-1H-naphtho[1,2-b][1,4]diazepine-2,4(3H,5H)-dione;
(Compound B14) 5-[4-(2,3-dimethylbenzoylamino)phenyl]-1H-naphtho[1,2-b][1,4]diazepine-2,4(3H,5H)-dione;
(Compound B15) 5-[4-(2,5-dimethylbenzoylamino)phenyl]-1H-naphtho[1,2-b][1,4]diazepine-2,4(3H,5H)-dione;
(Compound B16) 5-[4-(5-bromo-2-chlorobenzoylamino)phenyl]-1H-naphtho[1,2-b] [1,4]diazepine-2,4(3H,5H)-dione;
(Compound B17) 5-[4-(2,4-dichlorobenzoylamino)phenyl]-1H-naphtho[1,2-b][1,4]diazepine-2,4(3H,5H)-dione;
(Compound B18) 5-[4-(2-hydroxybenzoylamino)phenyl]-1H-naphtho[1,2-b][1,4]diazepine-2,4(3H,5H)-dione;
(Compound B19) 5-[4-(2,3-dihydroxybenzoylamino)phenyl]-1H-naphtho[1,2-b][1,4]diazepine-2,4(3H,5H)-\dione;
(Compound B20) 1-[4-(2,4-dioxo-1,2,3,4-tetrahydronaphtho[1,2-b] [1,4]diazepin-5-yl)phenyl]-3-phenylurea;
(Compound B21) 5-[4-[(2,6-dichlorophenylacetyl)amino]phenyl]-1H-naphtho[1,2-b][1,4]diazepine-2,4(3H,5H)-dione;
(Compound B22) 5-[4-[(2-methoxyphenylacetyl)amino]phenyl]-1H-naphtho[1,2-b][1,4]diazepine-2,4(3H,5H)-dione;
(Compound B23) 5-[4-[(2-hydroxyphenylacetyl)amino]phenyl]-1H-naphtho[1,2-b][1,4]diazepine-2,4(3H,5H)-dione;
(Compound B24) 1-(2-chlorophenyl)-3-[4-(2,4-dioxo-1,2,3,4-tetrahydronaphtho[1,2-b][1,4]diazepin-5-yl)phenyl]thiourea;
(Compound B25) 5-[4-[3-(trifluoromethyl)benzoylamino]phenyl]-1H-naphtho[1,2-b][1,4]diazepine-2,4(3H,5H)-dione;
(Compound B26) 5-[4-[2-[2-(trifluoromethyl)phenyl]acetylamino]phenyl]-1H-naphtho[1,2-b][1,4]diazepine-2,4(3H,5H)-dione;
(Compound B27) 1-(2-chlorophenyl)-3-[4-(2,4-dioxo-1,2,3,4-tetrahydronaphtho[1,2-b][1,4]diazepin-5-yl)phenyl]urea;
(Compound B28) 5-[4-[(2-phenylpropionyl)amino]phenyl]-1H-naphtho[1,2-b][1,4]diazepine-2,4(3H,5H)-dione;
(Compound B29) 5-[4-(2-chloro-3-methoxybenzoylamino)phenyl]-1H-naphtho[1,2-b][1,4]diazepine-2,4(3H,5H)-dione;
(Compound B30) 5-[4-(3-phenylpropionylamino)phenyl]-1H-naphtho[1,2-b][1,4]diazepine-2,4(3H,5H)-dione;
(Compound B31) 5-[4-[(1H-indole-3-carbonyl)amino]phenul]-1H-naphtho[1,2-b][1,4]diazepine-2,4 (3H, 5H) -dione;
(Compound B32) 5-[4-(2-chloro-3-hydroxybenzoylamino)phenyl]-1H-naphtho[1,2-b][1,4]diazepine-2,4(3H,5H)-dione;
(Compound B33) 5-[4-[(2-methyl-2-phenylpropionyl)amino]phenyl]-1H-naphtho[1,2-b][1,4]diazepine-2,4(3H,5H)-dione;
(Compound B34) 5-[4-(2-phenoxyacetylamino)phenyl]-1H-naphtho[1,2-b][1,4]diazepine-2,4(3H,5H)-dione;
(Compound B35) 5-[4-[2-(2-chloro-4-methoxyphenyl)acetylamino]phenyl]-1H-naphtho[1,2-b][1,4]diazepine-2,4(3H,5H)-dione;
(Compound B36) 5-[4-[(1-methyl-1H-imidazole-2-carbonyl)amino]phenyl]-1H-naphtho[1,2-b][1,4]diazepine-2,4(3H,5H)-dione;
(Compound B37) 5-[4-[2-(2,4-dichlorophenyl)acetylamino]phenyl]-1H-naphtho[1,2-b][1,4]diazepine-2,4(3H,5H)-dione;
(Compound B38) 5-[4-[2-(2-chloro-4-hydroxyphenyl)acetylamino]phenyl]-1H-naphtho[1,2-b][1,4]diazepine-2,4(3H,5H)-dione;
(Compound B39) 5-[4-(3-phenylpropenylamino)phenyl]-1H-naphtho[1,2-b][1,4]diazepine-2,4(3H,5H)-dione;
(Compound B40) 5-[4-[(3-pyridylacetyl)amino]phenyl]-1H-naphtho[1,2-b][1,4]diazepine-2,4(3H,5H)-dione hydrochloride;
(Compound B41) 5-[4-(1H-benzimidazole-2-carbonylamino)phenyl]-1H-naphtho[1,2-b][1,4]diazepine-2,4 (3H, 5H) -dione;
(Compound B42) 1-[4-(2,3-dimethylbenzoylamino)phenyl]-7-methoxy-1H-1,5-benzodiazepine-2,4(3H,5H)-dione;
(Compound B43) 5-[4-[(benzoylamino)methyl]phenyl]-1H-naphtho[1,2-b][1,4]diazepine-2,4(3H,5H)-dione;
(Compound B44) 5-[4-[(2-chlorobenzoylamino)methyl]phenyl]-1H-naphtho[1,2-b][1,4]diazepine-2,4(3H,5H)-dione;
(Compound B45) 1-[4-(2,3-dimethylbenzoylamino)phenyl]-7-hydroxy-1H-1,5-benzodiazepine-2,4(3H,5H)-dione;
(Compound B46) 5-[4-(2-chlorobenzoylamino)phenyl]-1H-naphtho[1,2-b][1,4]diazepine-2,4(3H,5H)-dione;
(Compound B47) 5-[4-(2-bromobenzoylamino)phenyl]-1H-naphtho[1,2-b][1,4]diazepine-2,4(3H,5H)-dione;
(Compound B48) 5-[4-(2-iodobenzoylamino)phenyl]-1H-naphtho[1,2-b] [1,4]diazepine-2,4(3H,5H)-dione;
(Compound B49) 5-[4-(2,3-dimethylbenzoylamino)-3-fluorophenyl]-1H-naphtho[1,2-b][1,4]diazepine-2,4(3H,5H)-dione;
(Compound B50) 5-[4-[2-(2-methylphenyl)acetylamino]phenyl]-1H-naphtho[1,2-b][1,4]diazepine-2,4(3H,5H)-dione;
(Compound B51) 5-[4-[(quinoxalin-2-yl)carbonylamino]phenyl]-1H-naphtho[1,2-b][1,4]diazepine-2,4(3H,5H)-dione;
(Compound B52) 5-[4-[(5-methylthiophen-2-yl)carbonylamino]phenyl]-1H-naphtho[1,2-b][1,4]diazepine-2,4(3H,5H)-dione;
(Compound B53) 5-[3-[(2-chlorophenylacetyl)amino]phenyl]-1H-naphtho[1,2-b][1,4]diazepine-2,4(3H,5H)-dione;
(Compound B54) 5-[4-[(2,4,6-trimethylbenzoyl)amino]phenyl]-1H-naphtho[1,2-b][1,4]diazepine-2,4(3H,5H)-dione;
(Compound B55) 5-[4-(cyclohexylcarbonylamino)phenyl]-1,2,3,4,8,9,10,11-octahydronaphtho[1,2-b][1,4]diazepine-2,4(3H,5H)-dione;
(Compound B56) 1-[4-(2,3-dimethylbenzoyl)aminophenyl]-6-methyl-1H-1,5-benzodiazepine-2,4(3H,5H)-dione;
(Compound B57) 5-[4-[(2-ethylbenzoyl)amino]phenyl]-1H-naphtho[1,2-b][1,4]diazepine-2,4(3H,5H)-dione;
(Compound B58) 5-[4-[(6-methylpyridin-2-yl)carbonylamino]phenyl]-1,2,3,4,8,9,10,11-octahydronaphtho[1,2-b] [1,4]diazepine-2,4(3H,5H)-dione;
(Compound B59) 5-[4-[(2-methylpyridin-3-yl)carbonylamino]phenyl]-1,2,3,4,8,9,10,11-octahydronaphtho[1,2-b][1,4]diazepine-2,4(3H,5H)-dione;
(Compound B60) 1-[4-(2,4-dioxo-1,2,3,4-tetrahydronaphtho[1,2-b][1,4]diazepin-5-yl)phenyl]-3-(2-methylphenyl)thiourea;
(Compound B61) 5-[4-(2-methoxy-3-methylbenzoyl)aminophenyl]-1,2,3,4,8,9,10,11-octahydronaphtho[1,2-b] [1,4]diazepine-2,4(3H,5H)-dione;
(Compound B62) 5-[4-(2,3-dichlorobenzoyl)aminophenyl]-1,2,3,4,8,9,10,11-octahydronaphtho[1,2-b] [1,4]diazepine-2,4(3H,5H)-dione;
(Compound B63) 5-[4-(2,3-dimethylbenzoylamino)-3-hydroxyphenylJ-1H-naphtho[1,2-b][1,4]diazepine-2,4(3H,5H)-dione;
(Compound B64) 5-[4-(2-chloro-3-methoxybenzoylamino)phenyl]-1,3-dihydronaphtho[1,2-e]-1,4-diazepin-2-one;
(Compound B65) 5-[4-[(4-dimethylaminobenzoyl)amino]phenyl]-1H-naphtho[1,2-b][1,4]diazepine-2,4(3H,5H)-dione;
(Compound B66) 5-[4-[2-(2,4-dichlorophenoxy)acetylamino]phenyl]-1H-naphtho[1,2-b][1,4]diazepine-2,4(3H,5H)-dione;
(Compound B67) 5-[4-[2-(2-methylphenoxy)acetylamino]phenyl]-1H-naphtho[1,2-b][1,4]diazepine-2,4(3H,5H)-dione;
(Compound B68) N-[4-(2,4-dioxo-1,2,3,4-tetrahydronaphtho[1,2-b][1,4]diazepin-5-yl)butyl]-2-chloro-3-methoxybenzamide;
(Compound B69) 5-[4-(2-chloro-3-hydroxybenzoylamino)phenyl]-1,3-dihydronaphtho[1,2-e]-1,4-diazepin-2-one;
(Compound B70) 5-[4-(2-acetylbenzoylamino)phenyl]-1H-naphtho[1,2-b][1,4]diazepine-2,4(3H,5H)-dione;
(Compound B71) 5-[4-(2-tert-butylbenzoylamino)phenyl]-1H-naphtho[1,2-b][1,4]diazepine-2,4 (3H, 5H) -dione;
(Compound B72) 5-[2-(2-iodobenzoyl)aminoethyl]-1H-naphtho[1,2-b][1,4]diazepine-2,4(3H,5H)-dione;
(Compound B73) 5-[3-[(2-iodobenzoyl)amino]phenyl]-1H-naphtho[1,2-b][1,4]diazepine-2,4(3H,5H)-dione;
(Compound B74) 6,7-dimethyl-1-[4-(2-iodobenzoyl)aminophenyl]-1H-1,5-benzodiazepine-2,4(3H,5H)-dione;
(Compound B75) 5-[4-[(1-methylpiperidin-4-yl)carbonylamino]phenyl]-1H-naphtho[1,2-b][1,4]diazepine-2,4(3H,5H)-dione hydrochloride;
(Compound B76) 5[4-[(benzofuran-2-yl)carbonylamino]phenyl]-1H-naphtho[1,2-b][1,4]diazepine-2,4(3H,5H)-dione;
(Compound B77) 5-[4-[(1-methyl-1H-indol-3-yl)carbonylamino]phenyl]-1H-naphtho[1,2-b][1,4]diazepine-2,4(3H,5H)-dione;
(Compound B78) 5-[4-(2-propenylbenzoylamino)phenyl]-1H-naphtho[1,2-b][1,4]diazepine-2,4(3H,5H)-dione;
(Compound B79) 5-[4-(2-propylbenzoylamino)phenyl]-1H-naphtho[1,2-b][1,4]diazepine-2,4(3H,5H)-dione;
(Compound B80) 5-[3-fluoro-4-(2-iodobenzoylamino)phenyl]-1H-naphtho[1,2-b][1,4]diazepine-2,4 (3H, 5H) -dione;
(Compound B81) 5-[4-(2-hydroxy-3-methylbenzoyl)aminophenyl]-1,2,3,4,8,9,10,11-octahydronaphtho[1,2-b][1,4]diazepine-2,4(3H,5H)-dione;
(Compound B82) 5-[4-[(2-isopropoxybenzoyl)amino]phenyl]-1H-naphtho[1,2-b][1,4]diazepine-2,4(3H,5H)-dione;
(Compound B83) 5-[4-[(3-methylthiophen-2-yl)carbonylamino]phenyl]-1H-naphtho[1,2-b] [1,4]diazepine-2,4(3H,5H)-dione;
(Compound B84) 5-[4-(2-phenoxypropionylamino)phenyl]-1H-naphtho[1,2-b][1,4]diazepine-2,4(3H,5H)-dione;
(Compound B85) 5-[4-[2-(4-chloro-2-methylphenoxy)acetylamino]phenyl]-1H-naphtho[1,2-b][1,4]diazepine-2,4(3H,5H)-dione;
(Compound B86) 5-[4-[(4-fluoro-2-trifluoromethyl)benzoyl]aminophenyl]-1H-naphtho[1,2-b][1,4]diazepine-2,4(3H,5H)-dione;
(Compound B87) 5-[4-(4-fluoro-2-methoxybenzoyl)aminophenyl]-1H-naphtho[1,2-b][1,4]diazepine-2,4(3H,5H)-dione;
(Compound B88) 5-[4-(4-fluoro-2-hydroxybenzoyl)aminophenyl]-1H-naphtho[1,2-b][1,4]diazepine-2,4(3H,5H)-dione;
(Compound B89) 5-[3-[(2-iodophenylacetyl)amino]phenyl]-1H-naphtho[1,2-b][1,4]diazepine-2,4(3H,5H)-dione;
(Compound B90) 5-[4-(2-methyl-2-phenoxypropionylamino)phenyl]-1H-naphtho[1,2-b][1,4]diazepine-2,4(3H,5H)-dione;
(Compound B91) 5-[4-(2-tert-butylbenzoylamino)phenyl]-1,3-dihydronaphtho[1,2-e]-1,4-diazepin-2-one;
(Compound B92) 5-[4-[(3-dimethylaminobenzoyl)amino]phenyl]-1H-naphtho[1,2-b][1,4]diazepine-2,4(3H,5H)-dione;
(Compound B93) 5-[4-(4-iodo-2-methoxybenzoylamino)phenyl]-1H-naphtho[1,2-b][1,4]diazepine-2,4(3H,5H)-dione;
(Compound B94) 5-[4-(6-fluoro-2-methoxybenzoylamino)phenyl]-1H-naphtho[1,2-b][1,4]diazepine-2,4(3H,5H)-dione;
(Compound B95) 5-[4-(2-hydroxy-4-iodobenzoylamino)phenyl]-1H-naphtho[1,2-b][1,4]diazepine-2,4(3H,5H)-dione;
(Compound B96) 5-[4-(6-fluoro-2-hydroxybenzoylamino)phenyl]-1H-naphtho[1,2-b][1,4]diazepine-2,4(3H,5H)-dione;
(Compound B97) 5-[4-(2-fluorobenzoyl)aminophenyl]-1H-naphtho[1,2-b][1,4]diazepine-2,4(3H,5H)-dione;
(Compound B98) 5-[4-[(2-dimethylaminobenzoyl)amino]phenyl]-1H-naphtho[1,2-b][1,4]diazepine-2,4(3H,5H)-dione;
(Compound B99) 5-[4-(2-methoxy-6-methylbenzoylamino)phenyl]-1H-naphtho[1,2-b][1,4]diazepine-2,4 (3H, 5H) -dione;
(Compound B100) 5-[4-(2-hydroxy-6-methylbenzoylamino)phenyl]-1H-naphtho[1,2-b][1,4]diazepine-2,4(3H,5H)-dione;
(Compound B101) 5-[4-[3-(2-methylphenyl)propionylamino]phenyl]-1H-naphtho[1,2-b][1,4]diazepine-2,4(3H,5H)-dione;
(Compound B102) 5-(4-phenylcarbamoylphenyl)-1H-naphtho[1,2-b][1,4]diazepine-2,4(3H,5H)-dione;
(Compound B103) 5-(4-benzylcarbamoylphenyl)-1H-naphtho[1,2-b][1,4]diazepine-2,4(3H,5H)-dione;
(Compound B104) 5-[4-[3-(2-methylphenyl)propenoylamino]phenyl]-1H-naphtho[1,2-b][1,4]diazepine-2,4(3H,5H)-dione;
(Compound B105) 5-[4-[3-(2-chlorophenyl)propionylamino]phenyl]-1H-naphtho[1,2-b][1,4]diazepine-2,4(3H,5H)-dione;
(Compound B106) 5-[4-(2-iodobenzoyl)aminophenyl]-1,2,3,4,8,9,10,11-octahydronaphtho[1,2-b][1,4]diazepine-2,4 (3H, 5H) -dione;
(Compound B107) 5-[4-[(1-methyl-1H-pyrrol-2-ylacetyl)amino]phenyl]-1,2,3,4,8,9,10,11-octahydronaphtho[1,2-b] [1,4]diazepine-2,4(3H,5H)-dione;
(Compound B108) 5-[4-(2-chlorobenzyl)carbamoylphenyl]-1H-naphtho[1,2-b][1,4]diazepine-2,4(3H,5H)-dione;
(Compound B109) 5-[4-[3-(2-chlorophenyl)propenoylamino]phenyl]-1H-naphtho[1,2-b][1,4]diazepine-2,4(3H,5H)-dione;
(Compound B110) 5-[4-(2-chlorophenyl)carbamoylphenyl]-1H-naphtho[1,2-b][1,4]diazepine-2,4(3H,5H)-dione;
(Compound B111) 5-[4-(6-bromo-2,3-methylenedioxybenzoylamino)phenyl]-1H-naphtho[1,2-b][1,4]diazepine-2,4(3H,5H)-dione;
(Compound B112) 5-[4-(6-bromo-2-methoxybenzoylamino)phenyl]-1H-naphtho[1,2-b][1,4]diazepine-2,4(3H,5H)-dione;
(Compound B113) 5-[4-[(2-tert-butylbenzoyl)amino]phenyl]-1,2,3,4,8,9,10,11-octahydronaphtho[1,2-b] [1,4]diazepine-2,4(3H,5H)-dione;
(Compound B114) 5-[2-(2-iodobenzoyl)aminopyridin-5-yl]-1H-naphtho[1,2-b][1,4]diazepine-2,4(3H,5H)-dione;
(Compound B115) 5-[4-(6-bromo-2-hydroxybenzoylamino)phenyl]-1H-naphtho[1,2-b][1,4]diazepine-2,4(3H,5H)-dione;
(Compound B116) 5-[4-(6-chloro-2-methoxybenzoylamino)phenyl]-1H-naphtho[1,2-b][1,4]diazepine-2,4(3H,5H)-dione;
(Compound B117) 5-[4-(2-iodobenzoylamino)phenyl]-1H-[1,4]diazepino[2,3-h]quinoline-2,4(3H,5H)-dione;
(Compound B118) 5-[4-(6-chloro-2-hydroxybenzoylamino)phenyl]-1H-naphtho[1,2-b][1,4]diazepine-2,4(3H,5H)-dione;
(Compound B119) 5-[4-(2-hydroxy-6-methoxybenzoylamino)phenyl]-1H-naphtho[1,2-b][1,4]diazepine-2,4(3H,5H)-dione;
(Compound B120) 5-[4-[2-methoxy-6-(trifluoromethyl)benzoylamino]phenyl]-1H-naphtho[1,2-b][1,4]diazepine-2,4(3H,5H)-dione;
(Compound B121) 5-[4-[2-hydroxy-6-(trifluoromethyl)benzoylamino]phenyl]-1H-naphtho[1,2-b][1,4]diazepine-2,4(3H,5H)-dione;
(Compound B122) 5-[4-[(2-isopropenylbenzoyl)amino]phenyl]-1,2,3,4,8,9,10,11-octahydronaphtho[1,2-b] [1,4]diazepine-2,4(3H,5H)-dione;
(Compound B123) 5-[4-[(2-isopropylbenzoyl)amino]phenyl]-1,2,3,4,8,9,10,11-octahydronaphtho[1,2-b] [1,4]diazepine-2,4(3H,5H)-dione;
(Compound B124) 5-[4-[2-chloro-5-(methylthio)benzoylamino]phenyl]-1H-naphtho[1,2-b][1,4]diazepine-2,4(3H,5H)-dione;
(Compound B125) 5-[4-[2-(methylthio)benzoylamino]phenyl]-1H-naphtho[1,2-b][1,4]diazepine-2,4(3H,5H)-dione;
(Compound B126) 5-[4-[3-(methylthio)benzoylamino]phenyl]-1H-naphtho[1,2-b][1,4]diazepine-2,4(3H,5H)-dione;
(Compound B127) 5-[4-[2-ethyl-6-methoxybenzoylamino]phenyl]-1H-naphtho[1,2-b][1,4]diazepine-2,4(3H,5H)-dione;
(Compound B128) 5-[4-(3-methanesulfonylbenzoylamino)phenyl]-1H-naphtho[1,2-b][1,4]diazepine-2,4(3H,5H)-dione;
(Compound B129) 6-ethyl-1-[4-(2-iodobenzoyl)aminophenyl]-1H-1,5-benzodiazepine-2,4(3H,5H)-dione;
(Compound B130) 5-[4-[2-ethyl-6-hydroxybenzoylamino]phenyl]-1H-naphtho[1,2-b][1,4]diazepine-2,4(3H,5H)-dione;
(Compound B131) 5-[4-(3-methanesulfinylbenzoylamino)phenyl]-1H-naphtho[1,2-b][1,4]diazepine-2,4(3H,5H)-dione;
(Compound B132) 5-[4-(2-chloro-5-methanesulfinylbenzoylamino)phenyl]-1H-naphtho[1,2-b][1,4]diazepine-2,4(3H,5H)-dione;
(Compound B133) 5-[4-(2-methanesulfinylbenzoylamino)phenyl]-1H-naphtho[1,2-b][1,4]diazepine-2,4(3H,5H)-dione;
(Compound B134) 5-[4-[[2-(4-morpholinyl)acetyl]amino]phenyl]-1,2,3,4,8,9,10,11-octahydronaphtho[1,2-b] [1,4]diazepine-2,4(3H,5H)-dione hydrochloride;
(Compound B135) 5-[4-(2-chloro-6-methoxybenzoylamino)phenyl]-1,3-dihydronaphtho[1,2-e]-1,4-diazepin-2-one;
(Compound B136) 5-[4-[[(3-chloropyridin-2-yl)carbonyl]amino]phenyl]-1H-naphtho[1,2-b][1,4]diazepine-2,4(3H,5H)-dione;
(Compound B137) 5-[4-(2-chloro-6-hydroxybenzoylamino)phenyl]-1,3-dihydronaphtho[1,2-e]-1,4-diazepin-2-one;
(Compound B138) 5-[4-(3-chloro-2-methoxybenzoylamino)phenyl]-1H-naphtho[1,2-b][1,4]diazepine-2,4(3H,5H)-dione;
(Compound B139) 5-[4-[(3-methylpyridin-2-yl)carbonylamino]phenyl]-1H-naphtho[1,2-b][1,4]diazepine-2,4(3H,5H)-dione;
(Compound B140) 5-[4-[[(3-chloropyridin-2-yl)carbonyl]amino]phenyl]-1,2,3,4,8,9,10,11-octahydronaphtho[1,2-b] [1,4]diazepine-2,4(3H,5H)-dione;
(Compound B141) 5-[4-(3-chloro-2-hydroxybenzoylamino)phenyl]-1H-naphtho[1,2-b][1,4]diazepine-2,4(3H,5H)-dione;
(Compound B142) 5-[4-[[(3-hydroxypyridin-2-yl)carbonyl]amino]phenyl]-1H-naphtho[1,2-b][1,4]diazepine-2,4(3H,5H)-dione;
(Compound B143) 5-[4-[(3-vinylpyridin-2-yl)carbonylamino]phenyl]-1H-naphtho[1,2-b][1,4]diazepine-2,4(3H,5H)-dione;
(Compound B144) 5-[4-[(3-ethylpyridin-2-yl)carbonylamino]phenyl]-1H-naphtho[1,2-b][1,4]diazepine-2,4(3H,5H)-dione;
(Compound B145) N-[4-(2,4-dioxo-1,2,3,4-tetrahydronaphtho-[1,2-b][1,4]-diazepin-5-yl)phenyl]-2-nitrobenzenesulfonamide;
(Compound B146) N-[4-(2,4-dioxo-1,2,3,4-tetrahydronaphtho[1,2-b][1,4]diazepin-5-yl)phenyl]benzenesulfonamide;
(Compound B147) 3-bromo-N-[4-(2,4-dioxo-1,2,3,4-tetrahydronaphtho[1,2-b][1,4]diazepin-5-yl)phenyl]benzenesulfonamide;
(Compound B148) N-[4-(2,4-dioxo-1,2,3,4-tetrahydronaphtho[1,2-b] [1,4]diazepin-5-yl)phenyl]-3-methoxybenzenesulfonamide;
(Compound B149) N-[3-(2-oxo-2,3-dihydro-1H-naphtho[1,2-e] [1,4]diazepin-5-yl)phenyl]benzenesulfonamide;
(Compound B150) N-[3-(2,4-dioxo-1,2,3,4-tetrahydronaphtho-[1,2-b][1,4]-diazepin-5-yl)phenyl]-2-nitrobenzenesulfonamide;
(Compound B151) N-[4-(2,4-dioxo-1,2,3,4,8,9,10,11-octahydro-naphtho[1,2-b][1,4]-diazepin-5-yl)phenyl]-2-nitro-benzenesulfonamide;
(Compound B152) N-[4-(2,4-dioxo-1,2,3,4,8,9,10,11-octahydro-naphtho[1,2-b][1,4]-diazepin-5-yl)phenyl]-N-methyl-2-nitrobenzenesulfonamide;
(Compound B153) N-[3-(2,4-dioxo-1,2,3,4-tetrahydronaphtho-[1,2-b][1,4]-diazepin-5-yl)phenyl]-N-methyl-2-nitrobenzenesulfonamide;
(Compound B154) 4-(2,4-dioxo-1,2,3,4,8,9,10,11-octahydronaphtho[1,2-b][1,4]diazepin-5-yl)-N-phenylbenzenesulfonamide;
(Compound B155) N-[3-(2,4-dioxo-1,2,3,4-tetrahydronaphtho[1,2-b]-[1,4]diazepin-5-yl)phenyl]-2-naphthalenesulfonamide;
(Compound B156) N-[3-(2,4-dioxo-1,2,3,4-tetrahydronaphtho[1,2-b]-[1,4]diazepin-5-yl)phenyl]-1-naphthalenesulfonamide;
(Compound B157) N-[4-(2,4-dioxo-1,2,3,4-tetrahydronaphtho[1,2-b] [1,4]diazepin-5-yl)phenyl]cyclohexanesulfonamide;
(Compound B158) N-[4-(2,4-dioxo-1,2,3,4-tetrahydronaphtho[1,2-b] [1,4]diazepin-5-yl)phenyl]-3-pyridinesulfonamide hydrochloride;
(Compound B159) N-[4-(2,4-dioxo-1,2,3,4-tetrahydronaphtho[1,2-b] [1,4]diazepin-5-yl)phenyl]-4-isopropylbenzenesulfonamide;
(Compound B160) N-[4-(2,4-dioxo-1,2,3,4-tetrahydronaphtho[1,2-b][1,4]diazepin-5-yl)phenyl]phenylmethanesulfonamide;
(Compound B161) N-[4-(2,4-dioxo-1,2,3,4-tetrahydronaphtho[1,2-b] [1,4]diazepin-5-yl)phenyl]-2-thiophene-sulfonamide;
(Compound B162) N-[4-(2,4-dioxo-1,2,3,4-tetrahydronaphtho[1,2-b] [1,4]diazepin-5-yl)phenyl]-2-naphthalenesulfonamide;
(Compound B163) 4-(2,4-dioxo-1,2,3,4,8,9,10,11-octahydronaphtho-[1,2-b][1,4]diazepin-5-yl)phenyl 3-bromobenzene-sulfonate;
(Compound B164) N-benzyl-N-[4-(1-benzyl-2,4-dioxo-1,2,3,4-tetrahydronaphtho[1,2-b][1,4]diazepin-5-yl)phenyl]-2-nitrobenzenesulfonamide;
(Compound B165) N-benzyl-N-[4-(2,4-dioxo-1,2,3,4-tetrahydronaphtho[1,2-b][1,4]diazepin-5-yl)phenyl]-2-nitrobenzenesulfonamide;
(Compound B166) 3-bromo-N-[4-(2,4-dioxo-1,2,3,4-tetrahydronaphtho[1,2-b][1,4]diazepin-5-yl)phenyl]-N-methylbenzenesulfonamide;
(Compound B167) N-[4-(2,4-dioxo-1,2,3,4-tetrahydronaphtho-[1,2-b][1,4]-diazepin-5-yl)phenyl]-N-methyl-2-nitrobenzenesulfonamide;
(Compound B168) N-[4-(2,4-dioxo-1,2,3,4-tetrahydronaphtho-[1,2-b][1,4]-diazepin-5-yl)phenyl]-N-(2-hydroxyethyl)-2-nitrobenzenesulfonamide;
(Compound B169) N-[4-(7-chloro-2,4-dioxo-2,3,4,5-tetrahydro-1H-benzo[b][1,4]diazepin-1-yl)phenyl]benzenesulfonamide;
(Compound B170) N-[4-(7-bromo-2,4-dioxo-2,3,4,5-tetrahydro-1H-benzo[b][1,4]diazepin-1-yl)phenyl]benzenesulfonamide;
(Compound B171) N-[4-[(2,4-dioxo-7-(trifluoromethyl)-2,3,4,5-tetrahydro-1H-benzo[b][1,4]diazepin-1-yl)]phenyl]benzenesulfonamide;
(Compound B172) N-[4-(2,4-dioxo-2,3,4,5-tetrahydro-1H-benzo[b][1,4]diazepin-1-yl)phenyl]benzenesulfonamide;
(Compound B173) 1-(2-chlorophenyl)-N-[4-(2,4-dioxo-1,2,3,4-tetrahydronaphtho[1,2-b][1,4]diazepin-5-yl)phenyl]methanesulfonamide;
(Compound B174) 1-(3-bromophenyl)-N-[4-(2,4-dioxo-1,2,3,4-tetrahydronaphtho[1,2-b][1,4]diazepin-5-yl)phenyl]methanesulfonamide;
(Compound B175) N-[4-(2,4-dioxo-1,2,3,4-tetrahydronaphtho-[1,2-b][1,4]-diazepin-5-yl)phenyl]-2-trifluoromethylbenzenesulfonamide;
(Compound B176) N-[4-(7-bromo-6-methyl-2,4-dioxo-2,3,4,5-tetrahydro-1H-benzo[b] [1,4]diazepin-1-yl)phenyl]benzenesulfonamide;
(Compound B177) 1-(2-chlorophenyl)-N-[4-(2,4-dioxo-1,2,3,4,8,9,10,11-octahydronaphtho[1,2-b][1,4]diazepin-5-yl)phenyl]methanesulfonamide;
(Compound B178) 3-bromo-N-[4-(2,4-dioxo-1,2,3,4,8,9,10,11-octahydronaphtho[1,2-b][1,4]diazepin-5-yl)phenyl]benzenesulfonamide;
(Compound B179) N-[4-(2,4-dioxo-1,2,3,4,8,9,10,11-octahydronaphtho[1,2-b] [1,4]diazepin-5-yl)phenyl]-3-methoxybenzenesulfonamide;
(Compound B180) 1-(2-bromophenyl)-N-[4-(2,4-dioxo-1,2,3,4-tetrahydronaphtho[1,2-b][1,4]diazepin-5-yl)phenyl]methanesulfonamide;
(Compound B181) N-[4-(2,4-dioxo-1,2,3,4-tetrahydronaphtho[1,2-b][1,4]diazepin-5-yl)phenyl]-1-(2-methylphenyl)methanesulfonamide;
(Compound B182) N-[4-(2,4-dioxo-1,2,3,4-tetrahydronaphtho[1,2-b][1,4]diazepin-5-yl)phenyl]-1-(2-nitrophenyl)methanesulfonamide;
(Compound B183) N-[4-(2,4-dioxo-1,2,3,4-tetrahydronaphtho[1,2-b] [1,4]diazepin-5-yl)phenyl]-2-phenylethanesulfonamide;
(Compound B184) 1-(2,3-dichlorophenyl)-N-[4-(2,4-dioxo-1,2,3,4,8,9,10,11-octahydronaphtho[1,2-b][1,4]diazepin-5-yl)phenyl]methanesulfonamide;
(Compound B185) 1-(2-chlorophenyl)-N-[4-(2,4-dioxo-7-methoxy-1H-benzo[1,2-b][1,4]diazepin-1-yl)phenyl]methanesulfonamide;
(Compound B186) 1-(2-chlorophenyl)-N-[4-(2,4-dioxo-7-hydroxy-1H-benzo[1,2-b][1,4]diazepin-1-yl)phenyl]methanesulfonamide;
(Compound B187) 1-(4-chlorophenyl)-N-[4-(2,4-dioxo-1,2,3,4-tetrahydronaphtho[1,2-b][1,4]diazepin-5-yl)phenyl]methanesulfonamide;
(Compound B188) 1-(2-chlorophenyl)-N-[4-(2,4-dioxo-1,2,3,4-tetrahydronaphtho[1,2-b][1,4]diazepin-5-yl)benzyl]methanesulfonamide;
(Compound B189) 1-(2-chlorophenyl)-N-[4-(2,4-dioxo-1,2,3,4-tetrahydronaphtho[1,2-b][1,4]diazepin-5-yl)-2-methoxyphenyl]methanesulfonamide;
(Compound B190) 1-(2-chlorophenyl)-N-[4-(2,4-dioxo-1,2,3,4-tetrahydronaphtho[1,2-b][1,4]diazepin-5-yl)-2-hydroxyphenyl]methanesulfonamide;
(Compound B191) 1-(2,6-dichlorophenyl)-N-[4-(2,4-dioxo-1,2,3,4,8,9,10,11-octahydronaphtho[1,2-b][1,4]diazepin-5-yl)phenyl]methanesulfonamide;
(Compound B192) 1-(2-chlorophenyl)-N-[4-(2,4-dioxo-6-methyl-1H-benzo[1,2-b][1,4]diazepin-1-yl)phenyl]methanesulfonamide;
(Compound B193) 1-(2-chlorophenyl)-N-[3-(2,4-dioxy-1,2,3,4-tetrahydronaphtho[1,2-b][1,4]diazepin-5-yl)propyl]methanesulfonamide;
(Compound B194) 1-(2-chlorophenyl)-N-[2-(2,4-dioxo-1,2,3,4-tetrahydronaphtho[1,2-b][1,4]diazepin-5-yl)ethyl]methanesulfonamide;
(Compound B195) N-[4-(2,4-dioxo-1,2,3,4,8,9,10,11-octahydronaphtho[1,2-b] [1,4]diazepin-5-yl)phenyl]-1-(2-iodophenyl)methanesulfonamide;
(Compound B196) 1-(2-chlorophenyl)-N-[4-(2,4-dioxo-1,2,3,4,8,9,10,11-octahydronaphtho[1,2-b][1,4]diazepin-5-yl)phenyl]-N-methylmethanesulfonamide;
(Compound B197) 1-(2-chlorophenyl)-N-[4-(2-oxo-2,3-dihydro-1H-naphtho[1,2-e] [1,4]diazepin-5-yl)phenyl]methanesulfonamide;
(Compound B198) 1-[2-(trifluoromethyl)phenyl]-N-[4-(2,4-dioxo-1,2,3,4-tetrahydronaphtho[1,2-b][1,4]diazepin-5-yl)phenyl]phenyl-N-methylmethanesulfonamide;
(Compound B199) 1-(2-ethylphenyl)-N-[4-(2,4-dioxo-1,2,3,4-tetrahydronaphtho[1,2-b][1,4]diazepin-5-yl)phenyl]phenyl-N-methylmethanesulfonamide;
(Compound B200) 1-(2,3-dimethylphenyl)-N-[4-(2,4-dioxo-1,2,3,4-tetrahydronaphtho[1,2-b][1,4]diazepin-5-yl)phenyl]phenyl-N-methylmethanesulfonamide;
(Compound B201) 2-(2-chlorophenyl)-N-[4-(2,4-dioxo-1,2,3,4-tetrahydronaphtho[1,2-b][1,4]diazepin-5-yl)phenyl]phenyl-N-methylethanesulfonamide;
(Compound B202) 1-(2-nitrophenyl)-N-[4-(2,4-dioxo-1,2,3,4-tetrahydronaphtho[1,2-b][1,4]diazepin-5-yl)phenyl]phenyl-N-methylmethanesulfonamide;
(Compound B203) 1-(2-aminophenyl)-N-[4-(2,4-dioxo-1,2,3,4-tetrahydronaphtho[1,2-b][1,4]diazepin-5-yl)phenyl]phenyl-N-methylmethanesulfonamide;
(Compound B204) 1-(2-dimethylaminophenyl)-N-[4-(2,4-dioxo-1,2,3,4-tetrahydronaphtho[1,2-b][1,4]diazepin-5-yl)phenyl]phenyl-N-methylmethanesulfonamide;
(Compound B205) 5-[4-[(pyridin-4-yl)carbonylamino]phenyl]-1H-naphtho[1,2-b][1,4]diazepine-2,4(3H,5H)-dione hydrochloride;
(Compound B206) 5-[4-[2-[(pyridin-3-yl)oxy]acetylamino]phenyl]-1H-naphtho[1,2-b][1,4]diazepine-2,4(3H,5H)-dione hydrochloride;
(Compound B207) 5-[4-[(pyridin-3-yl)carbonylamino]phenyl]-1H-naphtho[1,2-b][1,4]diazepine-2,4(3H,5H)-dione hydrochloride;
(Compound B208) 5-[4-[(2-methylpyridin-3-yl)carbonylamino]phenyl]-1H-naphtho[1,2-b][1,4]diazepine-2,4(3H,5H)-dione hydrochloride;
(Compound B209) 5-[4-[(2-chloropyridin-3-yl)carbonylamino]phenyl]-1H-naphtho[1,2-b][1,4]diazepine-2,4(3H,5H)-dione;
(Compound B210) 5-[4-[2-[(pyridin-2-yl)oxy]acetylamino]phenyl]-1H-naphtho[1,2-b][1,4]diazepine-2,4(3H,5H)-dione;
(Compound B211) 5-[4-[[4-(trifluoromethyl)pyridin-3-yl]carbonylamino]phenyl]-1H-naphtho[1,2-b][1,4]diazepine-2,4 (3H, 5H) -dione;
(Compound B212) 5-[4-[(2-chloropyridin-3-yl)carbonylamino]phenyl]-1H-[1,4]diazepino[2,3-f]isoquinoline-2,4(3H,5H)-dione;
(Compound B213) 5-[4-[(2-chloropyridin-3-yl)carbonylamino]phenyl]-8,9,10,11-tetrahydro-1H-[1,4]diazepino[2,3-f]isoquinoline-2,4(3H,5H)-dione; and
(Compound B214) 5-[4-[(2-isopropylbenzoyl)amino]phenyl]-1H-naphtho[1,2-b][1,4]diazepine-2,4(3H,5H)-dione

Stereoisomers of the compounds represented by the formulas, such as a cis-trans isomer, an optically active form, or a racemic form thereof, may exist as well, all of which are comprised in the present invention.

Tautomers of the compounds represented by the formulas may exist as well, and the tautomers show the same activities as those of the compounds and are comprised in the present invention.

Furthermore, the compounds represented by the formulas may have one or two or more asymmetric carbons depending on the type of substituent, and any optical isomer or any mixture or a racemic form of optical isomers, which is based on the asymmetric carbon, or a diastereoisomer or any mixture of diastereoisomers, which is based on two or more asymmetric carbons, may also be used as the active ingredient of the medicament of the present invention. In a case where the compounds represented by the formulas have a double bond or a cyclic structure, a geometric isomer may exist, and, in addition to a pure form of the geometric isomer, a mixture of geometric isomers, which are contained in the mixture at any ratio, may also be used as the active ingredient of the medicament of the present invention.

In addition to the compounds represented by the formulas, any solvate of a free form of the compound or a salt form of the compound may also be used as active ingredient of the medicament of the present invention. The solvate also comprises a hydrate.

In the present specification, the compounds represented by the formulas are not particularly limited, and the compounds represented by the formulas can comprise the stereoisomer of the compound, such as the cis-trans isomer, the optically active form, or the racemic form thereof; the tautomer of the compound; any optical isomer or racemic form of the compound, which is based on an asymmetric carbon; and the diastereoisomer of the compound and a mixture thereof, which is based on two or more asymmetric carbons.

In addition to the compounds represented by the formulas, acid addition salts or base addition salts of these compounds may also be used as the active ingredient of the medicament of the present invention. As the acid addition salt, for example, a mineral acid salt such as hydrochloride, sulfate, and nitrate; an organic acid salt such as methanesulfonate, p-toluenesulfonate, oxalate, and malate; and the like can be used, however, the acid addition salt is not limited thereto. Examples of the base addition salt can comprise a metal salt such as a lithium salt, a sodium salt, a potassium salt, a magnesium salt, and a calcium salt; an ammonium salt; an organic amine salt such as a triethylamine salt and an ethanolamine salt; and the like, however, the base addition salt is not limited thereto. Among these salts, a pharmaceutically acceptable salt is preferably used as the active ingredient of the medicament of the present invention.

The medicament of the present invention can be used for preventing or treating irritable bowel syndrome or inflammatory bowel disease. Preferably, the medicament of the present invention can be used for preventing or treating ulcerative colitis or Crohn's disease which is the inflammatory bowel disease. More preferably, the medicament of the present invention can be used for remission induction therapy or remission maintenance therapy in the inflammatory bowel disease. Even more preferably, the medicament of the present invention can exhibit high effectiveness in remission induction therapy or remission maintenance therapy in the Crohn's disease or the ulcerative colitis.

Furthermore, the medicament of the present invention can be used for suppressing or reducing adhesions or ulcers in an intestinal tissue of the irritable bowel syndrome or the inflammatory bowel disease. Furthermore, the medicament of the present invention can be used for suppressing or reducing a region of transmural inflammation in the intestinal tissue of the irritable bowel syndrome or the inflammatory bowel disease.

Furthermore, the medicament of the present invention can be used for suppressing or reducing histological damage in the intestinal tissue of the irritable bowel syndrome or the inflammatory bowel disease. Preferably, the medicament of the present invention can be used for suppressing or reducing mucosal necrosis in the intestinal tissue of the irritable bowel syndrome or the inflammatory bowel disease. More preferably, the medicament of the present invention can be used for suppressing or reducing cellular infiltration in the intestinal tissue of the irritable bowel syndrome or the inflammatory bowel disease. Even more preferably, the medicament of the present invention can be used for protecting or maintaining mucosa in the intestinal tissue of the irritable bowel syndrome or the inflammatory bowel disease.

As another aspect, the medicament of the present invention can be used for the following uses.

The medicament of the present invention can be used for preventing or treating the irritable bowel syndrome or the inflammatory bowel disease accompanied by adhesions or ulcers in an intestinal tissue.

The medicament of the present invention can be used for preventing or treating the irritable bowel syndrome or the inflammatory bowel disease accompanied by development of a region of transmural inflammation in the intestinal tissue.

The medicament of the present invention can be used for preventing or treating the irritable bowel syndrome or the inflammatory bowel disease accompanied by histological damage in the intestinal tissue.

The medicament of the present invention can be used for preventing or treating the irritable bowel syndrome or the inflammatory bowel disease accompanied by necrosis or damage of mucosa in the intestinal tissue.

The medicament of the present invention can be used for preventing or treating the irritable bowel syndrome or the inflammatory bowel disease accompanied by cellular infiltration in the intestinal tissue.

The medicament of the present invention can be used for preventing or treating the irritable bowel syndrome or the inflammatory bowel disease accompanied by increase in a proinflammatory cytokine.

The medicament of the present invention can be used for preventing or treating the irritable bowel syndrome or the inflammatory bowel disease accompanied by increase in interleukin-1β.

The medicament of the present invention can be used for preventing or treating the irritable bowel syndrome or the inflammatory bowel disease accompanied by colonic damage and increase in a proinflammatory cytokine.

The medicament of the present invention can be used for preventing or treating the irritable bowel syndrome or the inflammatory bowel disease accompanied by colonic damage and increase in interleukin-1β.

All of these inflammatory bowel diseases are subjects of application of the medicament of the present invention.

The medicament of the present invention can be administered by oral administration or parenteral administration. The medicament of the present invention can be manufactured in a suitable dosage form such as a tablet, granules, a powder, a capsule, a suspension, an inhalation, a dry powder inhaler, an inhalation liquid, an aerosol inhaler, an injection, and a suppository, according to a general method in the technical field of pharmaceutical preparations.

These pharmaceutical preparations can be manufactured using a general technique. For example, in a case of the tablet, a general pharmaceutically acceptable additive such as a diluent, a disintegrant, a binder, a lubricant, and a coloring agent can be used. Examples of the diluent can comprise lactose, D-mannitol, microcrystalline cellulose, glucose, and the like. Examples of the disintegrant can comprise starch, carboxymethylcellulose calcium (CMC-Ca), and the like. Examples of the lubricant can comprise magnesium stearate, talc, and the like. Examples of the binder can comprise hydroxypropyl cellulose (HPC), gelatin, polyvinyl pyrrolidone (PVP), and the like.

A pharmaceutically acceptable additive such as a solvent, a stabilizer, a solubilizing agent, a suspension, an emulsifier, an analgesic agent, a buffer, and a preservative is used in adjustment of the injection. The pharmaceutically acceptable additive and a method of preparing a pharmaceutical preparation can be suitably selected by those skilled in the art.

In other words, the medicament of the present invention can be provided as a pharmaceutical composition comprising the active ingredient and the pharmaceutically acceptable additive.

Types of the inhalation for the parenteral administration comprise an aerosol, a dry powder for inhalation, a liquid for inhalation (for example, a solution for inhalation, a suspension for inhalation, and the like), and a capsule inhalation, and the liquid for inhalation may be in a form that is used by being dissolved or suspended in water or another suitable medium at the time of use. The inhalation can be applied using a suitable inhalation container. For example, when administering the liquid for inhalation, a sprayer (an atomizer or a nebulizer) or the like can be used, and when administering the dry powder for inhalation, an inhaler for a dry powder drug or the like can be used.

The inhalation can be manufactured according to a known method. For example, the inhalation is manufactured by obtaining a dry powder or a liquid of the compounds represented by the general formulas (A) to (BII), blending the dry powder or the liquid of the compounds into an inhalation propellant or a carrier, and filling a suitable inhalation container with the blended product. In a case of powderizing the compounds represented by the general formulas (A) to (BII), the powderization is performed according to a general method. For example, a dry powder is prepared by obtaining a fine powder of the compounds and lactose, starch, magnesium stearate, or the like, thereby obtaining a homogeneous mixture, or by granulating the compounds. In addition, in a case where the compounds represented by the general formulas (A) to (BII) are liquefied, for example, the compounds may be dissolved in a liquid carrier such as water, physiological saline, and an organic solvent. As the propellant, a conventionally known propellant, for example, alternative chlorofluorocarbon, a liquefied gas propellant (for example, fluorohydrocarbon, liquefied petroleum, diethyl ether, dimethyl ether, and the like), a compressed gas (for example, a soluble gas (for example, carbon dioxide gas, nitrous oxide gas, and the like), an insoluble gas (for example, nitrogen gas and the like), and the like are used.

A pharmaceutically acceptable additive may be suitably blended into the inhalation as necessary. The additive may be any additive that is generally used. For example, a solid diluent (for example, sucrose, lactose, glucose, mannitol, sorbitol, maltose, cellulose, and the like), a liquid diluent (for example, propylene glycol and the like), a binder (starch, dextrin, methylcellulose, hydroxypropylcellulose, polyvinylpyrrolidone, polyethylene glycol, sucrose, and the like), a lubricant (for example, magnesium stearate, light anhydrous silicic acid, talc, sodium lauryl sulfate, and the like), a corrigent (for example, citric acid, menthol, glycyrrhizin ammonium salt, glycine, an orange powder, and the like), a preservative (for example, sodium benzoate, sodium bisulfite, methylparaben, propylparaben, and the like), a stabilizer (for example, citric acid, sodium citrate, and the like), a suspending agent or an emulsifier (for example, methyl cellulose, polyvinyl pyrrolidone, polyvinyl alcohol, lecithin, sorbitan trioleate, and the like), a dispersing agent (for example, a surfactant and the like), a solvent (for example, water and the like), a tonicity agent (for example, sodium chloride, concentrated glycerin, and the like), a pH regulator (for example, hydrochloric acid, sulfuric acid, and the like), a solubilizer (for example, ethanol and the like), an antiseptic agent (benzalkonium chloride, paraben, and the like), a colorant, a buffer agent (sodium phosphate, sodium acetate, and the like), a thickening agent (cariboxyvinyl polymer and the like), an absorption promoter, and the like are used. The liquid for inhalation is prepared by, for example, suitably selecting the antiseptic agent, the colorant, the buffer agent, the tonicity agent, the thickening agent, the absorption promoter, or the like as necessary. Furthermore, the dry powder for inhalation is prepared by, for example, suitably selecting the lubricant, the binder, the diluent, the colorant, the antiseptic agent, the absorption promoter (bile salt, chitosan, and the like), or the like as necessary.

Furthermore, in order to prepare the compounds represented by the general formulas (A) to (BII) in a sustained release form, the inhalation may contain a biodegradable polymer. Examples of the biodegradable polymer can comprise a fatty acid ester polymer or a copolymer thereof, polyacrylic acid esters, polyhydroxybutyric acids, polyalkylene oxalates, polyorthoester, polycarbonate, and polyamino acids, and one polymer can be used alone, or two or more thereof can be used in combination. Furthermore, a phospholipid such as egg yolk lecithin, chitosan, or the like may also be used. Examples of the fatty acid ester polymer or a copolymer thereof can comprise polylactic acid, polyglycolic acid, polycitric acid, polymalic acid, and a lactic acid-glycolic acid copolymer, and one polymer or copolymer can be used alone, or two or more thereof can be used in combination. In addition, one of poly-α-cyanoacrylic acid ester, poly-β-hydroxybutyric acid, polytrimethylene oxide, polyorthoester, polyorthocarbonate, polyethylenecarbonate, poly-γ-benzyl-L-glutamic acid, and poly-L-alanine can be used alone, or two or more thereof can be used in combination. The fatty acid ester polymer or a copolymer thereof is preferably polylactic acid, polyglycolic acid, or a lactic acid-glycolic acid copolymer, and is more preferably a lactic acid-glycolic acid copolymer. Furthermore, a microsphere or a nanosphere may be prepared by encapsulating a drug using the biodegradable polymer such as a lactic acid-glycolic acid copolymer.

A dose of the medicament of the present invention is not particularly limited, and generally, to an adult,
approximately 0.01 µg to 100 mg, and preferably 0.3 µg to 10 mg can be administered per day as an active ingredient amount, in a case of administration by an inhalation, a dry powder inhaler, an inhalation liquid, and an aerosol inhaler,
approximately 0.01 mg to 100 mg can be administered per day as an active ingredient amount, in a case of administration by an injection, and
approximately 0.01 mg to 2,000 mg can be administered per day, in a case of oral administration. Note that the dose is not limited to the above doses, and the dose can be increased or decreased depending on the age or a symptom.

The medicament of the present invention can be used in combination with another drug that is useful in treating or preventing various irritable bowel syndromes or inflammatory bowel diseases. Individual components in such combination can be administered in divided pharmaceutical preparations or in a single pharmaceutical preparation separately at different times or at the same time, during the period of treatment or prevention. Therefore, it should be understood that the present invention comprises both administration at the same time or administrations at different times, and the administration in the present invention should be understood in such a way. A range of the combination of the medicament of the present invention and another drug that is useful in treating or preventing various irritable bowel syndromes or inflammatory bowel diseases comprises, in principle, a combination with a certain pharmaceutical preparation that is useful in treating or preventing various irritable bowel syndromes or inflammatory bowel diseases.

Each pharmaceutical preparation in the combined pharmaceutical preparation according to the present invention can be selected in various forms and can be manufactured with the above pharmaceutical preparations at the same time. Furthermore, a drug combination which comprises the medicament of the present invention and another drug for treating or preventing various irritable bowel syndromes or inflammatory bowel diseases can also be easily manufactured by those skilled in the art according to a conventional method or a conventional art.

The combination comprises not only a combination of the medicament of the present invention and one other active substance, but also a combination of the medicament of the present invention and two or more other active substances. There are many examples of the combination of the medicament of the present invention and one, two, or more activators selected from the drug for treating or preventing various irritable bowel syndromes or inflammatory bowel diseases.

### [Examples]

Hereinafter, the present invention is more specifically described with Examples. The scope of the present invention is limited to the following compounds:
(A16) 5-[3-(5-thioxo-4H-[1,2,4]oxadiazol-3-yl)phenyl]-1H-naphtho[1,2-b][1,4]diazepine-2,4(3H,5H)-dione;
(A17) a 5-[3-(5-thioxo-4H-[1,2,4]oxadiazol-3-yl)phenyl]-1H-naphtho[1,2-b][1,4]diazepine-2,4(3H,5H)-dione sodium salt; and
(B48) 5-[4-(2-iodobenzoylamino)phenyl]-1H-naphtho[1,2-b][1,4]diazepine-2,4(3H,5H)-dione.

In the following Examples, as a P2X4 receptor antagonist, a 5-[3-(5-thioxo-4H-[1,2,4]oxadiazol-3-yl)phenyl]-1H-naphtho[1,2-b][1,4]diazepine-2,4(3H,5H)-dione sodium salt (a compound in Example 14 of WO 2010/093061 A: hereinafter, referred to as "Compound A"), 5-[4-(2-iodobenzoylamino)phenyl]-1H-naphtho[1,2-b][1,4]diazepine-2,4(3H,5H)-dione (a compound in Example 48 of WO 2013/105608 A: hereinafter, referred to as "Compound B"), and other compounds shown below (not part of the claimed invention) were used:
a 5-[3-(1H-tetrazol-5-yl)phenyl]-1H-naphtho[1,2-b][1,4]diazepine-2,4(3H,5H)-dione sodium salt; 5-[4-[2-(trifluoromethyl)benzoyl]aminophenyl]-1H-naphtho[1,2-b][1,4]diazepine-2,4(3H,5H)-dione; 5-[4-[(2-chlorophenylacetyl)amino]phenyl]-1H-naphtho[1,2-b][1,4]diazepine-2,4(3H,5H)-dione; 1-[4-(2,4-dioxo-1,2,3,4-tetrahydronaphtho[1,2-b] [1,4]diazepin-5-yl)phenyl]-3-phenylurea; 5-[4-[(2-ethylbenzoyl)amino]phenyl]-1H-naphtho[1,2-b][1,4]diazepine-2,4(3H,5H)-dione; 5-[4-(2-tert-butylbenzoylamino)phenyl]-1H-naphtho[1,2-b][1,4]diazepine-2,4(3H,5H)-dione; 5-[4-(2-iodobenzoyl)aminophenyl]-1,2,3,4,8,9,10,11-octahydronaphtho[1,2-b][1,4]diazepine-2,4(3H,5H)-dione; 5-[4-(6-chloro-2-hydroxybenzoylamino)phenyl]-1H-naphtho[1,2-b][1,4]diazepine-2,4(3H,5H)-dione; N-[4-(2,4-dioxo-1,2,3,4-tetrahydronaphtho[1,2-b][1,4]diazepin-5-yl)phenyl]benzenesulfonamide; 1-(2-chlorophenyl)-N-[4-(2,4-dioxo-1,2,3,4-tetrahydronaphtho[1,2-b][1,4]diazepin-5-yl)phenyl]methanesulfonamide; 1-(2-chlorophenyl)-N-[4-(2,4-dioxo-1,2,3,4,8,9,10,11-octahydronaphtho[1,2-b][1,4]diazepin-5-yl)phenyl]methanesulfonamide; 1-(2-bromophenyl)-N-[4-(2,4-dioxo-1,2,3,4-tetrahydronaphtho[1,2-b][1,4]diazepin-5-yl)phenyl]methanesulfonamide; N-[4-(2,4-dioxo-1,2,3,4-tetrahydronaphtho[1,2-b][1,4]diazepin-5-yl)phenyl]-1-(2-methylphenyl)methanesulfonamide; N-[4-(2,4-dioxo-1,2,3,4-tetrahydronaphtho[1,2-b][1,4]diazepin-5-yl)phenyl]-2-phenylethanesulfonamide; 1-(2-chlorophenyl)-N-[4-(2,4-dioxo-1,2,3,4,8,9,10,11-octahydronaphtho[1,2-b][1,4]diazepin-5-yl)phenyl]-N-methylmethanesulfonamide; 1-(2-chlorophenyl)-N-[4-(2-oxo-2, 3-dihydro-1H-naphtho [1, 2-e][1,4]diazepin-5-yl)phenyl]methanesulfonamide; 5-[4-[(2-methylpyridin-3-yl)carbonylamino]phenyl]-1H-naphtho[1,2-b][1,4]diazepine-2,4(3H,5H)-dione hydrochloride; 5-[4-[(2-chloropyridin-3-yl)carbonylamino]phenyl]-1H-naphtho[1,2-b][1,4]diazepine-2,4(3H,5H)-dione; 5-[4-[2-[(pyridin-2-yl)oxy]acetylamino]phenyl]-1H-naphtho[1,2-b][1,4]diazepine-2,4(3H,5H)-dione; and 5-[4-[(2-isopropylbenzoyl)amino]phenyl]-1H-naphtho[1,2-b][1,4]diazepine-2,4(3H,5H)-dione.

Note that, in Examples of the present application, significant differences are indicated with symbols "*" and "a" in Figs. 1 to 8 and Figs. 10 to 13, each of which indicates that P < 0.05. In addition, in Figs. 1 to 8 and Figs. 10 to 13, the symbol "*" indicates that there is a significant difference as a result of comparison with control rats, and the symbol "a" indicates that there is a significant difference as a result of comparison with a DNBS-induced colitis model.

Furthermore, the number of samples in Examples of the present application was 8 to 10.

### Example 1: Measurement of P2X4 Receptor Antagonistic Action of Compound Which Is Active Ingredient of Present Invention

### (Test Method)

A P2X4 receptor antagonistic action of a compound which is the active ingredient of the present invention was measured. 1321N1 cells into which ATP receptors (human P2X4) were introduced were used as a P2X4 receptor stable expression system. The P2X4-receptor-expressing cells were seeded into a 96-well plate and cultured for 24 hours under a condition of 37°C and 5% CO2 to be used for calcium measurement. Fura-2AM, which is a calcium fluorescent indicator, was dissolved in an extracellular fluid for calcium imaging, and the seeded cells were treated therewith and left to stand at room temperature for 45 minutes, thereby incorporating Fura-2AM into the cells. EnVision (PerkinElmer) which is a microplate reader was used for the measurement. 510 nm fluorescences F340 and F380 that are emitted when the cells are irradiated with light radiating from a xenon lamp and transmitted through 340 nm and 380 nm filters, respectively, were observed, and change in a ratio value F340/F380 was used as an index of intracellular calcium change. Measurement was performed by adding ATP to each well such that the final concentration of ATP becomes 1 µM and observing an ATP-induced intracellular calcium response over time. Inhibitory activity of a test substance was measured by performing pretreatment on the test substance for 15 minutes by adding ATP thereto, and the inhibitory activity was calculated by comparison with a case of absence of the test substance. The results are shown in the following Table 29.

### (Test Result)

**[Table 29]**

| Test compound | IC₅₀(µM) |
|---|---|
| CompoundA2 | 0.53 |
| Compound A17(Compound A) | 0.27 |
| Compound B2 | 0.75 |
| Compound B13 | 0.54 |
| Compound B20 | 1.2 |
| Compound B48(Compound B) | 0.30 |
| Compound B57 | 0.72 |
| Compound B71 | 0.4 |
| Compound B106 | 1.8 |
| Compound B118 | 1.10 |
| Compound B146 | 0.67 |
| Compound B173 | 0.064 |
| Compound B177 | 0.056 |
| Compound B180 | 0.05 |
| Compound B181 | 0.068 |
| Compound B183 | 0.42 |
| Compound B196 | 0.97 |
| Compound B197 | 0.44 |
| Compound B208 | 1.3 |
| Compound B209 | 0.94 |
| Compound B210 | 1.4 |
| Compound B214 | 0.62 |

### Example 2 (Preparation of Model Animal to Be Used)

Sprague-Dawley rats (body weights of 200 to 225 g) which were male albino rats were used as model animals. Three model animals were housed in one cage in a temperature-controlled environment at 22-24°C, 50-60% humidity, and a light cycle of 12 hours, were given ad libitum access to standard laboratory feed and tap water, and were not used for at least one week after being brought into the laboratory.

The model animals were reared and handled in accordance with the provision of Directive 210/63/UE of the Council of the European Communities approved and adopted by the Italian government. The experiment was approved by the Ethics Committee on Animal Experimentation of the University of Pisa and the Italian Ministry of Health (authorization no. 674/2016-PR).

### (Preparation of DNBS-Induced Colitis Model)

A model in which colitis was induced by 2,4-dinitrobenzenesulfonic acid (hereinafter, denoted as DNBS, manufactured by Sigma-Aldrich, Inc.) (in the present specification, referred to as a "DNBS-induced colitis model") was prepared.

### (Induction of Colitis)

The induction of colitis was performed according to the method in Antonioli L, et al. (Non Patent Literature 8). 15 mg of 2,4-dinitrobenzenesulfonic acid (DNBS) in 0.25 ml of 50% ethanol was rectally administered through a polyethylene PE-60 catheter inserted 8 cm proximal to the anus, during a short period of anesthesia by Isoflurane (manufactured by Abbott Laboratories). To the control rat, 0.25 ml of 50% ethanol was administered.

### (Control Rat)

Among the Sprague-Dawley rats (body weights of 200 to 225 g), which were male albino rats, model animals that were not treated with DNBS were used as a control group (in the present specification, referred to as "control rats"). Note that, in the drawings, control rats are expressed as Control.

An anti-inflammatory effect of Compound A on the DNBS model was confirmed in Examples 3 to 8.

The test compound that was used, an administration method, observation of the model during the test, and sample preparation were performed by the following methods.

### (Test Compound, Administration Method, and the Like)

Compound A (3, 10, and 30 mg/kg) or dexamethasone (1 mg/kg) was orally administered to the rats immediately after the delivery of DNBS.

Thus, a remission maintenance therapy effect of Compound A can be confirmed in the present experiment.

In addition, Compound A (30 mg/kg) was orally administered to the control rats, separately from the above group.

Note that, in Examples 3 to 8, a group that is simply expressed as a "group administered with Compound A" or a "group administered with dexamethasone" refers to a group administered with Compound A or dexamethasone immediately after the delivery of DNBS.

### (Observation of DNBS-Induced Colitis Model During Test)

Food intake was monitored everyday since initiation of the drug therapy. Furthermore, body weights of the rats were measured every morning, and evidence of blood in diarrhea and a stool or rectal bleeding was examined during the experimental period.

A formed pellet stool was defined as normal, a pasty and semi-formed stool that does not stick to the anus was defined as a loose stool, and a case of presence of a stool that adheres to the fur or the fur around the tail is defined as diarrhea. Furthermore, a case of presence of blood visible around the rectal area of an animal was defined as the rectal bleeding.

### (Sample Preparation)

The DNBS-induced colitis model and the control rats were euthanized in a CO₂ chamber. After death, mid-abdominal incision was performed, and the colon was excised, which was then opened in a longitudinal direction (longitudinal muscle direction), washed with sterile physiological saline, and split into two parts in the longitudinal direction.

### Example 3 (Change in Body Weight and Spleen Weight)

Significant decrease in body weights was recorded in the DNBS-induced colitis model. Specifically, the body weights decreased by -15 ± 5 g (-37.6 ± 4.4%) on average 6 days after the DNBS administration. On the other hand, the control rats showed increase in body weights (+37.5 ± 8.7 g, (+23.5 ± 3.8%)) (Fig. 1A).

Body weights also decreased in the group administered with dexamethasone (1 mg/kg), whereas increase in body weights was observed in the groups administered with Compound A (3, 10, and 30 mg/kg) (Fig. 1A).

Here, a spleen weight is assumed as an index of systematic inflammation. Significant increase in spleen weights was observed (+42%) in the DNBS-induced colitis model (Fig. 1B).

On the other hand, in the groups administered with Compound A and dexamethasone, the spleen weights significantly decreased compared to the DNBS-induced colitis model (Fig. 1B).

As a result, significant improvement with respect to the body weight decrease and the spleen weight increase in the DNBS-induced colitis model by the administration of Compound A was confirmed.

### Example 4 (Macroscopic Damage)

Evaluation of macroscopic damage was performed by the following method.

### (Evaluation)

Evaluation was performed by scoring the macroscopic damage of the samples. As described above, colonic tissues that had been excised and washed with physiological saline were used as the samples.

The scoring was performed according to the criteria determined by making a minor amendment on the criteria reported in Non Patent Literature 11 (Non Patent Literature 12). The macroscopic scoring criteria for colonic damage are as shown in the following (1) to (4) (Non Patent Literatures 8 to 10), and the total points in (1) to (4) are used as a score.
(1) Regarding presence of ulcers No ulcers: 0 points, hyperemia with no ulcers: 1 point, ulcers without hyperemia or thickening of an intestinal wall: 2 points, ulcers with inflammation at one site: 3 points, ulcers with inflammation at two or more sites: 4 points, a wide site of damage extending more than 1 cm along the length of the colon: 5 points, and a wide site of damage extending more than 2 cm along the length of the colon: 6 points
(2) Regarding presence of adhesion between colonic tissue and other organs No adhesions: 0 points, adhesion with only one adjacent organ: 1 point, and adhesions with a plurality of adjacent organs: 2 points
(3) Regarding hardness of stool Formed stool: 0 points, loose stool: 1 point, and liquid stool: 2 points
(4) Thickness (mm) of colonic tissue (colonic wall): 1 point was scored for 1 mm

### (Confirmation of Anti-Inflammatory Action of Compound A)

6 days after the DNBS administration, distal colons appeared thickened and ulcerated, with apparent areas of transmural inflammation, in the DNBS-induced colitis model. Adhesions were often observed, intestines dilated occasionally, and the score for the macroscopic damage was 7.8 ± 1.4.

On the other hand, the groups administered with Compound A at doses of 10 and 30 mg/kg showed significant decrease in the macroscopic damage scores compared to the DNBS-induced colitis model (Fig. 2). The group administered with dexamethasone showed significant decrease in the macroscopic damage score compared to the DNBS-induced colitis model (Fig. 2).

As a result, significant improvement in the tissue parameter (macroscopic score) by the administration of Compound A was confirmed.

### Example 5 (Microscopic Damage)

Evaluation of microscopic damage was performed by the following method.

### (Evaluation)

Evaluation of microscopic damage and inflammation was performed with an optical microscope using a tissue section obtained from a specimen of the entire intestine and stained with hematoxylin/eosin. Evaluation of severity of enteritis was performed by scoring the microscopic damage (histological damage) of the samples. As described above, colonic tissues that had been excised and washed with physiological saline were used as the samples.

The scoring was performed according to the criteria determined by making a minor amendment on the criteria reported in Non Patent Literature 11 (Non Patent Literature 12). The microscopic scoring (scoring of histological damage) criteria for the colonic damage are as shown in the following (1) to (5) (Non Patent Literatures 8 to 10), and the total points in (1) to (5) are used as a score.

Histological criteria:
(1) Loss of mucosal structure (no loss of mucosal structure: 0 points and mild to severe loss of mucosal structure: 1 to 3 points)
(2) Cellular infiltration (no cellular infiltration: 0 points and mild to severe cellular infiltration: 1 to 3 points)
(3) Muscle thickening (no muscle thickening: 0 points and mild to severe muscle thickening: 1 to 3 points)
(4) Crypt abscess (none: 0 points and present: 1 point)
(5) Reduction in goblet cells (not reduced: 0 points and reduced: 1 point)

### (Confirmation of Anti-Inflammatory Action of Compound A)

In histological examination of colon specimens of the DNBS-induced colitis model, colitis was clearly recognized, the gland structure was completely destroyed, and association with a wide area of mucosal necrosis was shown. A submucosal tissue was thickened by marked infiltration of inflammatory cells associated with edema and vasodilation. These changes were occasionally associated with a muscle layer, and the muscle layer seemed to be thickened as well. The mucosa and the submucosal tissue surrounding the necrosis area showed inflammation associated with the marked cellular infiltration, compared to the tissues obtained from the control rats (Fig. 3).

6 days after the DNBS administration, severe damage was shown in colon sections of the rats in which inflammation was induced (DNBS-induced colitis model) (Fig. 3).

In the colonic tissues obtained from the groups administered with Compound A, histological damage was not affected, compared to the colonic tissues of the DNBS-induced colitis model (Fig. 3). On the other hand, the colonic tissues obtained from the group administered with 1 mg/kg of dexamethasone showed significant improvement in the microscopic score compared to the colonic tissues of the DNBS-induced colitis model (Fig. 3).

### Example 6 (TNF and IL-1β Assay)

Proinflammatory cytokines (tumor necrosis factor (in the present specification, referred to as TNF) and interleukin-1β (in the present specification, referred to as IL-1β)) in the colonic tissues of the DNBS-induced colitis model that are subjected to the colonic damage and the proinflammatory cytokines in the case of the administration of Compound A were evaluated.

### (Sample Preparation)

A tissue sample stored at -80°C was weighed and thawed. The tissue was homogenized at 4°C with 0.3 ml of PBS (pH 7.2) per 100 mg of the tissue and centrifuged for 20 minutes at 13,400 g and at a rotation speed of 10,894 rpm. Then, an aliquot (100 µl) of a supernatant was used for the assay.

### (Evaluation of Inflammation Inducibility Index in Tissue)

Levels of the proinflammatory cytokines TNF and IL-1β in tissues were measured with an enzyme-linked immunosorbent assay (ELISA) kit (BioSource International, Camarillo, CA).

### (Confirmation of Anti-Inflammatory Action of Compound A)

The colonic damage in the DNBS-induced colitis model was associated with significant increase in both TNF (increased from 2.2 ± 0.3 to 12.4 ± 0.8 pg/mg) (Fig. 4A) and IL-1β (increased from 9.3 ± 0.4 to 32.5 ± 1.4 pg/mg) (Fig. 4B).

In the group administered with Compound A, these inflammation indices were not affected (Figs. 4A and 4B). In contrast, inflammation was completely prevented in the group administered with dexamethasone (Figs. 4A and 4B).

### Example 7 (Occludin Expression in Colonic Tissue)

An occludin (an integral plasma-membrane protein located at tight junctions) expression effect in the colonic tissues of the DNBS-induced colitis model that are subjected to the colonic damage and the expression effect in the case of the administration of Compound A were observed by western blotting.

### (Sample Preparation)

The weight of the colonic tissue (30 µg of proteins) was measured, and the tissue was homogenized in a lysis buffer.

### (Test Method)

Proteins were quantified by the Bradford method. The proteins were separated with a precast 4-20% polyacrylamide gel (manufactured by Bio-Rad Laboratories, Inc., Mini-PROTEAN^{®} TGX gel) and then transferred onto a PVDF membrane (manufactured by Bio-Rad Laboratories, Inc., Trans-Blot^{®} TurboTM PVDF Transfer packs). The membrane was blocked with 3% BSA diluted with Tris-buffered saline (TBS, 20 mM Tris-HCl, pH 7.5, 150 mM NaCl) containing 0.1% Tween 20. An anti-occludin antibody (ab167161, manufactured by Abcam plc., dilution 1 : 5,000) was used as the antibody. Protein bands were detected using an ECL reagent (Clarity^{®} Western ECL Blotting Substrate, Bio-Rad Laboratories, Inc.). Concentrations were measured with ImageJ software.

### (Observation of Occludin Expression)

Colonic mucosa of the DNBS-induced colitis model showed significant decrease in occludin compared to the control rats (Fig. 5).

The occludin expression increased in inflamed tissues of the group administered with Compound A (10 mg/kg) (Fig. 5) or dexamethasone (1 mg/kg, not shown in the drawing), compared to the DNBS-induced colitis model.

From this result, it was understood that Compound A counteracts the decrease in tight junction occludin observed in the DNBS-induced colitis model. Therefore, it was understood that Compound A contributes in maintaining health of the large intestinal tissue.

### Example 8 (Procaspase-1 and Caspase-1 Expressions in Colonic Tissue)

Procaspase-1 and caspase-1 expression effects in the colonic tissues of the DNBS-induced colitis model that are subjected to the colonic damage and the expression effects in the case of the administration of Compound A were observed by western blotting. Sample preparation and a test method were performed by the same methods as those in Example 7.

### (Sample Preparation and Test Method)

Samples were prepared and a test was performed in the same manner as in Example 7, except that an anti-procaspase-1 antibody and an anti-caspase antibody (all ad1872, manufactured by Abcam plc., dilution 1 : 1,000) were used as the antibodies, instead of the anti-occludin antibody.

### (Observation of Procaspase-1 and Caspase-1 Expressions)

Increase in the procaspase-1 expression was observed in the DNBS-induced colitis model by the western blotting (Fig. 6A). In a parallel way, induction of colitis in the DNBS-induced colitis model was associated with increase in the caspase-1 expression at the colon level (Fig. 6B).

The group administered with Compound A (10 mg/kg) showed that the caspase-1 expression cannot be decreased in a colon in which inflammation was induced (Fig. 6B).

An anti-inflammatory effect of Compound B on the DNBS model was confirmed in Examples 9 to 14.

The test compound that was used and an administration method were performed by the following methods.

### (Test Compound, Administration Method, and the Like)

Compound B (3, 10, and 30 mg/kg) or dexamethasone (1 mg/kg) was orally administered to the rats immediately after the delivery of DNBS.

Thus, a remission maintenance therapy effect of Compound B can be confirmed in the present experiment.

In addition, Compound B (30 mg/kg) was orally administered to the control rats, separately from the above group.

Note that, in Examples 9 to 14, a group that is simply expressed as a "group administered with Compound B" or a "group administered with dexamethasone" refers to a group administered with Compound B or dexamethasone immediately after the delivery of DNBS.

Observation of the model during the test and sample preparation were performed by the same methods as the above-described methods used for testing Compound A.

### Example 9 (Change in Body Weight and Spleen Weight)

Significant decrease in body weights was recorded in the DNBS-induced colitis model. Specifically, the body weights decreased by -15 ± 5 g (-37.6 ± 4.4%) on average 6 days after the DNBS administration. On the other hand, the control rats showed increase in body weights (+37.5 ± 8.7 g, (+23.5 ± 3.8%)) (Fig. 7A).

Body weights also decreased in the group administered with dexamethasone (1 mg/kg), whereas increase in body weights was observed in the groups administered with Compound B (3, 10, and 30 mg/kg) (Fig. 7A).

Here, a spleen weight is assumed as an index of systematic inflammation. Significant increase in spleen weights was observed (+42%) in the DNBS-induced colitis model (Fig. 7B).

On the other hand, in the groups administered with Compound B and dexamethasone, the spleen weights significantly decreased compared to the DNBS-induced colitis model (Fig. 7B).

As a result, significant improvement with respect to the body weight decrease and the spleen weight increase in the DNBS-induced colitis model by the administration of Compound B was confirmed.

### Example 10 (Macroscopic Damage)

Evaluation of macroscopic damage was performed by the same method as that in Example 4.

### (Confirmation of Anti-Inflammatory Action of Compound B)

6 days after the DNBS administration, distal colons appeared thickened and ulcerated, with apparent areas of transmural inflammation, in the DNBS-induced colitis model. Adhesions were often observed, intestines dilated occasionally, and the macroscopic damage scored 7.8 ± 1.4.

On the other hand, the groups administered with Compound B at doses of 3, 10, and 30 mg/kg showed significant decrease in the macroscopic damage scores compared to the DNBS-induced colitis model (Fig. 8). The group administered with dexamethasone showed significant decrease in the macroscopic damage score compared to the DNBS-induced colitis model (Fig. 8).

As a result, significant improvement in the tissue parameter (macroscopic score) by the administration of Compound B was confirmed.

### Example 11 (Microscopic Damage)

Evaluation of microscopic damage was performed by the same method as that in Example 5.

### (Confirmation of Anti-Inflammatory Action of Compound B)

In histological examination of colon specimens of the DNBS-induced colitis model, colitis was clearly recognized, the gland structure was completely destroyed, and association with a wide area of mucosal necrosis was shown. A submucosal tissue was thickened by marked infiltration of inflammatory cells associated with edema and vasodilation. These changes were occasionally associated with a muscle layer, and the muscle layer seemed to be thickened as well. The mucosa and the submucosal tissue surrounding the necrosis area showed inflammation associated with the marked cellular infiltration (Fig. 9B), compared to the tissues obtained from the control rats (Fig. 9A).

6 days after the DNBS administration, severe damage was shown in colon sections of the rats in which inflammation was induced (DNBS-induced colitis model) (Fig. 9B and Fig. 10). In the colonic tissues obtained from the groups administered with Compound B, histological damage decreased in a dose-dependent manner, compared to the colonic tissues of the DNBS-induced colitis model (Figs. 9C-E and Fig. 10). The groups administered with Compound B at doses of 10 and 30 mg/kg showed significant improvement in the microscopic score compared to the colonic tissues of the DNBS-induced colitis model (Fig. 10). The colonic tissues obtained from the group administered with 1 mg/kg of dexamethasone showed significant improvement in the microscopic score compared to the colonic tissues of the DNBS-induced colitis model (Fig. 9F and Fig. 10).

### Example 12 (TNF and IL-1β Assay)

Proinflammatory cytokines (TNF and IL-1β) in the colonic tissues of the DNBS-induced colitis model that are subjected to the colonic damage and the proinflammatory cytokines in the case of the administration of Compound B were evaluated.

Sample preparation for the evaluation and the evaluation of inflammation inducibility indices (TNF and IL-1β) in a tissue were performed by the same methods as those in Example 6.

### (Confirmation of Anti-Inflammatory Action of Compound B)

The colonic damage in the DNBS-induced colitis model was associated with dominant increase in TNF (increased from 4.5 ± 0.4 to 12.1 ± 1.7 pg/mg). In the group administered with Compound B, this inflammation index was not affected (Fig. 11A). In contrast, inflammation was completely prevented in the group administered with 1 mg/kg of dexamethasone (Fig. 11A).

Furthermore, the DNBS-induced colitis model was associated with significant increase in IL-1β (increased from 9.5 ± 5.4 to 32.7 ± 1.8 pg/mg) (Fig. 11B). In the groups administered with Compound B (3, 10, and 30 mg/kg) and the group administered with dexamethasone (1 mg/kg), tissue IL-1β significantly decreased compared to the DNBS-induced colitis model (Fig. 11B).

Therefore, it was understood that Compound B has an effect of decreasing the proinflammatory cytokine IL-1β.

### Example 13 (Occludin Expression in Colonic Tissue)

An occludin (an integral plasma-membrane protein located at tight junctions) expression effect in the colonic tissues of the DNBS-induced colitis model that are subjected to the colonic damage and the expression effect in the case of the administration of Compound B were observed by western blotting. Sample preparation and a test method were performed by the same methods as those in Example 7.

### (Observation of Occludin Expression)

Colonic mucosa of the DNBS-induced colitis model showed significant decrease in occludin compared to the control rats (Fig. 12).

The occludin expression increased in inflamed tissues of the group administered with Compound B (10 mg/kg) (Fig. 12) or dexamethasone (1 mg/kg, not shown in the drawing), compared to that in the DNBS-induced colitis model.

From this result, it was understood that Compound B counteracts the decrease in tight junction occludin observed in the DNBS-induced colitis model. Therefore, it was understood that Compound B contributes in maintaining health of the large intestinal tissue.

### Example 14 (Procaspase-1 and Caspase-1 Expressions in Colonic Tissue)

Procaspase-1 and caspase-1 expression effects in the colonic tissues of the DNBS-induced colitis model that are subjected to the colonic damage and the expression effects in the case of the administration of Compound B were observed by western blotting. Sample preparation and a test method were performed by the same methods as those in Example 8.

### (Observation of Procaspase-1 and Caspase-1 Expressions)

Increase in the procaspase-1 expression was observed in the DNBS-induced colitis model by the western blotting (Fig. 13A). In a parallel way, induction of colitis in the DNBS-induced colitis model was associated with the caspase-1 expression at the colon level (Fig. 13B).

The group administered with Compound B (10 mg/kg) showed a decreased caspase-1 expression in the colon in which inflammation was induced, compared to the DNBS-induced colitis model (Fig. 13B).

### Industrial Applicability

The medicament of the present invention can be used as a medicament for remission induction therapy or remission maintenance therapy in the inflammatory bowel disease.

The medicament of the present invention can be used as a medicament for the remission induction therapy or the remission maintenance therapy in Crohn's disease or the ulcerative colitis.

The medicament of the present invention can be used as a medicament for suppressing or reducing adhesions or ulcers in an intestinal tissue of the irritable bowel syndrome or the inflammatory bowel disease.

The medicament of the present invention can be used as a medicament for suppressing or reducing a region of transmural inflammation in the intestinal tissue of the irritable bowel syndrome or the inflammatory bowel disease.

The medicament of the present invention can be used as a medicament for suppressing or reducing histological damage in the intestinal tissue of the irritable bowel syndrome or the inflammatory bowel disease.

The medicament of the present invention can be used as a medicament for suppressing or reducing mucosal necrosis in the intestinal tissue of the irritable bowel syndrome or the inflammatory bowel disease.

The medicament of the present invention can be used as a medicament for suppressing or reducing cellular infiltration in the intestinal tissue of the irritable bowel syndrome or the inflammatory bowel disease.

The medicament of the present invention can be used as a medicament for protecting or maintaining mucosa in the intestinal tissue of the irritable bowel syndrome or the inflammatory bowel disease.

## Claims

1. A medicament for use in preventing or treating irritable bowel syndrome or inflammatory bowel disease, which comprises a compound having a P2X4 receptor antagonistic action or a pharmaceutically acceptable salt thereof as an active ingredient, which is selected from the group consisting of:
(A16) 5-[3-(5-thioxo-4H-[1,2,4]oxadiazol-3-yl)phenyl]-1H-naphtho[1,2-b][1,4]diazepine-2,4(3H,5H)-dione;
(A17) a 5-[3-(5-thioxo-4H-[1,2,4]oxadiazol-3-yl)phenyl]-1H-naphtho[1,2-b][1,4]diazepine-2,4(3H,5H)-dione sodium salt; and
(B48) 5-[4-(2-iodobenzoylamino)phenyl]-1H-naphtho[1,2-b][1,4]diazepine-2,4(3H,5H)-dione.

2. The medicament for use according to claim 1, wherein the inflammatory bowel disease is Crohn's disease or ulcerative colitis.

3. The medicament for use according to claim 1 or 2, wherein preventing or treating inflammatory bowel disease is remission induction therapy or remission maintenance therapy of the inflammatory bowel disease.

4. The medicament for use according to any one of claims 1 to 3, which is used for suppression or reduction of adhesions or ulcers in the intestinal tissue of the irritable bowel syndrome or the inflammatory bowel disease.

5. The medicament for use according to any one of claims 1 to 4, which is used for suppression or reduction of a region of transmural inflammation in the intestinal tissue of the irritable bowel syndrome or the inflammatory bowel disease.

6. The medicament for use according to any one of claims 1 to 5, which is used for suppression or reduction of histological damage, mucosal necrosis or cellular infiltration in intestinal tissue of the irritable bowel syndrome or the inflammatory bowel disease.

7. The medicament for use according to any one of claims 1 to 6, which is used for protection or maintenance of mucosa in intestinal tissue of the irritable bowel syndrome or the inflammatory bowel disease.

## Patentansprüche

1. Medikament zur Verwendung bei der Vorbeugung oder Behandlung des Reizdarmsyndroms oder einer entzündlichen Darmerkrankung, umfassend
eine Verbindung mit einer P2X4-Rezeptor-antagonistischen Wirkung oder eine
pharmazeutisch verträgliches Salz davon als Wirkstoff, der aus der Gruppe ausgewählt ist, die besteht aus: (A16) 5-[3-(5-Thioxo-4H-[1,2,4]oxadiazol-3-yl)phenyl]-1H-naphtho[1,2-b][1,4]diazepin-2,4(3H,5H)-dion;
(A17) einem 5-[3-(5-Thioxo-4H-[1,2,4]oxadiazol-3-yl)phenyl]-1H-naphtho[1,2-b][1,4]diazepin-2,4(3H,5H)-dion-Natriumsalz; und
(B48) 5-[4-(2-Jodbenzoylamino)phenyl]-1H-Naphtho[1,2-b][1,4]diazepin-2,4(3H,5H)-dion.

2. Medikament zur Verwendung nach Anspruch 1, wobei die entzündliche Darmerkrankung Morbus Crohn oder Colitis ulcerosa ist.

3. Medikament zur Verwendung nach Anspruch 1 oder 2, wobei das Verhindern oder Behandeln einer entzündlichen Darmerkrankung eine Remissionsinduktionstherapie oder Remissionserhaltungstherapie der entzündlichen Darmerkrankung ist.

4. Medikament zur Verwendung nach einem der Ansprüche 1 bis 3, das zur Unterdrückung oder Reduzierung von Verwachsungen oder Geschwüren im Darmgewebe des Reizdarmsyndroms oder der entzündlichen Darmerkrankung verwendet wird.

5. Medikament zur Verwendung nach einem der Ansprüche 1 bis 4, das zur Unterdrückung oder Reduzierung einer Region einer transmuralen Entzündung im Darmgewebe des Reizdarmsyndroms oder der entzündlichen Darmerkrankung verwendet wird.

6. Medikament zur Verwendung nach einem der Ansprüche 1 bis 5, das zur Unterdrückung oder Reduzierung von histologischen Schäden, Schleimhautnekrosen oder zellulärer Infiltration im Darmgewebe des Reizdarmsyndroms oder der entzündlichen Darmerkrankung verwendet wird.

7. Medikament zur Verwendung nach einem der Ansprüche 1 bis 6, das zum Schutz oder zur Erhaltung der Schleimhaut im Darmgewebe des Reizdarmsyndroms oder der entzündlichen Darmerkrankung verwendet wird.

## Revendications

1. Médicament pour une utilisation dans la prévention ou le traitement du syndrome du côlon irritable ou une maladie inflammatoire de l'intestin, lequel comprend
un composé ayant une action antagoniste sur le récepteur P2X4 ou
un sel pharmaceutiquement acceptable de celui-ci en tant qu'ingrédient actif, lequel est sélectionné dans le groupe constitué de : (Al6) 5-[3-(5-thioxo-4H-[1,2,4]oxadiazol-3-yl)phényle]-1H-naphtho[1,2-b][1,4]diazépine-2, 4(3H,5H)-dione ;
(Al7) un sel de sodium de 5-[3-(5-thioxo-4H-[1,2,4]oxadiazol-3-yl)phényl]-1H naphto[1,2-b][1,4]diazépine-2,4(3H,5H)-dione ; et
(B48) 5-[4-(2-iodobenzoylamino)phényle]-1H-naphto[1,2-b][1,4]diazépine-2, 4(3H,5H)-dione.

2. Médicament pour une utilisation selon la revendication 1, dans lequel la maladie inflammatoire de l'intestin est la maladie de Crohn ou la colite ulcéreuse.

3. Médicament pour une utilisation selon la revendication 1 ou 2, dans lequel la prévention ou le traitement de la maladie inflammatoire de l'intestin est une thérapie d'induction de la rémission ou une thérapie de maintien de la rémission de la maladie inflammatoire de l'intestin.

4. Médicament pour une utilisation selon l'une des revendications 1 à 3, lequel est utilisé pour supprimer ou réduire les adhérences ou les ulcères dans le tissu intestinal du syndrome de l'intestin irritable ou de la maladie inflammatoire de l'intestin.

5. Médicament pour une utilisation selon l'une des revendications 1 à 4, lequel est utilisé pour supprimer ou réduire une région d'inflammation transmurale dans le tissu intestinal du syndrome de l'intestin irritable ou de la maladie inflammatoire de l'intestin.

6. Médicament pour une utilisation selon l'une des revendications 1 à 5, lequel est utilisé pour supprimer ou réduire les lésions histologiques, une nécrose de la muqueuse ou une infiltration cellulaire dans le tissu intestinal du syndrome de l'intestin irritable ou de la maladie inflammatoire de l'intestin.

7. Médicament pour une utilisation selon l'une des revendications 1 à 6, lequel est utilisé pour la protection ou l'entretien de la muqueuse dans le tissu intestinal du syndrome de l'intestin irritable ou de la maladie inflammatoire de l'intestin.
